# EUROPEAN PATENT APPLICATION

(11) **EP 4 759 814 A1**
(43) Date of publication of application: **17.06.2026**
(21) Application number: 24854422.3
(22) Date of filing: 09.08.2024
(51) Int. Cl.: C07D 487/04, A61K 31/4985, A61K 45/06, A61P 35/00, C07D 471/14, C07D 491/107

(54) **TRICYCLIC COMPOUND AND USE THEREOF AS MAT2A INHIBITOR**

(30) Priority: 11.08.2023 KR 20230105705; 02.04.2024 KR 20240044928
(71) Applicant: SK Biopharmaceuticals Co., Ltd., Seongnam-si, Gyeonggi-do 13494 (KR)
(72) Inventor: KIM, Jinhee, Seongnam-si, Gyeonggi-do 13494 (KR); JUNG, Hoiyun, Seongnam-si, Gyeonggi-do 13494 (KR); CHO, Gyeong Hi, Seongnam-si, Gyeonggi-do 13494 (KR); KI, So Young, Seongnam-si, Gyeonggi-do 13494 (KR); NA, Jungtae, Seongnam-si, Gyeonggi-do 13494 (KR); KOH, Ilkyoo, Seongnam-si, Gyeonggi-do 13494 (KR); CHO, Hyunho, Seongnam-si, Gyeonggi-do 13494 (KR); LEE, Ho Yeon, Seongnam-si, Gyeonggi-do 13494 (KR); SHIN, Yong Je, Seongnam-si, Gyeonggi-do 13494 (KR); MOON, Mijin, Seongnam-si, Gyeonggi-do 13494 (KR)
(74) Representative: Vossius & Partner Patentanwälte Rechtsanwälte mbB
(86) International application number: PCT/KR2024/011924
(87) International publication number: WO 2025/037871

(57) **Abstract**

The present application relates to a tricyclic compound and use thereof as an MAT2A inhibitor.

## Description

### [TECHNICAL FIELD]

The present application relates to tricyclic compounds and a use thereof as MAT2A inhibitors.

### [BACKGROUND ART]

Methionine Adenosyltransferase 2A (MAT2A) is a key intracellular enzyme responsible for the biosynthesis of S-adenosylmethionine (SAM) from methionine and adenosine triphosphate (ATP). MAT2A, which is also known as a rate-limiting enzyme of the methionine cycle, produces SAM, which is involved in the methylation of nucleic acids, phospholipids, histones, biogenic amines, and proteins, as well as the synthesis of polyamines and glutathione. Accordingly, MAT2A plays a critical role in regulating cellular growth, differentiation, and function through modulation of intracellular SAM levels.

Specifically, MAT2A has been reported as a therapeutic target exhibiting vulnerability in cancers deficient in Methylthioadenosine Phosphorylase (MTAP). MTAP is an enzyme that catalyzes the degradation methylthioadenosine (MTA), and the deletion of MTAP leads to MTA accumulation in cancer cells, and the increased MTA levels partially inhibit the activity of Protein Arginine Methyltransferase 5 (PRMT5). In MTAP-deficient cancers, where PRMT5 activity is suppressed, MAT2A-being responsible for the production of SAM, a cofactor required for PRMT5 activity-can indirectly influence PRMT5 signaling upstream and induce a synthetic lethal vulnerability. Accordingly, MAT2A inhibitors have been developed as therapeutic agents targeting MTAP-deficient cancers throught a synthetic lethality strategy. Preclinical studies have demonstrated that MAT2A inhibitors dose-dependently reduce intratumoral SAM levels and supresses tumor growth in xenograft models. In clinical studies, MAT2A inhibitors have also shown reductions in plasma SAM levels across various doses in patients with advanced solid tumors harboring homozygous MTAP deletions, supporting continued investigation of MAT2A inhibitors as treatments for MTAP-deficient cancers.

However, the initially developed MAT2A inhibitor, AG-270, was observed to cause hepatotoxicity associated with inhibition of UGT1A1 in Phase 1 clinical trials. Although MTAP deficiency is frequently observed in malignacies such as glioblastoma (GBM; approximately 40%) and non-small cell lung cancer (approximately 15%), which commonly presents with brain metastases, sufficient blood-brain barrier penetration. This limitation represents a significant unmet medical need in clinical devolopment of MAT2A-targeted therapies.

### [DETAILED DESCRIPTION OF THE INVENTION]

### [TECHNICAL PROBLEM]

The present inventors have identified that the tricyclic compounds according to an embodiment of the present application exhibits inhibitory activity against MAT2A. Therefore, an object of the present application is to provide tricyclic ring-based compounds of Chemical Formula 1 exhibiting potent inhibitory activity againt MAT2A, its optical isomers, diastereomers, isotopologues, hydrates, solvates, or pharmaceutically acceptable salts thereof.

Another object of the present application is to provide a pharmaceutical composition comprising, as an active ingredient, the tricyclic ring-based compounds, its optical isomers, diastereomers, isotopologues, hydrates, solvates, or pharmaceutically acceptable salts thereof.

### [TECHNICAL SOLUTION]

According to an embodiment of the present invention, there is provided a compound of Chemical Formula 1, its optical isomers, diastereomers, racemates, isotopologues, solvates, hydrates, or pharmaceutically acceptable salts thereof: 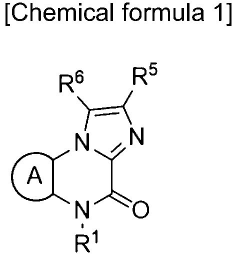
in Chemical formula 1,
A is 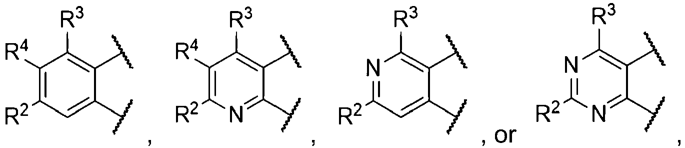
R² is selected from (C₁₋₆)alkyl, (C₂₋₆)alkenyl, (C₂₋₆)alkynyl, (C₁₋₆)alkoxy, (C₁₋₆)alkylsulfonyl, halo, (C₁₋₆)haloalkyl, (C₁₋₆)haloalkoxy, (C₃₋₁₀)cycloalkyl, cyano, nitro, amino, acetyl, (C₁₋₆)alkylamino, (C₁₋₆)alkylcarbonyl, (C₁₋₆)alkoxycarbonyl, aminocarbonyl, (C₁₋₆)alkylaminocarbonyl, (C₁₋₆)hydroxyalkyl, (C₁₋₆)hydroxyalkoxy, (C₁₋₆)hydroxyalkylamino, (C₁₋₆)alkoxy-(C₁₋₆)alkyl, (C₁₋₆)alkoxy-(C₁₋₆)alkoxy, (C₁₋₆)alkoxy-(C₁₋₆)alkylamino, (C₁₋₆)aminoalkyl, (C₁₋₆)aminoalkoxy, (C₁₋₆)aminoalkylamino, 3 to 10-membered heteroaryl containing one or more heteroatoms selected from N, O, and S, 3 to 10-membered heteroaryloxy containing one or more heteroatoms selected from N, O, and S, 3 to 10-membered heteroarylamino containing one or more heteroatoms selected from N, O, and S, 3 to 10-membered heterocyclyl containing one or more heteroatoms selected from N, O, and S, 3 to 10-membered heterocyclyloxy containing one or more heteroatoms selected from N, O, and S, 3 to 10-membered heterocyclylamino containing one or more heteroatoms selected from N, O, and S, 3 to 10-membered heterocyclyloxyalkoxy containing one or more heteroatoms selected from N, O, and S, or 3 to 10-membered heterocyclyloxyalkylamino containing one or more heteroatoms selected from N, O, and S, wherein the heterocyclyl or heteroaryl is unsubstituted, or substituted with one or more substituents from R^{a}, R^{b}, and R^{c}, wherein R^{a}, R^{b}, and R^{c} are independently selected from (C₁₋₆)alkyl, (C₃₋₁₀)cycloalkyl, (C₁₋₆)haloalkyl, (C₁₋₆)haloalkoxy, (C₁₋₆)alkoxy, hydroxy, halo, cyano, (C₁₋₆)hydroxyalkyl, (C₁₋₆)alkoxy-(C₁₋₆)alkyl, and (C₁₋₆)aminoalkyl;
R³ is hydrogen, (C₁₋₆)alkyl, (C₂₋₆)alkenyl, (C₂₋₆)alkynyl, (C₁₋₆)alkoxy, halo, halo(C₁₋₆)alkyl-(C₁₋₆)alkoxy, (C₁₋₆)alkylsulfonyl, (C₁₋₆)haloalkyl, (C₁₋₆)haloalkoxy, (C₃₋₁₀)cycloalkyl, (C₁₋₆)alkyloxy, cyano, amino, (C₁₋₆)alkylamino, aminocarbonyl-(C₁₋₆)alkylaminocarbonyl, (C₁₋₆)hydroxyalkyl, (C₁₋₆)hydroxyalkoxy, (C₁₋₆)hydroxyalkylamino, (C₂₋₆)alkoxyalkyl, (C₁₋₆)alkoxy-(C₁₋₆)alkoxy, (C₁₋₆)alkoxy-(C₁₋₆)alkylamino, (C₁₋₆)aminoalkyl, (C₁₋₆)aminoalkoxy, (C₁₋₆)aminoalkylamino, 3 to 10-membered heteroaryl containing one or more heteroatoms selected from N, O, and S, 3 to 10-membered heteroaryloxy containing one or more heteroatoms selected from N, O, and S, 3 to 10-membered heteroarylamino containing one or more heteroatoms selected from N, O, and S, 3 to 10-membered heterocyclyl containing one or more heteroatoms selected from N, O, and S, 3 to 10-membered heterocyclyloxy containing one or more heteroatoms selected from N, O, and S, 3 to 10-membered heterocyclylamino containing one or more heteroatoms selected from N, O, and S, 3 to 10-membered heterocyclyl-(C₁₋₆)alkyloxy containing one or more heteroatoms selected from N, O, and S, 3 to 10-membered heterocyclyloxy-(C₁₋₆)alkoxy containing one or more heteroatoms selected from N, O, and S, or 3 to 10-membered heterocyclyloxy-(C₁₋₆)alkylamino containing one or more heteroatoms selected from N, O, and S, wherein the alkoxy, heterocyclyl or the heteroaryl is unsubstituted, or substituted with one or more from R^{a}, R^{b}, and R^{c}, wherein R^{a}, R^{b}, and R^{c} are independently selected from (C₁₋₆)alkyl, (C₃₋₁₀)cycloalkyl, (C₁₋₆)haloalkyl, (C₁₋₆)haloalkoxy, (C₁₋₆)alkoxy, hydroxy, halo, cyano, (C₁₋₆)alkoxycarbonyl, (C₁₋₆)hydroxyalkyl, (C₁₋₆)alkoxy-(C₁₋₆)alkyl, and (C₁₋₆)aminoalkyl;
R⁴ is hydrogen, (C₁₋₆)alkyl, (C₁₋₆)alkenyl, (C₁₋₆)alkynyl, (C₁₋₆)alkoxy, (C₁₋₆)alkyl-(C₁₋₆)alkoxy, (C₁₋₆)alkylthiol, (C₁₋₆)alkylsulfonyl, halo, (C₁₋₆)haloalkyl, (C₁₋₆)haloalkoxy, (C₃₋₁₀)cycloalkyl, cyano, amino, (C₁₋₆)alkylamino, aminocarbonyl, or (C₁₋₆)alkylaminocarbonyl;
R¹ is 5 to 20-membered mono- or bicyclic aryl, or 3 to 10-membered mono- or bicyclic heteroaryl containing one or more heteroatoms selected from N, O, and S, wherein the aryl or the heteroaryl is unsubstituted or substituted with one or more of R^{d};
R^{d} is halo, oxo, cyano, hydroxy, (C₁₋₆)alkyl, (C₁₋₆)alkoxy, (C₃₋₁₀)cycloalkyl, (C₃₋₁₀)cycloalkyl-(C₁₋₆)alkyl, (C₁₋₆)haloalkyl, (C₁₋₆)haloalkoxy, (C₁-C₆)hydroxyalkyl, (C₁-C₆)alkoxy-(C₁-C₆)alkyl, (C₁-C₆)alkylamino, di(C₁-C₆)alkylamino, (C₁-C₆)alkylsulfonamide, 3 to 10-membered heteroaryl containing one or more heteroatoms selected from N, O, and S, 3- to 10-membered heterocycloalkyl containing one or more heteroatoms selected from N, O, and S, or phenyl;
R⁵ and R⁶ are independently hydrogen, halo, hydroxy, acetyl, (C₁₋₆)alkyl, (C₁₋₆)haloalkyl, (C₁₋₆)hydroxyalkyl, (C₁₋₆)alkoxy(C₁₋₆)alkyl, -(C₁₋₆)alkyl-amino, aminocarbonyl, (C₁₋₆)alkylaminocarbonyl, di(C₁₋₆)alkylaminocarbonyl, aminocarbonyl(C₁₋₆)alkyl, aminosulfonyl(C₁₋₆)alkyl, (C₁₋₆)alkoxy, -amino(C₁₋₆)-alkyl, -amino-di(C₁₋₆)alkyl, (C₁₋₆)alkylcarbonyl, or -(C₁₋₆)alkyl-R^{e}, and
the R^{e} is halo, (C₁₋₆)alkoxy, -amino-(C₁₋₆)alkyl-(C₁₋₆)alkoxy, -amino-(C₁₋₆)alkyl-hydroxy, (C₁₋₆)haloalkoxy, (C₁₋₆)alkoxy-(C₁₋₆)alkoxy, (C₁₋₆)hydroxyalkoxy, (C₁₋₆)alkylamino, or 3 to 10-membered mono or bicyclic heterocycle containing one or more heteroatoms selected from N, O, and S, and R^{e} is unsubstituted or substituted with (C₁₋₆)alkyl or hydroxy.

For example, the R² may be (C₁₋₆)alkyl, (C₁₋₆)alkoxy, halo, (C₁₋₆)haloalkyl, (C₁₋₆)haloalkoxy, (C₃₋₁₀)cycloalkyl, cyano, acetyl, or (C₁₋₆)alkylcarbonyl.

For example, the R² may be (C₁₋₃)alkyl, halo, acetyl, cyano, (C₃₋₆)cycloalkyl, (C₁₋₃)dihaloalkyl, (C₁₋₃)trihaloalkyl, (C₁₋₃)alkoxy, or (C₁₋₃)trihaloalkoxy.

For example, the R³ may be hydrogen, (C₁₋₆)alkyl, (C₁₋₆)alkoxy, halo, (C₁₋₆)haloalkoxy, halo(C₁₋₆)alkyl(C₁₋₆)alkoxy, halo(C₁₋₆)alkyl, (C₁₋₆)alkylamino, or pyrrolidine.

For example, the R⁴ may be hydrogen, (C₁₋₆)alkyl, (C₁₋₆)alkoxy, halo, or (C₁₋₆)haloalkyl.

For example, the R¹ may be unsubstituted or substituted with one or more, specifically 1 to 3, or more specifically 1 or 2 of R^{d}.

For example, the R¹ or substituted with one or more, specifically 1 to 3, or more specifically 1 or 2 of R^{d}.

For example, the R^{d} may be halo, oxo, cyano, hydroxy, (C₁₋₆)alkyl, (C₁₋₆)alkoxy, (C₃₋₁₀)cycloalkyl, (C₁₋₆)haloalkyl, (C₁₋₆)haloalkoxy, or (C₁-C₆)alkylsulfonamide.

For example, the R¹ may be phenyl, pyrazine, pyrazole, pyridine, indole, dihydrobenzofuran, or benzothiazole, and the R¹ may be unsubstituted or substituted with halo, cyano, hydroxy, (C₁₋₆)alkyl, (C₁₋₆)alkoxy, (C₃₋₁₀)cycloalkyl, (C₁₋₆)haloalkyl, (C₁₋₆)haloalkoxy, or (C₁-C₆)alkylsulfonamide.

For example, the R¹ may be substituted with phenyl, pyrazine, pyrazole, pyridine, indole, dihydrobenzofuran, or benzothiazole, and R¹ may be halo, cyano, hydroxy, (C₁₋₆)alkyl, (C₁₋₆)alkoxy, (C₃₋₁₀)cycloalkyl, (C₁₋₆)haloalkyl, (C₁₋₆)haloalkoxy, or (C₁₋C₆)alkylsulfonamide.

For example, the R⁵ and R⁶ may be independently hydrogen, halo, hydroxy, acetyl, (C₁₋₆)alkyl, (C₁₋₆)haloalkyl, (C₁₋₆)hydroxyalkyl, (C₁₋₆)alkoxy(C₁₋₆)alkyl, amino(C₁₋₆)alkyl, di(C₁₋₆)alkylaminocarbonyl, (C₁₋₆)alkoxy, (C₁₋₆)alkylamino, di(C₁₋₆)alkylamino, (C₁₋₆)alkylamino(C₁₋₆)alkyl, (C₁₋₆)alkylamino(C₁₋₆)alkylhydroxy, (C₁₋₆)alkylamino(C₁₋₆)alkyl(C₁₋₆)alkoxy, or (C₁₋₆)alkylcarbonyl.

For example, the R⁵ may be hydrogen, halo, (C₁₋₆)alkyl, (C₁₋₆)alkoxy, (C₁₋₆)alkylamino-, di(C₁₋₆)alkyl-amino-, (C₁₋₆)haloalkyl, acetyl, or -CH₂R^{e}, the R^{e} may be substituted with halo, (C₁₋₆)alkoxy, -amino-(C₁₋₆)alkyl-(C₁₋₆)alkoxy, -amino-(C₁₋₆)alkyl-hydroxy, (C₁₋₆)haloalkoxy, (C₁₋₆)alkoxy-(C₁₋₆)alkoxy, (C₁₋₆)hydroxyalkoxy, (C₁₋₆)alkyl-amino-, or 3 to 10-membered mono or bicyclic heterocycle containing one or more heteroatoms selected from N, O, and S, and the R^{e} may be unsubstituted or substituted with (C₁₋₆)alkyl or hydroxy.

For example, the R^{e} may be halo, (C₁₋₆)alkoxy, -amino-(C₁₋₆)alkyl-(C₁₋₆)alkoxy, - amino-(C₁₋₆)alkyl-hydroxy, (C₁₋₆)haloalkoxy, (C₁₋₆)alkoxy-(C₁₋₆)alkoxy, (C₁₋₆)hydroxyalkoxy, (C₁₋₆)alkyl-amino-, azetidine, morpholine, piperazine, or heterospirocarbocyclyl, and the R^{e} may be unsubstituted or substituted with (C₁₋₆)alkyl or hydroxy.

For example, the R^{e} may be methoxy, methylamine, hydroxyethylamine, methoxyethylamine, azetidine, 3-hydroxyazetidine, methylpiperazine, morpholine, or 2-oxa-7-azaspiro[3.5]nonane.

For example, the R⁶ may be hydrogen, halo, (C₁₋₆)alkyl, (C₁₋₆)haloalkyl, acetyl, or hydroxy.

For example, A may be

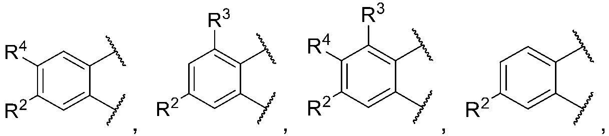

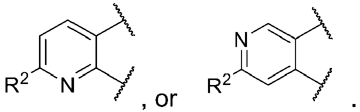

Specifically, the A may be any one selected from the group consisting of:

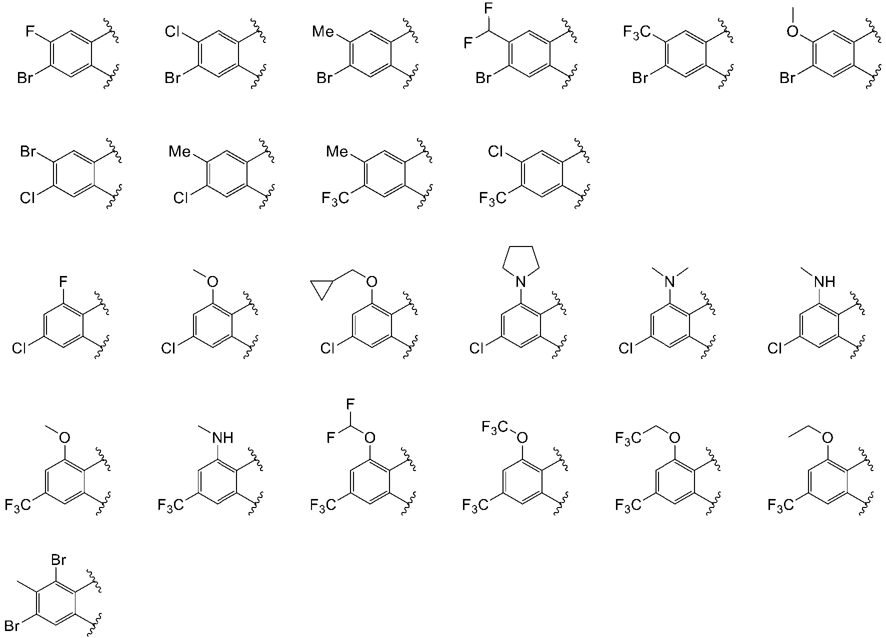

More specifically, A may be any one selected from the group consisting of:

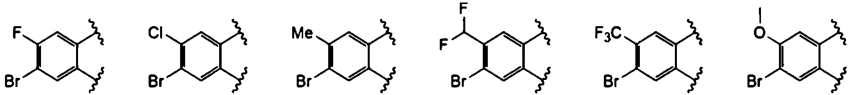

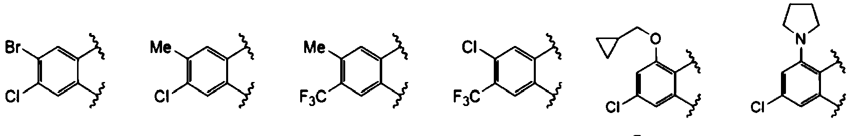

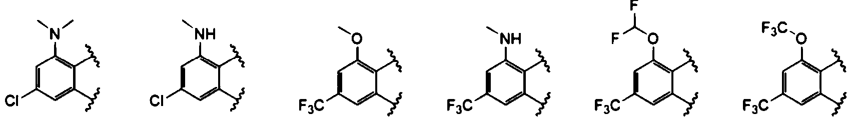

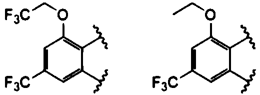

For example, the A is 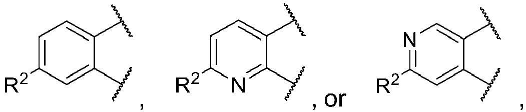 and the R² may be (C₁₋₆)alkyl, (C₁₋₆)alkoxy, halo, (C₁₋₆)haloalkyl, (C₁₋₆)haloalkoxy, (C₃₋₁₀)cycloalkyl, cyano, acetyl, or (C₁₋₆)alkylcarbonyl. The R² may be fluoro, chloro, bromo, iodo, methyl, isopropyl, acetyl, cyano, nitro, cyclopropyl, difluoromethyl, trifluoromethyl, methoxy, ethoxy, trifluoromethoxy, or trifluoroethoxy.

For example, the A is 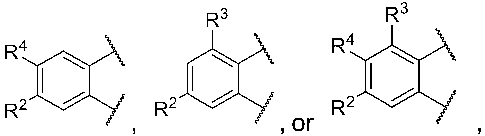 the R² may be (C₁₋₆)alkyl, (C₁₋₆)alkoxy, halo, (C₁₋₆)haloalkyl, (C₁₋₆)haloalkoxy, (C₃₋₁₀)cycloalkyl, cyano, acetyl, or (C₁₋₆)alkylcarbonyl, the R³ may be (C₁₋₆)alkyl, (C₁₋₆)alkoxy, halo, (C₁₋₆)haloalkoxy, halo(C₁₋₆)alkyl(C₁₋₆)alkoxy, halo(C₁₋₆)alkyl, (C₁₋₆)alkylamino, or pyrrolidine, and R⁴ may be (C₁₋₆)alkyl, (C₁₋₆)alkoxy, halo, or (C₁₋₆)haloalkyl.

For example, representative examples of compounds of Chemical formula 1 according to an embodiment of the present application include, but are not limited to, those listed in Table 1 below:

**[Table 1]**

| Example | Chemical structure | Chemical name |
|---|---|---|
| 1 | 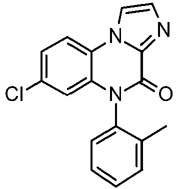 | 7-chloro-5-(*o*-tolyl)imidazo[1,2-*a*]quinoxalin-4(5*H*)-one |
| 2 | 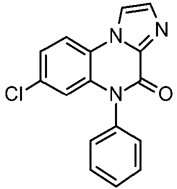 | 7-chloro-5-phenylimidazo[1,2-*a*]quinoxalin-4(5*H*)-one |
| 3 | 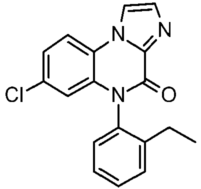 | 7-chloro-5-(2-ethylphenyl)imidazo[1,2-*a*]quinoxalin-4(5*H*)-one |
| 4 | 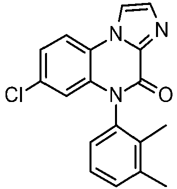 | 7-chloro-5-(2,3-dimethylphenyl)imidazo[1,2-*a*]quinoxalin-4(5*H*)-one |
| 5 | 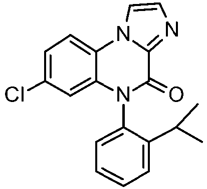 | 7-chloro-5-(2-isopropylphenyl)imidazo[1,2-*a*]quinoxalin-4(5*H*)-one |
| 6 | 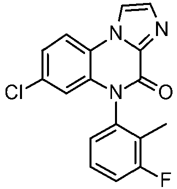 | 7-chloro-5-(3-fluoro-2-methylphenyl)imidazo[1,2-*a*]quinoxalin-4(5*H*)-one |
| 7 | 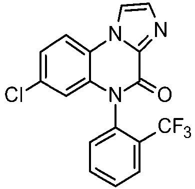 | 7-chloro-5-(2-(trifluoromethyl)phenyl)imidazo[1,2-*a*]quinoxalin-4(5*H*)-one |
| 8 | 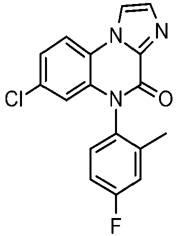 | 7-chloro-5-(4-fluoro-2-methylphenyl)imidazo[1,2-*a*]quinoxalin-4(5*H*)-one |
| 9 | 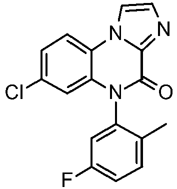 | 7-chloro-5-(5-fluoro-2-methylphenyl)imidazo[1,2-*a*]quinoxalin-4(5*H*)-one |
| 10 | 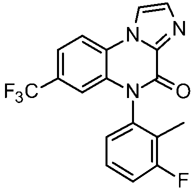 | 5-(3-fluoro-2-methylphenyl)-7-(trifluoromethyl)imidazo[1,2*-a*]quinoxalin-4(5*H*)-one |
| 11 | 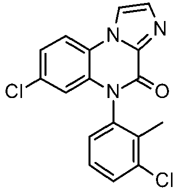 | 7-chloro-5-(3-chloro-2-methylphenyl)imidazo[1,2-*a*]quinoxalin-4(5*H*)-one |
| 12 | 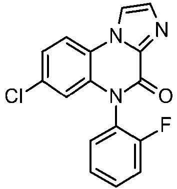 | 7-chloro-5-(2-fluorophenyl)imidazo[1,2-*a*]quinoxalin-4(5*H*)-one |
| 13 | 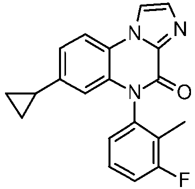 | 7-cyclopropyl-5-(3-fluoro-2-methylphenyl)imidazo[1,2-*a*]quinoxalin-4(5*H*)-one |
| 14 | 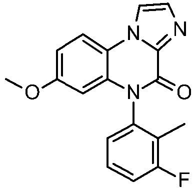 | 5-(3-fluoro-2-methylphenyl)-7-methoxyimidazo[1,2-*a*]quinoxalin-4(5*H*)-one |
| 15 | 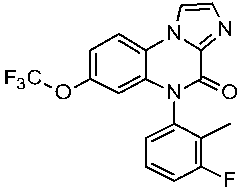 | 5-(3-fluoro-2-methylphenyl)-7-(trifluoromethoxy)imidazo[1,2-*a*]quinoxalin-4(5*H*)-one |
| 16 | 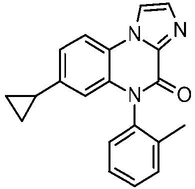 | 7-cyclopropyl-5-(*o*-tolyl)imidazo[1,2-*a*]quinoxalin-4(5*H*)-one |
| 17 | 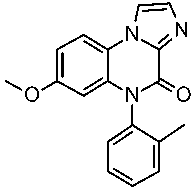 | 7-methoxy-5-(*o*-tolyl)imidazo[1,2-*a*]quinoxalin-4(5*H*)-one |
| 18 | 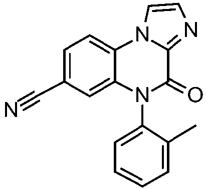 | 4-oxo-5-(*o*-tolyl)-4,5-dihydroimidazo[1,2-*a*]quinoxalin-7-carbonitrile |
| 19 | 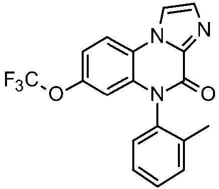 | 5-(*o*-tolyl)-7-(trifluoromethoxy)imidazo[1,2-*a*]quinoxalin-4(5*H*)-one |
| 20 | 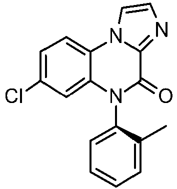 | (*R*ₐ)-7-chloro-5-(*o*-tolyl)imidazo[1,2-*a*]quinoxalin-4(5*H*)-one |
| 21 | 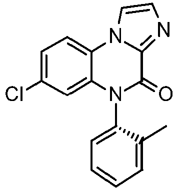 | (*S*ₐ)-7-chloro-5-(*o*-tolyl)imidazo[1,2-*a*]quinoxalin-4(5*H*)-one |
| 22 | 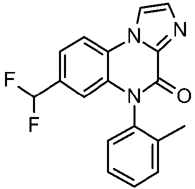 | 7-(difluoromethyl)-5-(*o*-tolyl)imidazo[1,2-*a*]quinoxalin-4(5*H*)-one |
| 23 | 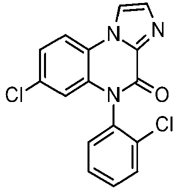 | 7-chloro-5-(2-chlorophenyl)imidazo[1,2-*a*]quinoxalin-4(5*H*)-one |
| 24 | 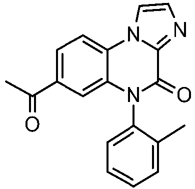 | 7-acetyl-5-(*o*-tolyl)imidazo[1,2-*a*]quinoxalin-4(5*H*)-one |
| 25 | 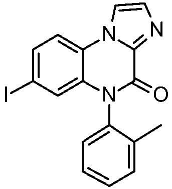 | 7-Iodo-5-(*o*-tolyl)imidazo[1,2-*a*]quinoxalin-4(5*H*)-one |
| 26 | 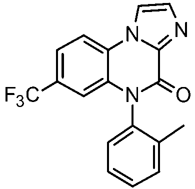 | 5-(*o-*tolyl)-7-(trifluoromethyl)imidazo[1,2-*a*]quinoxalin-4(5*H*)-one |
| 27 | 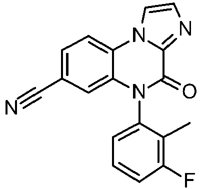 | 5-(3-fluoro-2-methylphenyl)-4-oxo-4,5-dihydroimidazo[1,2-*a*]quinoxalin-7-carbonitrile |
| 28 | 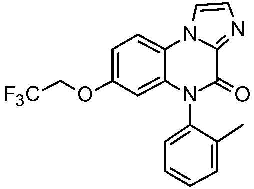 | 5-(*o*-tolyl)-7-(2,2,2-trifluoroethoxy)imidazo[1,2-*a*]quinoxalin-4(5*H*)-one |
| 29 | 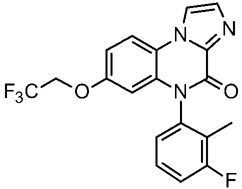 | 5-(3-fluoro-2-methylphenyl)-7-(2,2,2-trifluoroethoxy)imidazo[1,2-*a*]quinoxalin-4(5*H*)-one |
| 30 | 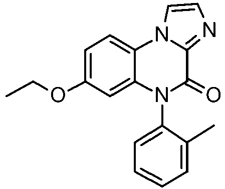 | 7-ethoxy-5-(*o*-tolyl)imidazo[1,2-*a*]quinoxalin-4(5*H*)-one |
| 31 | 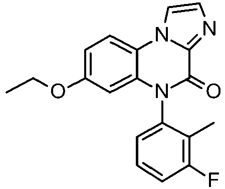 | 7-ethoxy-5-(3-fluoro-2-methylphenyl)imidazo[1,2-*a*]quinoxalin-4(5*H*)-one |
| 32 | 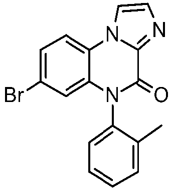 | 7-bromo-5-(*o*-tolyl)imidazo[1,2-*a*]quinoxalin-4(5*H*)-one |
| 33 | 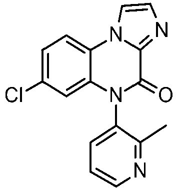 | 7-chloro-5-(2-methylpyridin-3-yl)imidazo[1,2-*a*]quinoxalin-4(5*H*)-one |
| 34 | 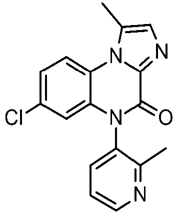 | 7-chloro-1-methyl-5-(2-methylpyridin-3-yl)imidazo[1,2-*a*]quinoxalin-4(5*H*)-one |
| 35 | 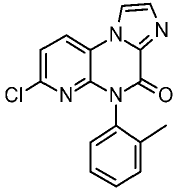 | 7-chloro-5-(*o*-tolyl)imidazo[1,2-*a*]pyrido[2,3-e]pyrazin-4(5*H*)-one |
| 36 | 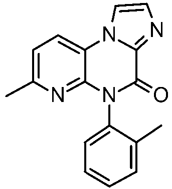 | 7-methyl-5-(*o*-tolyl)imidazo[1,2-*a*]pyrido[2,3-e]pyrazin-4(5*H*)-one |
| 37 | 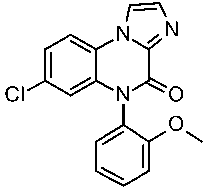 | 7-chloro-5-(2-methoxyphenyl)imidazo[1,2-*a*]quinoxalin-4(5*H*)-one |
| 38 | 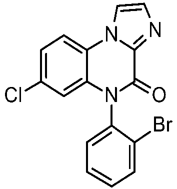 | 5-(2-bromophenyl)-7-chloroimidazo[1,2-*a*]quinoxalin-4(5*H*)-one |
| 39 | 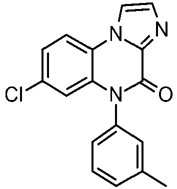 | 7-chloro-5-(*m*-tolyl)imidazo[1,2-*a*]quinoxalin-4(5*H*)-one |
| 40 | 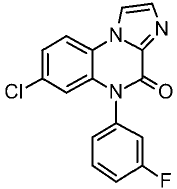 | 7-chloro-5-(3-fluorophenyl)imidazo[1,2-*a*]quinoxalin-4(5*H*)-one |
| 41 | 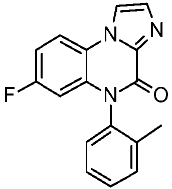 | 7-fluoro-5-(*o*-tolyl)imidazo[1,2-*a*]quinoxalin-4(5*H*)-one |
| 42 | 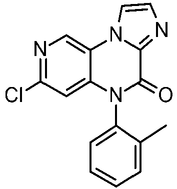 | 3-chloro-5-(*o*-tolyl)imidazo[1,2-*a*]pyrido[4,3-e]pyrazin-6(5*H*)-one |
| 43 | 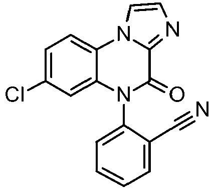 | 2-(7-chloro-4-oxoimidazo[1,2-*a*]quinoxalin-5(4*H*)-yl)benzonitrile |
| 44 | 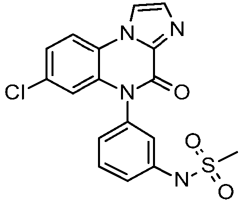 | *N*-(3-(7-chloro-4-oxoimidazo[1,2-*a*]quinoxalin-5(4*H*)-yl)phenyl)methanesulfonamide |
| 45 | 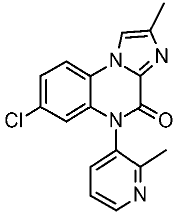 | 7-chloro-2-methyl-5-(2-methylpyridin-3-yl)imidazo[1,2-*a*]quinoxalin-4(5*H*)-one |
| 46 | 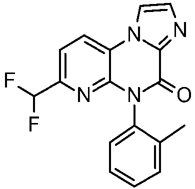 | 7-(difluoromethyl)-5-(*o*-tolyl)imidazo[1,2-*a*]pyrido[2,3-e]pyrazin-4(5*H*)-one |
| 47 | 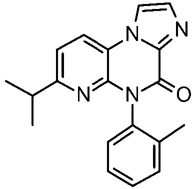 | 7-isopropyl-5-(*o*-tolyl)imidazo[1,2-*a*]pyrido[2,3-e]pyrazin-4(5*H*)-one |
| 48 | 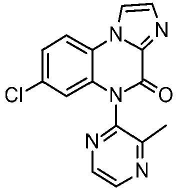 | 7-chloro-5-(3-methylpyrazin-2-yl)imidazo[1,2-*a*]quinoxalin-4(5*H*)-one |
| 49 | 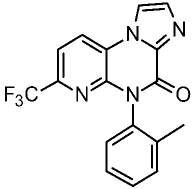 | 5-(*o*-tolyl)-7-(trifluoromethyl)imidazo[1,2-*a*]pyrido[2,3-e]pyrazin-4(5*H*)-one |
| 50 | 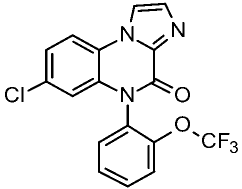 | 7-chloro-5-(2-(trifluoromethoxy)phenyl)imidazo[1,2-*a*]quinoxalin-4(5*H*)-one |
| 51 | 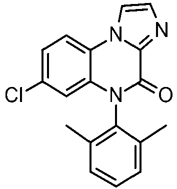 | 7-chloro-5-(2,6-dimethylphenyl)imidazo[1,2-*a*]quinoxalin-4(5*H*)-one |
| 52 | 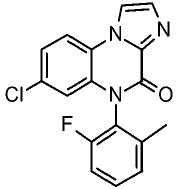 | 7-chloro-5-(2-fluoro-6-methylphenyl)imidazo[1,2-*a*]quinoxalin-4(5*H*)-one |
| 53 | 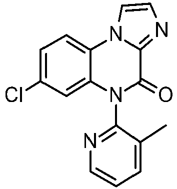 | 7-chloro-5-(3-methylpyridin-2-yl)imidazo[1,2-*a*]quinoxalin-4(5*H*)-one |
| 54 | 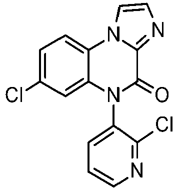 | 7-chloro-5-(2-chloropyridin-3-yl)imidazo[1,2-*a*]quinoxalin-4(5*H*)-one |
| 55 | 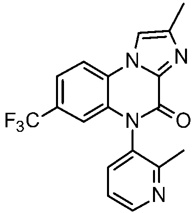 | 2-methyl-5-(2-methylpyridin-3-yl)-7-(trifluoromethyl)imidazo[1,2-*a*]quinoxalin-4(5*H*)-one |
| 56 | 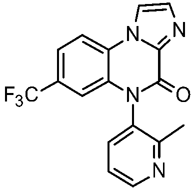 | 5-(2-methylpyridin-3-yl)-7-(trifluoromethyl)imidazo[1,2-*a*]quinoxalin-4(5*H*)-one |
| 57 | 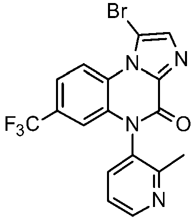 | 1-bromo-5-(2-methylpyridin-3-yl)-7-(trifluoromethyl)imidazo[1,2-*a*]quinoxalin-4(5*H*)-one |
| 58 | 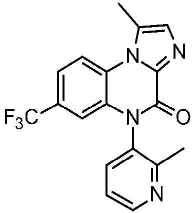 | 1-methyl-5-(2-methylpyridin-3-yl)-7-(trifluoromethyl)imidazo[1,2-*a*]quinoxalin-4(5*H*)-one |
| 59 | 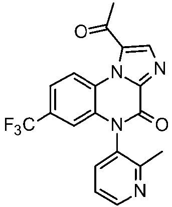 | 1-acetyl-5-(2-methylpyridin-3-yl)-7-(trifluoromethyl)imidazo[1,2-*a*]quinoxalin-4(5*H*)-one |
| 60 | 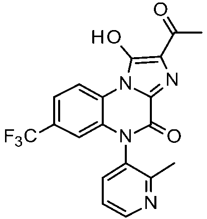 | 2-acetyl-1-hydroxy-5-(2-methylpyridin-3-yl)-7-(trifluoromethyl)imidazo[1,2-*a*]quinoxalin-4(5*H*)-one |
| 61 | 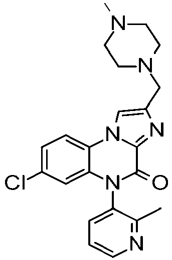 | 7-chloro-2-((4-methylpiperazin-1-yl)methyl)-5-(2-methylpyridin-3-yl)imidazo[1,2-*a*]quinoxalin-4(5*H*)-one |
| 62 | 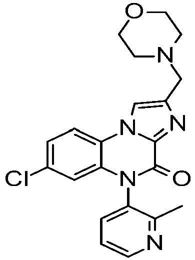 | 7-chloro-5-(2-methylpyridin-3-yl)-2-(morpholinomethyl)imidazo[1,2-*a*]quinoxalin-4(5*H*)-one |
| 63 | 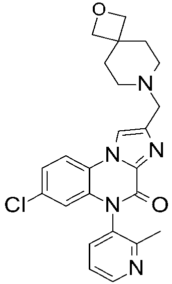 | 2-((2-oxa-7-azaspiro[3.5]nonane-7-yl)methyl)-7-chloro-5-(2-methylpyridin-3-yl)imidazo[1,2-*a*]quinoxalin-4(5*H*)-one |
| 64 | 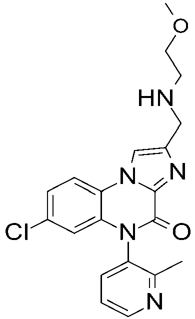 | 7-chloro-2-(((2-methoxyethyl)amino)methyl)-5-(2-methylpyridin-3-yl)imidazo[1,2-*a*]quinoxalin-4(5*H*)-one |
| 65 | 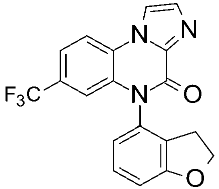 | 5-(2,3-dihydrobenzofuran-4-yl)-7-(trifluoromethyl)imidazo[1,2-*a*]quinoxalin-4(5*H*)-one |
| 66 | 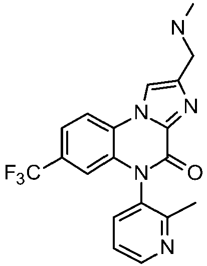 | 2-((methylamino)methyl)-5-(2-methylpyridin-3-yl)-7-(trifluoromethyl)-3,3a-dihydroimidazo[1,2-*a*]quinoxalin-4(5*H*)-one |
| 67 | 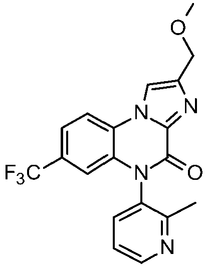 | 2-(methoxymethyl)-5-(2-methylpyridin-3-yl)-7-(trifluoromethyl)-3,3a-dihydroimidazo[1,2-*a*]quinoxalin-4(5*H*)-one |
| 68 | 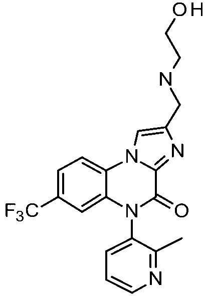 | 2-(((2-hydroxyethyl)amino)methyl)-5-(2-methylpyridin-3-yl)-7-(trifluoromethyl)-3,3a-dihydroimidazo[1,2-*a*]quinoxalin-4(5*H*)-one |
| 69 | 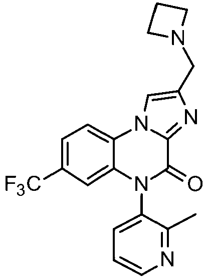 | 2-(azetidin-1-ylmethyl)-5-(2-methylpyridin-3-yl)-7-(trifluoromethyl)-3,3a-dihydroimidazo[1,2-*a*]quinoxalin-4(5*H*)-one |
| 70 | 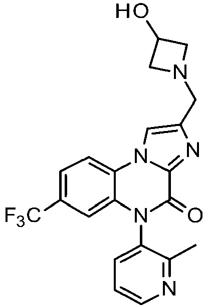 | 2-((3-hydroxyazetidin-1-yl)methyl)-5-(2-methylpyridin-3-yl)-7-(trifluoromethyl)-3,3a-dihydroimidazo[1,2-*a*]quinoxalin-4(5*H*)-one |
| 71 | 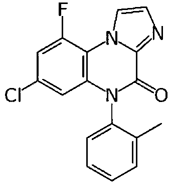 | 7-chloro-9-fluoro-5-(*o*-tolyl)imidazo[1,2-*a*]quinoxalin-4(5*H*)-one |
| 72 | 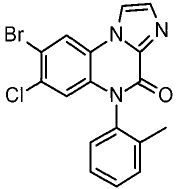 | 8-bromo-7-chloro-5-(*o*-tolyl)imidazo[1,2-*a*]quinoxalin-4(5*H*)-one |
| 73 | 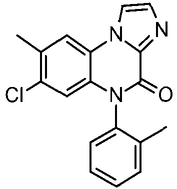 | 7-chloro-8-methyl-5-(*o*-tolyl)imidazo[1,2-*a*]quinoxalin-4(5*H*)-one |
| 74 | 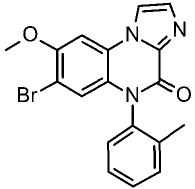 | 7-bromo-8-methoxy-5-(*o*-tolyl)imidazo[1,2-*a*]quinoxalin-4(5*H*)-one |
| 75 | 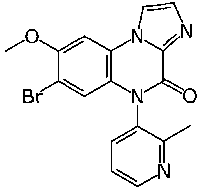 | 7-bromo-8-methoxy-5-(2-methylpyridin-3-yl)imidazo[1,2-*a*]quinoxalin-4(5*H*)-one |
| 76 | 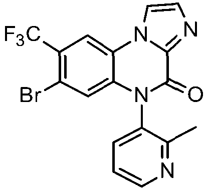 | 7-bromo-5-(2-methylpyridin-3-yl)-8-(trifluoromethyl)imidazo[1,2-*a*]quinoxalin-4(5*H*)-one |
| 77 | 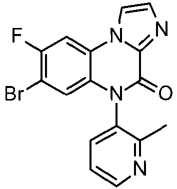 | 7-bromo-8-fluoro-5-(2-methylpyridin-3-yl)imidazo[1,2-*a*]quinoxalin-4(5*H*)-one |
| 78 | 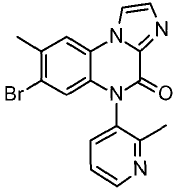 | 7-bromo-8-methyl-5-(2-methylpyridin-3-yl)imidazo[1,2-*a*]quinoxalin-4(5*H*)-one |
| 79 | 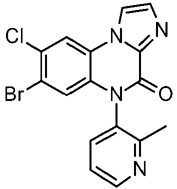 | 7-bromo-8-chloro-5-(2-methylpyridin-3-yl)imidazo[1,2-*a*]quinoxalin-4(5*H*)-one |
| 80 | 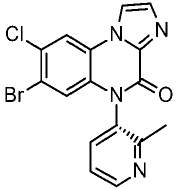 | (*R*ₐ)-7-bromo-8-chloro-5-(2-methylpyridin-3-yl)imidazo[1,2-*a*]quinoxalin-4(5*H*)-one |
| 81 | 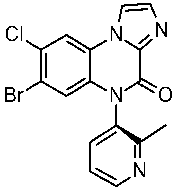 | (*S*ₐ)-7-bromo-8-chloro-5-(2-methylpyridin-3-yl)imidazo[1,2-*a*]quinoxalin-4(5*H*)-one |
| 82 | 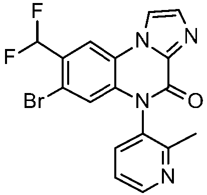 | 7-bromo-8-(difluoromethyl)-5-(2-methylpyridin-3-yl)imidazo[1,2-*a*]quinoxalin-4(5*H*)-one |
| 83 | 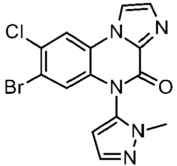 | 7-bromo-8-chloro-5-(1-methyl-1*H*-pyrazol-5-yl)imidazo[1,2-*a*]quinoxalin-4(5*H*)-one |
| 84 | 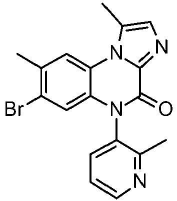 | 7-bromo-1,8-dimethyl-5-(2-methylpyridin-3-yl)imidazo[1,2-*a*]quinoxalin-4(5*H*)-one |
| 85 | 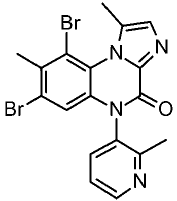 | 7,9-dibromo-1,8-dimethyl-5-(2-methylpyridin-3-yl)imidazo[1,2-*a*]quinoxalin-4(5*H*)-one |
| 86 | 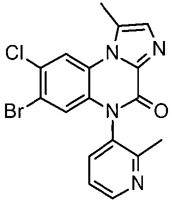 | 7-bromo-8-chloro-1-methyl-5-(2-methylpyridin-3-yl)imidazo[1,2-*a*]quinoxalin-4(5*H*)-one |
| 87 | 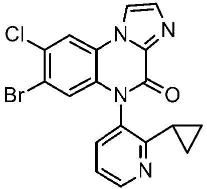 | 7-bromo-8-chloro-5-(2-cyclopropylpyridin-3-yl)imidazo[1,2-*a*]quinoxalin-4(5*H*)-one |
| 88 | 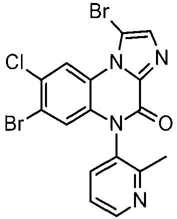 | 1,7-dibromo-8-chloro-5-(2-methylpyridin-3-yl)imidazo[1,2-*a*]quinoxalin-4(5*H*)-one |
| 89 | 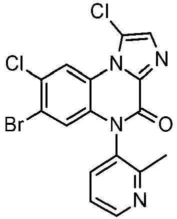 | 7-bromo-1,8-dichloro-5-(2-methylpyridin-3-yl)imidazo[1,2-*a*]quinoxalin-4(5*H*)-one |
| 90 | 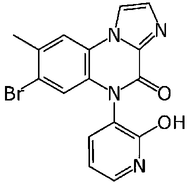 | 7-bromo-5-(2-hydroxypyridin-3-yl)-8-methylimidazo[1,2-*a*]quinoxalin-4(5*H*)-one |
| 91 | 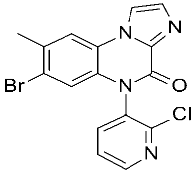 | 7-bromo-5-(2-chloropyridin-3-yl)-8-methylimidazo[1,2-*a*]quinoxalin-4(5*H*)-one |
| 92 | 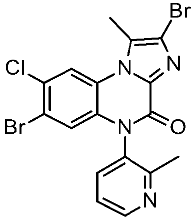 | 2,7-dibromo-8-chloro-1-methyl-5-(2-methylpyridin-3-yl)imidazo[1,2-*a*]quinoxalin-4(5*H*)-one |
| 93 | 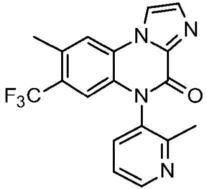 | 8-methyl-5-(2-methylpyridin-3-yl)-7-(trifluoromethyl)imidazo[1,2-*a*]quinoxalin-4(5*H*)-one |
| 94 | 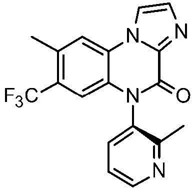 | (*R*ₐ)-8-methyl-5-(2-methylpyridin-3-yl)-7-(trifluoromethyl)imidazo[1,2-*a*]quinoxalin-4(5*H*)-one |
| 95 | 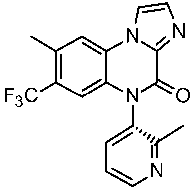 | (*S*ₐ)-8-methyl-5-(2-methylpyridin-3-yl)-7-(trifluoromethyl)imidazo[1,2-*a*]quinoxalin-4(5*H*)-one |
| 96 | 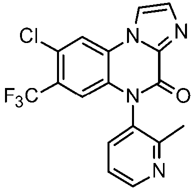 | 8-chloro-5-(2-methylpyridin-3-yl)-7-(trifluoromethyl)imidazo[1,2-*a*]quinoxalin-4(5*H*)-one |
| 97 | 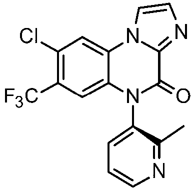 | (*R*ₐ)-8-chloro-5-(2-methylpyridin-3-yl)-7-(trifluoromethyl)imidazo[1,2-*a*]quinoxalin-4(5*H*)-one |
| 98 | 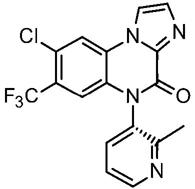 | (*S*ₐ)-8-chloro-5-(2-methylpyridin-3-yl)-7-(trifluoromethyl)imidazo[1,2-*a*]quinoxalin-4(5*H*)-one |
| 99 | 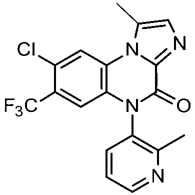 | 8-chloro-1-methyl-5-(2-methylpyridin-3-yl)-7-(trifluoromethyl)imidazo[1,2-*a*]quinoxalin-4(5*H*)-one |
| 100 | 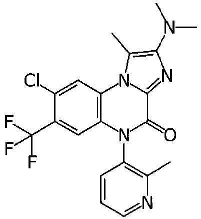 | 8-chloro-2-(dimethylamino)-1-methyl-5-(2-methylpyridin-3-yl)-7-(trifluoromethyl)imidazo[1,2*-a*]quinoxalin-4(5*H*)-one |
| 101 | 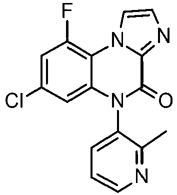 | 7-chloro-9-fluoro-5-(2-methylpyridin-3-yl)imidazo[1,2-*a*]quinoxalin-4(5*H*)-one |
| 102 | 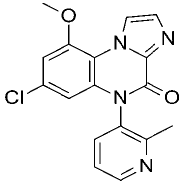 | 7-chloro-9-methoxy-5-(2-methylpyridin-3-yl)imidazo[1,2-*a*]quinoxalin-4(5*H*)-one |
| 103 | 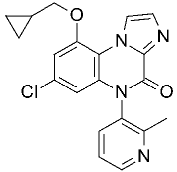 | 7-chloro-9-(cyclopropylmethoxy)-5-(2-methylpyridin-3-yl)imidazo[1,2-*a*]quinoxalin-4(5*H*)-one |
| 104 | 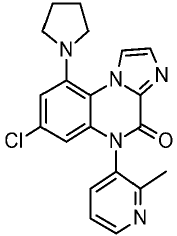 | 7-chloro-5-(2-methylpyridin-3-yl)-9-(pyrrolidine-1-yl)imidazo[1,2-*a*]quinoxalin-4(5*H*)-one |
| 105 | 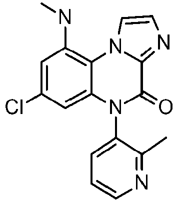 | 7-chloro-9-(methylamino)-5-(2-methylpyridin-3-yl)imidazo[1,2-*a*]quinoxalin-4(5*H*)-one |
| 106 | 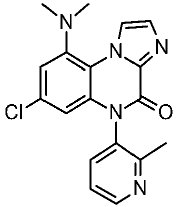 | 7-chloro-9-(dimethylamino)-5-(2-methylpyridin-3-yl)imidazo[1,2-*a*]quinoxalin-4(5*H*)-one |
| 107 | 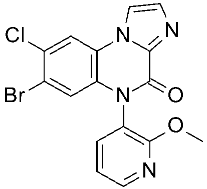 | 7-bromo-8-chloro-5-(2-methoxypyridin-3-yl)imidazo[1,2-a]quinoxalin-4(5H)-one |
| 108 | 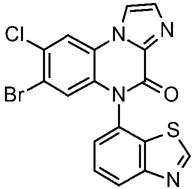 | 5-(benzo[d]thiazol-7-yl)-7-bromo-8-chloroimidazo[1,2-a]quinoxalin-4(5H)-one |
| 109 | 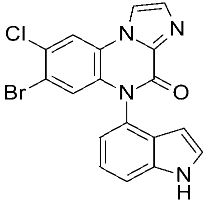 | 7-bromo-8-chloro-5-(1H-indol-4-yl)imidazo[1,2-*a*]quinoxalin-4(5*H*)-one |
| 110 | 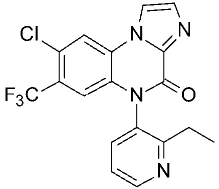 | 8-chloro-5-(2-ethylpyridin-3-yl)-7-(trifluoromethyl)imidazo[1,2-*a*]quinoxalin-4(5*H*)-one |
| 111 | 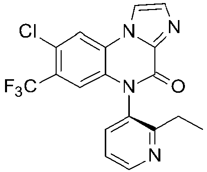 | (*R*ₐ)-8-chloro-5-(2-ethylpyridin-3-yl)-7-(trifluoromethyl)imidazo[1,2-*a*]quinoxalin-4(5*H*)-one |
| 112 | 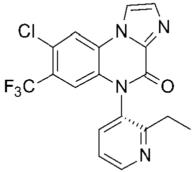 | (*S*ₐ)-8-chloro-5-(2-ethylpyridin-3-yl)-7-(trifluoromethyl)imidazo[1,2-*a*]quinoxalin-4(5*H*)-one |
| 113 | 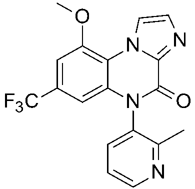 | 9-methoxy-5-(2-methylpyridin-3-yl)-7-(trifluoromethyl)imidazo[1,2-*a*]quinoxalin-4(5*H*)-one |
| 114 | 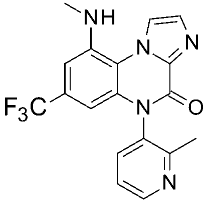 | 9-methylamino-5-(2-methylpyridin-3-yl)-7-(trifluoromethyl)imidazo[1,2-*a*]quinoxalin-4(5*H*)-one |
| 115 | 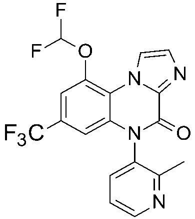 | 9-(difluoromethoxy)-5-(2-methylpyridin-3-yl)-7-(trifluoromethyl)imidazo[1,2-*a*]quinoxalin-4(5*H*)-one |
| 116 | 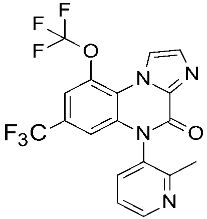 | 5-(2-methylpyridin-3-yl)-9-(trifluoromethoxy)-7-(trifluoromethyl)imidazo[1,2-*a*]quinoxalin-4(5*H*)-one |
| 117 | 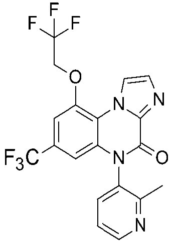 | 5-(2-methylpyridin-3-yl)-9-(2,2,2-trifluoroethoxy)-7-(trifluoromethyl)imidazo[1,2-*a*]quinoxalin-4(5*H*)-one |
| 118 | 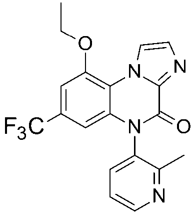 | 9-ethoxy-5-(2-methylpyridin-3-yl)-7-(trifluoromethyl)imidazo[1,2*-a*]quinoxalin-4(5*H*)-one |
| 119 | 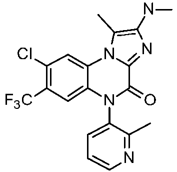 | 8-chloro-1-methyl-2-(methylamino)-5-(2-methylpyridin-3-yl)-7-(trifluoromethyl)imidazo[1,2-*a*]quinoxalin-4(5*H*)-one |
| 120 | 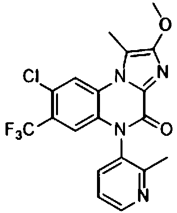 | 8-chloro-2-methoxy-1-methyl-5-(2-methylpyridin-3-yl)-7-(trifluoromethyl)imidazo[1,2-*a*]quinoxalin-4(5*H*)-one |

All technical terms used in the present application, unless otherwise defined, are used with meanings as commonly understood by a person of ordinary skill in the relevant field of the present application. In addition, although exemplary methods or samples are described in the present specification, those similar or equivalent thereto are also included in the scope of the present application. Furthermore, numerical values described in the present specification are considered to include the meaning of "about" even if not specified. The contents of all publications cited as references in the present specification are incorporated herein by reference in their entirety.

In the Chemical formula 1, the moieties listed as R¹ to R⁶, R^{a}, R^{b}, R^{c}, R^{d}, and R^{e} are used with meanings as commonly understood by a person of ordinary skill in the art.

Unless otherwise stated, "halogen" as used herein refers to fluorine, chlorine, bromine or iodine, when used alone or in combination with other additional terms (for example, haloalkyl).

Unless otherwise stated, "hydroxy" or "hydroxyl" as used herein means -OH, when used alone or in combination with other terms.

Unless otherwise stated, the term "amino" as used herein means -NH₂, when used alone or in combination with other terms.

Unless otherwise stated, the term "alkylamino" as used herein means amino in which one or more hydrogens are substituted with identical or different alkyl, for example (C1-6)alkyl, and includes, for example, methylamino, dimethylamino, and the like, but is not limited thereto.

Unless otherwise stated, the term "alkoxy" as used herein means alkyloxy, for example, alkyloxy having 1 to 6 carbon atoms.

Unless otherwise stated, the term "haloalkyl" as used herein means alkyl as defined herein, in which one or more hydrogens are substituted with identical or different halogen. Examples of the haloalkyl group include, but are not limited to, -CH₂Cl, -CH₂CF₃, -CH₂CCl₃, -CF₂, -CF₃, and the like.

Unless otherwise stated, the term "hydroxyalkyl" as used herein includes the substitution of one or more hydrogen atoms in an alkyl group with one or more hydroxy(-OH), for example, divalent or trivalent hydroxy.

Unless otherwise stated, the term "aminoalkyl" as used herein includes the substitution of one or more hydrogen atoms in an alkyl group with one or more amino(-NH₂), for example, divalent or trivalent amino.

Unless otherwise stated, the term "oxo" as used herein means a group of the chemical formula =O (i.e., oxygen with a double bond).

Unless otherwise stated, the term "alkyl" as used herein, unless otherwise mentioned, refers to a saturated, straight-chain or branched monovalent hydrocarbon radical. for example, the alkyl may be C₁₋₁₀ alkyl, C₁₋₈ alkyl, C₁₋₆ alkyl, or C₁₋₄ alkyl.

Unless otherwise stated, the term "alkenyl" as used herein, unless otherwise mentioned, refers to a monovalent hydrocarbon radical containing at least one carbon-carbon double bond, and each double bond may have an E- or Z-configuration form. for example, the alkenyl may be C₂₋₁₀ alkenyl, C₂₋₈ alkenyl, C₂₋₆ alkenyl, or C₂₋₄ alkenyl.

Unless otherwise stated, the term "alkynyl" as used herein, unless otherwise mentioned, refers to a monovalent group derived from an unsaturated, straight-chain or branched hydrocarbon moiety having at least one carbon-carbon triple bond. for example, the alkynyl may be C₂₋₁₀ alkynyl, C₂₋₈ alkynyl, C₂₋₆ alkynyl, or C₂₋₄ alkynyl.

These "alkyl", "alkenyl" and "alkynyl", when used alone or in combination with other additional terms (for example, haloalkyl), may be straight-chain or branched. According to each definition, it means a radical of a saturated aliphatic hydrocarbon group having 1 to 10, 1 to 9, 1 to 8, 1 to 7, 1 to 6, 1 to 5, 1 to 4, or 1 to 3 carbon atoms within the alkyl group. General examples of alkyl include, but are not limited to, methyl, ethyl, n-propyl, iso-propyl, n-butyl, iso-butyl, sec-butyl, tert-butyl, n-pentyl, iso-pentyl, neo-pentyl and tert-pentyl, 1-methylpentyl, 2-methylbutyl, 1-ethylpropyl, 1,2-dimethylpropyl, n-hexyl, 3,3-dimethylbutyl and isohexyl, and the like. Each of the double bonds of the alkenyl group and the triple bonds of the alkynyl group may be substituted at any position. Examples of alkenyl and alkynyl include, but are not limited to, ethenyl, prop-1-enyl, prop-2-enyl, but-2-enyl, 2-methylprop-2-enyl, 3-methylbut-2-enyl, hex-3-enyl, hex-4-enyl, prop-2-ynyl, but-2-ynyl, but-3-ynyl, hex-4-ynyl or hex-5-ynyl, and the like.

Unless otherwise stated, "cycloalkyl", "cycloalkenyl", or "cycloalkynyl" as used herein refers to a cyclic alkyl which may be substituted or unsubstituted, and refers to a radical of a hydrocarbon forming a single or fused ring having unsaturated, partially or fully saturated (for example, 3 to 24) carbon atoms. Specifically, the cycloalkyl, cycloalkenyl, or cycloalkynyl may have 3 to 10, 3 to 8, 3 to 6, 3 to 5, 4 to 10, 4 to 8, 4 to 6, or 4 to 5 carbon atoms. The cycloalkyl may include carbocyclyl, spirocarbocyclyl, fused carbocyclyl, and bridged carbocyclyl, but is not limited thereto.

According to an embodiment of the present application, the cycloalkyl may be cyclopropyl, cyclobutyl, cyclopentyl, cyclohexyl, cyclohexenyl, cycloheptyl, cycloheptenyl, cyclooctyl, cyclooctenyl, 2,5-cyclohexadienyl, spiro[3.5]nonane, spiro[3.3]heptane, bicyclo[1.1.1]pentane, bicyclo[2.2.2]octyl, adamant-1-yl, decahydronaphthyl, oxocyclohexyl, dioxocyclohexyl, thiocyclohexyl, 2-oxobicyclo[2.2.1]hept-1-enyl, benzene, naphthalenyl, and may include all possible isomers thereof without limitation, but is not limited thereto.

Unless otherwise stated, the term "saturated or unsaturated heterocyclyl" as used herein refers to a substituted or unsubstituted 3- to 24-membered hydrocarbon forming an unsaturated, partially or fully saturated single or fused ring containing one or more heteroatoms, for example, 1 to 8 heteroatoms selected from the group consisting of nitrogen (N), oxygen (O), and sulfur (S). Specifically, the heterocyclyl may be a 3- to 10-membered, 3- to 8-membered, 3- to 6-membered, 4- to 10-membered, 4- to 8-membered, or 4- to 6-membered hydrocarbon having one or more, for example, 1 to 3 heteroatoms. The heterocycle may include heteroaryl, heterocyclyl, heterospirocarbocyclyl, fused heterocyclyl, and bridged heterocyclyl, but is not limited thereto.

According to an embodiment of the present application, the heterocyclyl may include pyrrolidinyl, morpholinyl, pyrrolizidinyl, quinolizidinyl, azaspirooctanyl, piperidinyl, pyrrolidinyl, imidazolinyl, piperazinyl, piperazinyl-1-oxide, morpholinyl, thiamorpholinyl, tetrahydrofuranyl, diazabicyclooctanyl, diazaspirooctanyl, tetrahydropyridinyl, dihydropyridinyl, dihydropyranyl, tetrahydropyranyl, 2-oxa-6-azaspiroheptanyl, azetidinyl, oxazepanyl, 2-oxa-5-azabicyclo[2.2.1]heptanyl, pyridyl, tetrahydrofuranyl, 8-azabicyclo[3.2.1]octanyl, 2-azaspiro[3.3]heptanyl, 2-oxa-7-azaspiro[3.4]octanyl, 2-oxa-7-azaspiro[3.5]nonanyl, 2-azabicyclo[2.2.1]heptanyl, 3-oxa-8-azabicyclo[3.2.1]octanyl, pyrimidinyl, pyrazolyl, oxetane, and similar groups thereto, but is not limited thereto. For example, in the case of C₂₋₁₀ heterocyclyl, the designation of carbon number C₂₋₁₀ refers to a ring size of a 3-membered ring or more containing at least one heteroatom.

In the present application, the term "aryl," unless otherwise mentioned, represents an aromatic group which may be substituted or unsubstituted, and for example, may be 6- to 20-membered. For example, the aryl may include phenyl, biphenyl, naphthyl, toluyl, naphthalenyl, anthracenyl, indenyl, indanyl, or all possible isomers thereof without limitation.

In the present application, the term "heteroaryl," unless otherwise mentioned, refers to a monocyclic or bicyclic or higher aromatic group containing one or more, for example, 1 to 4, 1 to 3, or 1 to 2 heteroatoms selected from O, N, and S, and for example, may be 6- to 20-membered. For example, examples of monocyclic heteroaryl include thiazolyl, oxazolyl, thiophenyl, furanyl, pyrrolyl, imidazolyl, isoxazolyl, pyrazolyl, triazolyl, thiadiazolyl, tetrazolyl, oxadiazolyl, pyridinyl, pyridazinyl, pyrimidinyl, pyrazinyl, quinolinyl, isoquinolinyl, benzimidazolyl, indazolyl, 2-oxa-5-azabicyclo[2.2.1]heptanyl, or similar groups, but are not limited thereto. For example, examples of bicyclic heteroaryl include pyrrolizidinyl, indolyl, indolinyl, benzothiophenyl, benzofuranyl, dihydrobenzofuranyl, benzimidazolyl, benzoxazolyl, benzisoxazolyl, benzothiazolyl, benzothiadiazolyl, benzotriazolyl, quinolinyl, isoquinolinyl, azaspirooctanyl, purinyl, furopyridinyl, or similar groups, but are not limited thereto. For example, examples of tricyclic heteroaryl include thioxanthinyl, carbazolyl, carbolinyl, acridinyl, or similar groups thereto, but are not limited thereto.

Numerical ranges designated by using the term "to" in the present application refer to a range including the numerical values described before and after the term "to" as the lower limit and upper limit, respectively.

The compound of the present application may have an asymmetric carbon center and an asymmetric axis or an asymmetric plane, and thus may exist as all optical and stereoisomers including mixture racemates as well as substantially pure enantiomers such as R and S enantiomers, and all these isomers and mixtures are included in the scope of the present application. Regarding the pure enantiomer, the optical purity of such enantiomer represented by Chemical formula 1 and a pharmaceutically acceptable salt thereof is preferably 60 %ee or more, more preferably 95 %ee or more, and most preferably 98 %ee or more.

The term "ee" refers to enantiomeric excess. For example, in a given compound, one enantiomer may be present in greater quantity than the other, forming an enantiomerically enriched mixture. An enantiomer-rich form is characterized by a single enantiomer concentration exceeding 50% relative to the other enantiomer, typically ≥60%, ≥70%, ≥80%, or ≥90%, or even higher (e.g., >95%, >97%, >99%, >99.5%).

In the present specification, unless otherwise specified for convenience, the compound of Chemical formula 1 is used in a sense including all of the compound of Chemical formula 1, optical isomers, stereoisomers, racemates, isotopologues, solvates, hydrates thereof, and pharmaceutically acceptable salts thereof.

In the present specification, the term "isotopologue" refers to a compound containing one or more atoms with an abnormal ratio of isotopes relative to their natural abundance. For example, isotopologues of a compound may be radiolabeled in which hydrogen atoms may be selected from hydrogen, deuterium, or tritium, and the compound may contain isotopes such as carbon-13 (¹³C) or nitrogen-15 (¹⁵N). and the like.

The compound of Chemical formula 1, an optical isomer, a stereoisomer, a racemate, or an isotopologue thereof according to an embodiment of the present application may form a pharmaceutically acceptable salt. The pharmaceutically acceptable salt includes both addition salts of an acid or a base and stereochemically isomeric forms thereof. The salt is not particularly limited as long as it is any salt that maintains the activity of the parent compound in a subject to be administered and does not cause undesirable effects. Such salts include inorganic and organic salts, and may be, for example, acetic acid, nitric acid, aspartic acid, sulfonic acid, sulfuric acid, maleic acid, glutamic acid, formic acid, succinic acid, phosphoric acid, phthalic acid, tannic acid, tartaric acid, hydrobromic acid, propionic acid, benzenesulfonic acid, benzoic acid, stearic acid, esylic acid, lactic acid, bicarbonic acid, bisulfuric acid, bitartaric acid, oxalic acid, butyric acid, calcium edetate, camsylic acid, carbonic acid, chlorobenzoic acid, citric acid, edetic acid, toluenesulfonic acid, edisylic acid, esylic acid, fumaric acid, gluceptic acid, pamoic acid, gluconic acid, glycollylarsanilic acid, methylnitric acid, polygalacturonic acid, hexylresorcinic acid, malonic acid, hydrabamic acid, hydrochloric acid, hydroiodic acid, hydroxynaphthoic acid, isethionic acid, lactobionic acid, mandelic acid, estolic acid, mucic acid, napsylic acid, muconic acid, p-nitromethanesulfonic acid, hexamic acid, pantothenic acid, monohydrogen phosphoric acid, dihydrogen phosphoric acid, salicylic acid, sulfamic acid, sulfanilic acid, methanesulfonic acid, or theoclic acid. In addition, the form of the basic salt includes, for example, alkali and alkaline earth metal salts such as ammonium salts, lithium salts, sodium salts, potassium salts, magnesium salts, and calcium salts; salts with organic bases such as benzathine, N-methyl-D-glucamine, and hydrabamine salts; and salts with amino acids such as arginine and lysine. Also, the salt form may be converted into a free form by treatment with a suitable base or acid. The term "addition salt" includes solvates that the compound of Chemical formula 1 and salts thereof can form. Such solvates are, for example, hydrates and alcoholates.

The compound according to an embodiment of the present application may also be in the form of a solvate thereof. "solvate" refers to a complex or aggregate formed by one or more solute molecules, i.e., the compound of Chemical formula 1 or a pharmaceutically acceptable salt thereof, and one or more solvent molecules. The solvate may be, for example, a complex or aggregate formed with various solvent molecules such as water, methanol, ethanol, isopropanol, or acetic acid.

A solvate in which water is a solvent molecule is referred to as a "hydrate." Hydrates include compositions containing stoichiometric amounts of water as well as compositions containing variable amounts of water.

The compound according to an embodiment of the present application may also be in the form of a tautomer thereof. The term "tautomer" or "tautomeric form" refers to structural isomers of different energies that are interconvertible through a low energy barrier. Some non-limiting examples of proton tautomers (also known as prototropic tautomers) include interconversion via proton migration, such as keto-enol and imine-enamine isomerization. Valence tautomers include interconversion by reorganization of some of the bonding electrons.

The compound according to an embodiment of the present application may also be in the form of an isotopologue thereof. The term "isotopologue" refers to a compound in which at least one atom for any compound is exchanged with another atom having the same atomic number but having an atomic mass different from the atomic mass generally or predominantly occurring in nature.

Solvates, stereoisomers, tautomers, and isotopologues of the compound of Chemical formula 1 can be prepared from the compound using methods known in the art.

Terms and abbreviations used in the present specification have their original meanings unless otherwise defined.

An example of the present application also provides a method for preparing the compound of Chemical formula 1. Hereinafter, the method for preparing the compound of Chemical formula 1 will be described based on exemplary reaction schemes to aid in understanding the present application. However, a person of ordinary skill in the technical field to which the present application belongs can prepare the compound of Chemical formula 1 by various methods using known compounds or compounds that can be easily prepared therefrom based on the structure of Chemical formula 1, and all such methods should be interpreted as being included in the scope of the present application. Therefore, the description including the following Reaction Scheme 1 and Reaction Scheme 2 related to the method for preparing the compound of Chemical formula 1 merely presents exemplary methods, and the order of unit operations and the like may be selectively changed as necessary, and the scope of the manufacturing method of the present application is not limited thereto.

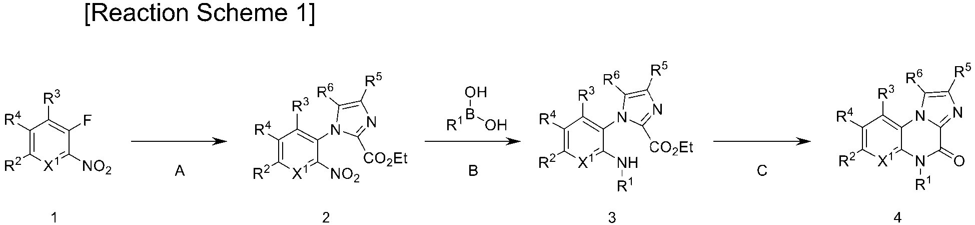

In the reaction scheme 1, X¹ may be CH or N.

For example, the above reaction scheme 1 may be the following reaction scheme 1-1:

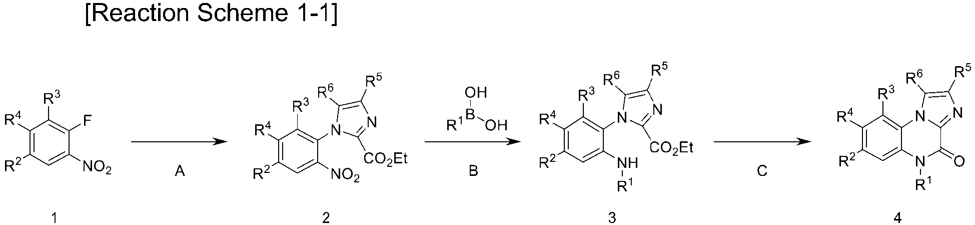

Step A: Compound 2 is obtained by adding ethyl 1H-imidazole-2-carboxylate and cesium carbonate to Compound 1 and applying heat.

Step B: Compound 3 is synthesized from Compound 2 in the presence of a boronic acid having the chemical formula Ar-B(OH)₂, phenyl silane, and a 1,2,2,3,4,4-hexamethylphosphetane 1-oxide catalyst.

Step C: Compound 4 is synthesized using a base such as potassium carbonate in a polar solvent such as ethanol.

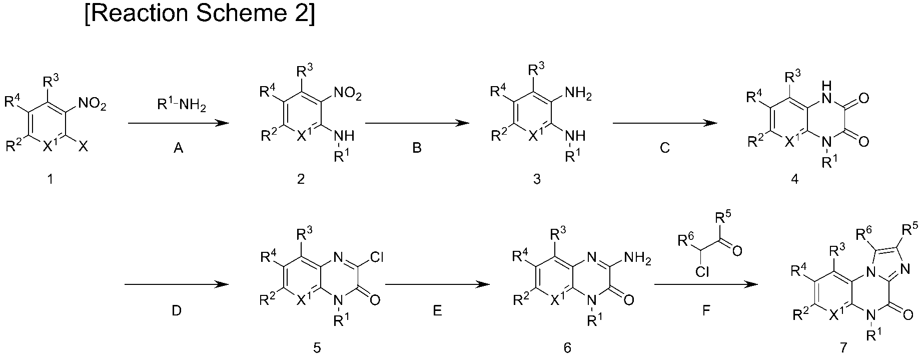

In the reaction scheme 2, X¹ can be CH or N.

For example, the reaction scheme 2 may be following reaction scheme 2-1:

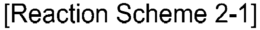

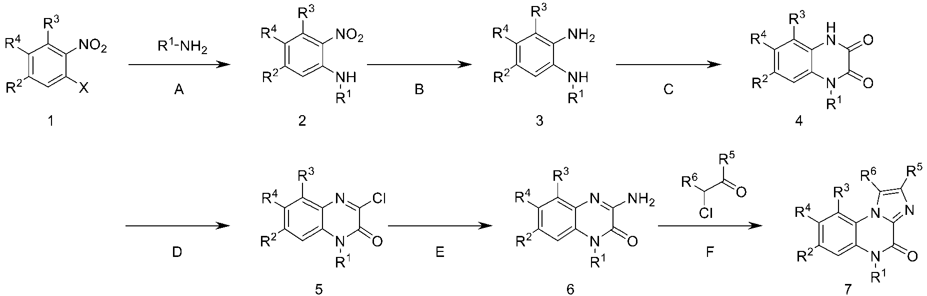

Step A: Compound 2 is obtained by reacting compound 1 with the desired amine under appropriate basic conditions.

Step B: Compound 3 is obtained by reduction from compound 2.

Step C: Compound 4 is obtained by adding diethyl oxalate to compound 3 and heating it, or by adding ethyl 2-chloro-2-oxoacetate under basic conditions.

Step D: Compound 5, in which the hydroxy group is substituted with a chlorine, is obtained by reacting compound 4 with thionyl chloride.

Step E: Compound 6, in which the chlorine substituent of compound 5 is substituted with an amine using ammonia, is obtained.

Step F: Compound 7 is obtained by cyclization of compound 6.

Another embodiment of the present application relates to a pharmaceutical composition comprising a therapeutically effective amount of a compound of Chemical Formula 1 according to an embodiment of the present application, an optical isomer, a stereoisomer, a racemate, an isotopologue, a solvate, a hydrate thereof, or a pharmaceutically acceptable salt thereof, as an active ingredient.

Specifically, the compound of Chemical Formula 1 or the like according to an embodiment of the present application can prevent, improve, or treat a disease or condition related to a MAT2A mutant protein, specifically a disease or condition caused by a MAT2A protein. For example, the compound of Chemical Formula 1 or the like according to the present application is an inhibitor of the MAT2A protein and can effectively inhibit the growth of MTAP-deficient cancer cells, and thus can be usefully used as a pharmaceutical composition for preventing or treating cancer. The cancer may be a cancer with reduced or deficient MTAP activity. Accordingly, the pharmaceutical composition may be a pharmaceutical composition for preventing or treating a disease related to the MAT2A protein.

More specifically, the pharmaceutical composition may prevent or treat a disease related to the MAT2A protein by inhibiting the activity of the MAT2A protein. The disease related to the MAT2A protein may be, for example, cancer. Accordingly, the pharmaceutical composition may act as a selective inhibitor for MTAP-deficient cancer associated with MAT2A, and the pharmaceutical composition may be a composition for preventing or treating MTAP-deficient cancer.

In addition, the pharmaceutical composition can be used to treat cancer by including the compound according to an embodient of the present application, an optical isomer, a stereoisomer, a racemate, an isotopologue, a solvate, a hydrate thereof, or a pharmaceutically acceptable salt thereof, and additionally at least one or more different therapeutic agents. The compound according to an embodiment of the present application, an optical isomer, a stereoisomer, a racemate, an isotopologue, a solvate, a hydrate thereof, or a pharmaceutically acceptable salt thereof may be used in combination with a different therapeutic agent to exhibit a synergistic effect. The term "synergistic" refers to a therapeutic combination that is more effective than the additive effect of two or more single agents. A combination therapy provides "synergy" and proves to be "synergistic", that is, the effect achieved when the active ingredients are used together is greater than the sum of the effects resulting from using the compounds separately. A synergistic effect can be obtained when the active ingredients are: (1) co-formulated in a combined unit dose formulation and administered or delivered simultaneously; or (2) delivered as separate formulations by substitution. When delivered in an alternative therapy, a synergistic effect can be obtained when the compounds are administered or delivered sequentially, for example, by different injections in separate syringes. Generally, during an alternative therapy, the effective dose of each active ingredient is administered sequentially, that is, in a series in time. In some embodiments, synergy is demonstrated by lower toxicity of the combination compared to the same dose of any single component at the same total dose. For example, when the compound of Chemical Formula 1 according to an embodiment of the present application is co-administered with a different therapeutic agent, the toxicity of a 50:50 (w/w) combination comprising the compound of Chemical Formula 1 and the different therapeutic agent may be lower than the toxicity of 100% (w/w) of the compound of Chemical Formula 1, and/or the toxicity of 100% (w/w) of the different therapeutic agent, where the combination has approximately the same level or higher efficacy. That is, the toxicity of the combination of the compound of Chemical Formula 1 and the different therapeutic agent is less than the toxicity of the single component, and the efficacy of the combination is greater than the efficacy of the single component. Furthermore, the reason for combining the compound of Chemical Formula 1 and the different therapeutic agent is not only to reduce toxicity and impart greater safety but also to enhance efficacy to a greater extent than that imparted by a single agent alone. An increase in efficacy is one of the benefits of combination therapy.

Another embodiment of the present application relates to a pharmaceutical combination for treating and/or preventing cancer in a subject, comprising a combination of a compound according to an embodiment of the present application, an optical isomer, a stereoisomer, a racemate, an isotopologue, a solvate, a hydrate thereof, or a pharmaceutically acceptable salt thereof; and a cytotoxic anticancer agent.

Another embodiment of the present application relates to a pharmaceutical combination for treating and/or preventing cancer in a subject, comprising a combination of a compound according to an embodiment of the present application, an optical isomer, a stereoisomer, a racemate, an isotopologue, a solvate, a hydrate thereof, or a pharmaceutically acceptable salt thereof; and a targeted anticancer agent.

The cytotoxic anticancer agent among the active ingredients of the pharmaceutical combination, may include at least one selected from the group consisting of an alkylating agent, an antimetabolite, a DNA topoisomerase inhibitor, and a microtubule inhibitor.

The targeted anticancer agent among the active ingredients of the pharmaceutical combination, may include at least one selected from the group consisting of a monoclonal antibody, a small molecule of targeted therapeutic agent, an antibody-drug conjugate, and an immune checkpoint inhibitor.

The cytotoxic anticancer agent or targeted anticancer agent, which is one of the active ingredients of the pharmaceutical combination according to an embodiment of the present application, may be used in combination with the compound according to an embodiment of the present application, an optical isomer, a stereoisomer, a racemate, an isotopologue, a solvate, a hydrate thereof, or a pharmaceutically acceptable salt thereof, in a weight ratio of 1:0.0001 to 1:10,000, 1:0.0001 to 1:1,000, 1:0.0001 to 1:100, 1:0.001 to 1:10,000, 1:0.001 to 1:1,000, 1:0.001 to 1:100, 1:0.01 to 1:10,000, 1:0.01 to 1:1,000, 1:0.01 to 1:100, 1:0.1 to 1:10,000, 1:0.1 to 1:1,000, or 1:0.1 to 1:100.

An example of the alkylating agent may be one selected from the group consisting of Cisplatin, Carboplatin, Oxaliplatin, Mechlorethamine, Cyclophosphamide, Ifosfamide, Melphalan, Chlorambucil, Carmustine (BCNU), Lomustine (CCNU), Nimustine, Altretamine, Busulfan, Dacarbazine, Procarbazine, Temozolomide, Thiotepa, and Lurbinectedin.

An example of the antimetabolite may be one selected from the group consisting of Pemetrexed, Fluorouracil (5-FU), Capecitabine, Cytarabine, Gemcitabine, Methotrexate, and Mercaptopurine (6-MP).

An example of the DNA topoisomerase inhibitor may be one selected from the group consisting of Etoposide, Teniposide, Topotecan, Irinotecan, SN-38, Dactinomycin, Doxorubicin, Daunorubicin, Mitomycin, and Bleomycin.

An example of the microtubule inhibitor may be one selected from the group consisting of Vinblastine, Vincristine, Vinorelvine, Paclitaxel, and Docetaxel.

An example of the monoclonal antibody may be one selected from the group consisting of Trastuzumab, Rituximab, and Bevacizumab. An example of the small molecule of targeted therapeutic agent may be one selected from the group consisting of kinase inhibitors, hormone receptor antagonists, EGFR/VEGR inhibitors, PARP inhibitors, PI3K/AKT/mTOR pathway inhibitors, and epigenetics inhibitors.

The epigenetics inhibitor may include Type I PRMT inhibitors and Type II PRMT inhibitors, and the type II PRMT inhibitor may be one or more selected from the group consisting of PRMT5 inhibitors, PRMT7 inhibitors, and PRMT9 inhibitors.

The PRMT5 inhibitor may be an inhibitor that inhibits PRMT5 non-competitively and/or cooperatively with MTA.

For example, the MTA-cooperative PRMT5 inhibitor preferentially inhibits PRMT5 in an environment where a high concentration of MTA exists through a mechanism in which the binding capacity for PRMT5 increases in the presence of MTA, and thus, when used together with a MAT2A inhibitor in MTAP-deficient cancer, it can exert a powerful PRMT5 enzyme inhibitory action.

The MTA-cooperative PRMT5 inhibitor may include at least one selected from the group consisting of AMG-193, MRTX1719, TNG908, and TNG462, but is not limited thereto.

An example of the antibody-drug conjugate may be a CD33, CD22, CD20, or Trop2 antibody-drug conjugate, but is not limited thereto.

An example of the immune checkpoint inhibitor may be a CD20 antibody, a CTLA-4 inhibitor, a PD-1/PDL-1 antibody, or a LAG-3 antibody, but is not limited thereto.

In another aspect of the present application, there is provided a method for preventing or treating a disease related to a Methionine Adenosyltransferase 2A (MAT2A) protein, for example, cancer, comprising administering a pharmaceutical composition or a pharmaceutical combination according to an embodiment of the present application to a subject in need thereof. The cancer may be a cancer with reduced MTAP activity or expression, or a cancer in which all or part of the MTAP gene is deleted.

The cancer may be, for example, one or more selected from the group consisting of acoustic neuroma, adenocarcinoma, adrenal cancer, anal cancer, angiosarcoma, benign monoclonal gammopathy, bile duct cancer, bladder cancer, breast cancer, brain cancer, lymphoma, multiple myeloma, lacrimal gland tumor, bronchial cancer, cervical cancer, craniopharyngioma, colorectal cancer, epithelial carcinoma, ependymoma, endothelial sarcoma, endometrial cancer, esophageal cancer, Barrett's adenocarcinoma, Ewing's sarcoma, eye cancer, gallbladder cancer, gastric cancer, colon cancer, gastrointestinal stromal tumor (GIST), head and neck cancer, oral cancer (OSCC), throat cancer, hematologic malignancy, hemangioblastoma, inflammatory myofibroblastic tumor, immunoglobulin light chain (AL) amyloidosis, kidney cancer, renal cell carcinoma, liver cancer, lung cancer, melanoma, uveal melanoma, acute myeloid leukemia (AML), myelodysplastic syndrome, mesothelioma, myeloproliferative disorder (MPD), chronic idiopathic myelofibrosis, chronic myeloid leukemia (CML), chronic neutrophilic leukemia (CNL), neuroblastoma, neurofibroma, neuroendocrine cancer, osteosarcoma, ovarian cancer, papillary adenocarcinoma, pancreatic cancer, penile cancer, prostate cancer, rectal cancer, rhabdomyosarcoma, salivary gland cancer, skin cancer, small intestine cancer, soft tissue sarcoma, thyroid cancer, urethral cancer, vaginal cancer, glioblastoma (GBM), glioma, peripheral nerve sheath tumor, thymic cancer, denoid cystic carcinoma (ACC), and vulvar cancer.

For example, the cancer may be one or more selected from the group consisting of lung cancer, bladder cancer, gastric cancer, esophageal cancer, head and neck cancer, pancreatic cancer, colon cancer, bile duct cancer, mesothelioma, peripheral nerve sheath tumor, thymic cancer, and adenoid cystic carcinoma (ACC).

The brain cancer may be, for example, any one or more selected from the group consisting of meningioma, glioma, medulloblastoma, glioblastoma, and metastatic brain cancer.

The compound of Chemical Formula 1 according to an embodiment of the present application, an optical isomer, a stereoisomer, a racemate, an isotopologue, a solvate, a hydrate thereof, or a pharmaceutically acceptable salt thereof has high binding capacity to the MAT2A protein. Accordingly, another example of the present application relates to a composition for binding to the MAT2A protein, more specifically, a composition for inhibiting the activity of the MAT2A protein, comprising the compound according to an embodiment of the present application, an optical isomer, a stereoisomer, a racemate, an isotopologue, a solvate, a hydrate thereof, or a pharmaceutically acceptable salt thereof.

Another example of the present application relates to a method for inhibiting the activity of the MAT2A protein, comprising administering a pharmaceutical composition or a pharmaceutical combination according to an embodiment of the present application to a subject in need thereof.

### [ADVANTAGEOUS EFFECTS]

The present application can provide a tricyclic compound useful as an agent for the prevention or treatment of cancer caused by MAT2A, a preparation method thereof, a pharmaceutical composition containing the same, and uses thereof as a medicament.

A tricyclic derivative compound exhibiting an excellent MAT2A inhibitory effect according to an embodiment of the present application, an optical isomer thereof, a stereoisomer, a racemate, an isotopologue, a solvate, a hydrate, or a pharmaceutically acceptable salt thereof acts as an MAT2A (methionine adenosyltransferase 2A) inhibitor, and can be effectively used for the prevention or treatment of a disease related to the MAT2A protein, for example, cancer.

In addition, a compound according to an embodiment of the present application, an optical isomer thereof, a stereoisomer, a racemate, an isotopologue, a solvate, a hydrate, or a pharmaceutically acceptable salt thereof may have improved MTAP-deficient cancer selectivity and blood-brain barrier permeability.

### [MODE FOR INVENTION]

Hereinafter, the present application will be described in more detail through examples. However, the examples are intended only to exemplify the present application and are not intended to limit the scope of the present application.

### Example 1: 7-chloro-5-(o-tolyl)imidazo[1,2-a]quinoxalin-4(5H)-one

Reaction scheme for the preparation of compound of Example 1

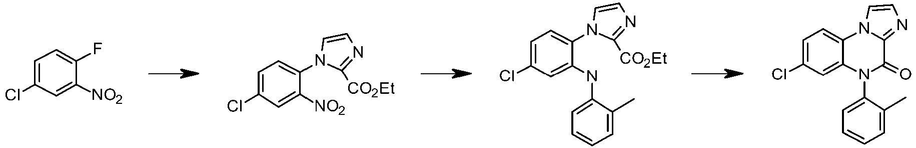

### Example 1-1: Ethyl 1-(4-chloro-2-nitrophenyl)-1H-imidazol-2-carboxylate

4-chloro-1-fluoro-2-nitrobenzene (20.0 g, 113 mmol, 1 eq.) and ethyl 1H-imidazole-2-carboxylate (15.9 g, 113 mmol, 1.00 eq.) were dissolved in dimethylformamide (100 mL), and added with cesium carbonate (48.2 g, 148 mmol, 1.3 eq.). The reaction mixture was stirred at 55 °C for 2 hours. Consumption of the starting material was monitored via TLC, and after cooling to room temperature upon completion of the reaction, 500 mL of water was added, and then the precipitate was filtered, washed with water (30 mL X 3), and dried to obtain ethyl 1-(4-chloro-2-nitrophenyl)-1H-imidazole-2-carboxylate (26.0 g, 84.9 mmol, 74.4% yield, 96.6% purity) as a light yellow solid.

¹H NMR (400 MHz, DMSO-d₆, ppm) δ 8.38 (d, J = 2.4 Hz, 1H), 8.01 (d, J = 8.6 Hz, 1H), 7.75 (d, J = 8.8 Hz, 1H), 7.70 (d, J = 0.8 Hz, 1H), 7.32 (d, J = 1.2 Hz, 1H), 4.12 (q, J = 7.2 Hz, 2H), 1.11 (t, J = 7.2 Hz, 3H).

### Example 1-2: Ethyl 1-(4-chloro-2-(o-tolylamino)phenyl)-1H-imidazol-2-carboxylate

The compound obtained in Example 1-1 (6.00 g, 20.2 mmol, 1 eq.), o-tolylboronic acid (5.52 g, 40.60 mmol, 2 eq.), and 1,2,2,3,4,4-hexamethylphosphetane 1-oxide (530 mg, 3.04 mmol, 0.15 eq.) were dissolved in toluene (45 mL), and were added with phenylsilane (4.39 g, 40.5 mmol, 5.01 mL, 2 eq.). The reaction mixture was purged with nitrogen three times to remove gas in the solution, and then stirred at 120 °C for 2 hours under a nitrogen atmosphere. After cooling the reaction mixture to room temperature, water (100 mL) was added, and the mixture was extracted with ethyl acetate (150 mL × 2). The organic layer was washed with a saturated aqueous sodium chloride solution (100 mL × 2), and then the organic layer was dried over sodium sulfate, filtered, and concentrated. The residue was purified by column chromatography (stationary phase: silica; mobile phase: petroleum ether/ethyl acetate = 50/1 to 10/1) to obtain the title compound in the form of a white solid (4.70 g, 10.5 mmol, 51.9% yield, 79.7% purity).

¹H NMR (400 MHz, DMSO-d₆, ppm) δ 7.49 (d, J = 1.2 Hz, 1H), 7.20-7.18 (m, 2H), 7.16-7.09 (m, 3H), 7.05-7.00 (m, 1H), 6.94 (d, J = 8.0 Hz, 1H), 6.84 (d, J = 8.4 Hz, 1H), 6.47 (d, J = 2.4 Hz, 1H), 4.15 (d, J = 6.8 Hz, 2H), 2.07 (s, 3H), 1.13 (t, J = 7.2 Hz, 3H).

### Example 1-3: 7-chloro-5-(o-tolyl)imidazo[1,2-a]quinoxalin-4(5H)-one

The compound obtained in Example 1-2 (3.10 g, 8.71 mmol, 1 eq.) was dissolved in ethanol (45 mL), and was added with potassium carbonate (1.33 g, 9.60 mmol, 1.1 eq.). The mixture was stirred at 80 °C for 2 hours. After confirming completion of the reaction through TLC, the reaction product was cooled at room temperature and concentrated under reduced pressure. The residue was purified by column chromatography (stationary phase: silica, mobile phase: dichloromethane/methanol = 100/1 to 20/1) to obtain the title compound as a light yellow solid (2.50 g, 7.76 mmol, 89.0% yield, 96.2% purity).

1H NMR (400 MHz, DMSO-d6, ppm) δ 8.68 (d, J = 1.3 Hz, 1H), 8.32 (d, J = 8.8 Hz, 1H), 7.70 (d, J = 1.3 Hz, 1H), 7.63 - 7.44 (m, 4H), 7.38 (d, J = 7.7 Hz, 1H), 6.36 (d, J = 2.2 Hz, 1H), 2.02 (s, 3H).

### Example 2: 7-chloro-5-phenylimidazo[1,2-a]quinoxalin-4(5H)-one

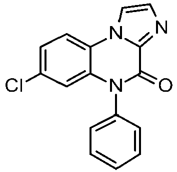

The title compound was synthesized in the same manner in Example 1-2 by changing o-tolyl boronic acid to phenyl boronic acid.

¹H NMR (400 MHz, CDCl₃, ppm) δ 7.89 (d, J = 1.3 Hz, 1H), 7.71 (d, J = 1.2 Hz, 1H), 7.69 - 7.56 (m, 4H), 7.37 - 7.28 (m, 3H), 6.72 (d, J = 2.1 Hz, 1H).

### Example 3: 7-chloro-5-(2-ethylphenyl)imidazo[1,2-a]quinoxalin-4(5H)-one

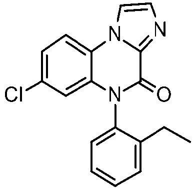

The title compound was synthesized in the same manner in Example 1-2 by changing o-tolyl boronic acid to (2-ethylphenyl) boronic acid.

¹H NMR (400 MHz, CDCl₃, ppm) δ 7.90 (d, J = 1.3 Hz, 1H), 7.72 (d, J = 1.2 Hz, 1H), 7.68 (d, J = 8.7 Hz, 1H), 7.58 - 7.50 (m, 2H), 7.49 - 7.42 (m, 1H), 7.29 (d, J = 2.2 Hz, 1H), 7.18 (d, J = 7.8 Hz, 1H), 6.61 (d, J = 2.2 Hz, 1H), 2.71 - 2.23 (m, 2H), 1.13 (t, J = 7.6 Hz, 3H).

### Example 4: 7-chloro-5-(2,3-dimethylphenyl)imidazo[1,2-a]quinoxalin-4(5H)-one

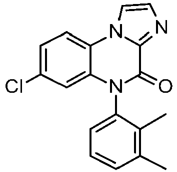

The title compound was synthesized in the same manner in Example 1-2 by changing o-tolyl boronic acid to (2,3-dimethylphenyl) boronic acid.

¹H NMR (400 MHz, CDCl₃, ppm) δ 7.90 (s, 1H), 7.72 (s, 1H), 7.68 (d, J = 8.7 Hz, 1H), 7.42 - 7.28 (m, 3H), 7.04 (d, J = 7.5 Hz, 1H), 6.63 (d, J = 2.1 Hz, 1H), 2.41 (s, 3H), 1.98 (s, 3H).

### Example 5: 7-chloro-5-(2-isopropylphenyl)imidazo[1,2-a]quinoxalin-4(5H)-one

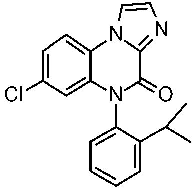

The title compound was synthesized in the same manner in Example 1-2 by changing o-tolyl boronic acid to (2-isopropylphenyl)boronic acid.

¹H NMR (400 MHz, CDCl₃, ppm) δ 7.91 (d, J = 1.3 Hz, 1H), 7.72 (s, 1H), 7.68 (d, J = 8.7 Hz, 1H), 7.63 - 7.54 (m, 2H), 7.47 - 7.38 (m, 1H), 7.29 (d, J = 2.2 Hz, 1H), 7.16 (d, J = 7.8 Hz, 1H), 6.62 (d, J = 2.1 Hz, 1H), 2.79 - 2.52 (m, 1H), 1.20 (d, J = 6.8 Hz, 3H), 1.08 (d, J = 6.9 Hz, 3H).

### Example 6: 7-chloro-5-(3-fluoro-2-methylphenyl)imidazo[1,2-a]quinoxalin-4(5H)-one

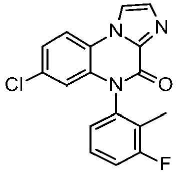

The title compound was synthesized in the same manner in Example 1-2 by changing o-tolyl boronic acid to 3-fluoro-2-methylphenyl)boronic acid.

¹H NMR (400 MHz, CDCl₃, ppm) δ 7.91 (d, J = 1.2 Hz, 1H), 7.73 (d, J = 1.2 Hz, 1H), 7.70 (d, J = 8.7 Hz, 1H), 7.47 - 7.38 (m, 1H), 7.36 - 7.27 (m, 2H), 7.05 (d, J = 7.9 Hz, 1H), 6.62 (d, J = 2.1 Hz, 1H), 2.02 (d, J = 1.9 Hz, 3H).

### Example 7: 7-chloro-5-(2-(trifluoromethyl)phenyl)imidazo[1,2-a]quinoxalin-4(5H)-one

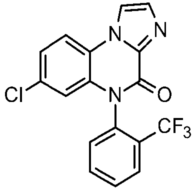

The title compound was synthesized in the same manner in Example 1-2 by changing o-tolyl boronic acid to (2-(trifluoromethyl)phenyl) boronic acid.

¹H NMR (400 MHz, CDCl₃, ppm) δ 7.97 (d, J = 7.8 Hz, 1H), 7.90 (s, 1H), 7.85 (t, J = 7.7 Hz, 1H), 7.75 (t, J = 7.8 Hz, 1H), 7.72 (s, 1H), 7.68 (d, J = 8.7 Hz, 1H), 7.41 (d, J = 7.9 Hz, 1H), 7.29 (dd, J = 8.7, 2.1 Hz, 1H), 6.49 (d, J = 2.1 Hz, 1H).

### Example 8: 7-chloro-5-(4-fluoro-2-methylphenyl)imidazo[1,2-a]quinoxalin-4(5H)-one

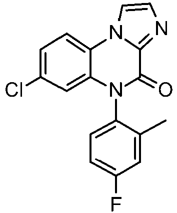

The title compound was synthesized in the same manner in Example 1-2 by changing o-tolyl boronic acid to (4-fluoro-2-methylphenyl) boronic acid.

¹H NMR (400 MHz, CDCl₃, ppm) δ 7.90 (d, J = 1.3 Hz, 1H), 7.72 (d, J = 1.3 Hz, 1H), 7.69 (d, J = 8.7 Hz, 1H), 7.30 (dd, J = 8.6, 2.1 Hz, 1H), 7.23 - 7.10 (m, 3H), 6.62 (d, J = 2.1 Hz, 1H), 2.10 (s, 3H).

### Example 9: 7-chloro-5-(5-fluoro-2-methylphenyl)imidazo[1,2-a]quinoxalin-4(5H)-one

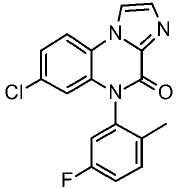

The title compound was synthesized in the same manner in Example 1-2 by changing o-tolyl boronic acid to (5-fluoro-2-methylphenyl) boronic acid.

¹H NMR (400 MHz, CDCl₃, ppm) δ 7.90 (d, J = 1.2 Hz, 1H), 7.72 (d, J = 1.3 Hz, 1H), 7.70 (d, J = 8.7 Hz, 1H), 7.50 - 7.41 (m, 1H), 7.31 (dd, J = 8.7, 2.1 Hz, 1H), 7.24 - 7.17 (m, 1H), 7.00 - 6.95 (m, 1H), 6.62 (d, J = 2.1 Hz, 1H), 2.06 (s, 3H).

### Example 10: 5-(3-fluoro-2-methylphenyl)-7-(trifluoromethyl)imidazo[1,2-a]quinoxalin-4(5H)-one

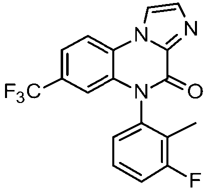

The title compound was synthesized using the same method as Example 6 by using 1-fluoro-2-nitro-4-(trifluoromethyl)benzene as the starting material.

¹H NMR (400 MHz, CDCl₃, ppm) δ 7.98 (d, J = 1.3 Hz, 1H), 7.88 (d, J = 8.5 Hz, 1H), 7.77 (d, J = 1.2 Hz, 1H), 7.59 (d, J = 8.5 Hz, 1H), 7.44 (q, J = 7.8 Hz, 1H), 7.31 (d, J = 8.7 Hz, 1H), 7.06 (d, J = 7.9 Hz, 1H), 6.87 (s, 1H), 2.02 (d, J = 2.0 Hz, 3H).

### Example 11: 7-chloro-5-(3-chloro-2-methylphenyl)imidazo[1,2-a]quinoxalin-4(5H)-one

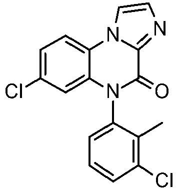

The title compound was synthesized in the same manner in Example 1-2 by changing o-tolyl boronic acid to (3-chloro-2-methylphenyl) boronic acid.

¹H NMR (400 MHz, CDCl₃, ppm) δ 7.91 (d, J = 1.2 Hz, 1H), 7.73 (d, J = 1.3 Hz, 1H), 7.70 (d, J = 8.7 Hz, 1H), 7.60 (d, J = 8.1 Hz, 1H), 7.39 (t, J = 8.1 Hz, 1H), 7.30 (dd, J = 8.7, 2.1 Hz, 1H), 7.15 (d, J = 7.8 Hz, 1H), 6.60 (d, J = 2.1 Hz, 1H), 2.14 (s, 3H).

### Example 12: 7-chloro-5-(2-fluorophenyl)imidazo[1,2-a]quinoxalin-4(5H)-one

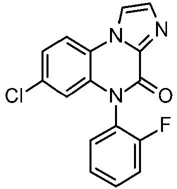

The title compound was synthesized in the same manner in Example 1-2 by changing o-tolyl boronic acid to (2-fluorophenyl) boronic acid.

¹H NMR (400 MHz, CDCl₃, ppm) δ 7.89 (d, J = 1.4 Hz, 1H), 7.72 (d, J = 1.4 Hz, 1H), 7.68 (d, J = 8.7 Hz, 1H), 7.63 - 7.56 (m, 1H), 7.47 - 7.34 (m, 3H), 7.30 (dd, J = 8.7, 2.1 Hz, 1H), 6.74 (d, J = 1.8 Hz, 1H).

### Example 13: 7-cyclopropyl-5-(3-fluoro-2-methylphenyl)imidazo[1,2-a]quinoxalin-4(5H)-one

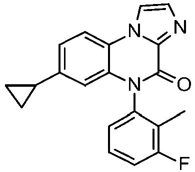

The title compound was synthesized using the same method as Example 6 by changing the starting material to 4-cyclopropyl-1-fluoro-2-nitrobenzene.

¹H NMR (400 MHz, CDCl₃, ppm) δ 7.95 - 7.87 (m, 1H), 7.72 - 7.68 (m, 1H), 7.67 - 7.62 (m, 1H), 7.46 - 7.36 (m, 1H), 7.27 - 7.21 (m, 1H), 7.10 - 7.03 (m, 1H), 6.98 - 6.90 (m, 1H), 6.36 (d, J = 1.6 Hz, 1H), 2.08 - 1.98 (m, 3H), 1.88 - 1.75 (m, 1H), 1.03 - 0.91 (m, 2H), 0.64 - 0.54 (m, 2H).

### Example 14: 5-(3-fluoro-2-methylphenyl)-7-methoxyimidazo[1,2-a]quinoxalin-4(5H)-one

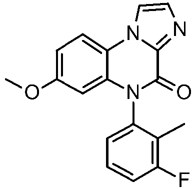

The title compound was synthesized using the same method as Example 6 by changing the starting material to 1-fluoro-4-methoxy-2-nitrobenzene.

¹H NMR (400 MHz, CDCl₃, ppm) δ 7.89 (s, 1H), 7.73 - 7.66 (m, 2H), 7.43 - 7.35 (m, 1H), 7.27 - 7.21 (m, 1H), 7.06 (d, J = 8.0 Hz, 1H), 6.90 - 6.85 (m, 1H), 6.12 (d, J = 2.4 Hz, 1H), 3.72 (s, 3H), 2.03 (q, J = 1.6 Hz, 3H).

### Example 15: 5-(3-fluoro-2-methylphenyl)-7-(trifluoromethoxy)imidazo[1,2-a]quinoxalin-4(5H)-one

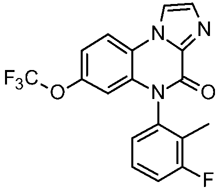

The title compound was synthesized using the same method as Example 6 by changing the starting material to 1-fluoro-2-nitro-4-(trifluoromethoxy)benzene.

¹H NMR (400 MHz, DMSO-d₆, ppm) δ 8.70 (d, J = 1.2 Hz, 1H), 8.41 (d, J = 8.8 Hz, 1H), 7.71 (d, J = 1.2 Hz, 1H), 7.57 - 7.40 (m, 3H), 7.29 (d, J = 8.0 Hz, 1H), 6.38 (d, J = 2.0 Hz, 1H), 1.95 (d, J = 2.0 Hz, 3H).

### Example 16: 7-cyclopropyl-5-(o-tolyl)imidazo[1,2-a]quinoxalin-4(5H)-one

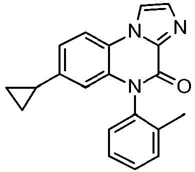

The title compound was synthesized using the same method as Example 1 by changing the starting material to 4-cyclopropyl-1-fluoro-2-nitrobenzene.

¹H NMR (400 MHz, DMSO-d₆, ppm) δ 8.63 (d, J = 1.2 Hz, 1H), 8.18 - 8.08 (m, 1H), 7.68 - 7.62 (m, 1H), 7.57 - 7.43 (m, 3H), 7.38 - 7.31 (m, 1H), 7.07 - 6.97 (m, 1H), 6.24 - 6.11 (m, 1H), 2.01 - 1.97 (m, 3H), 1.86 - 1.74 (m, 1H), 0.94 - 0.86 (m, 2H), 0.56 - 0.46 (m, 2H).

### Example 17: 7-methoxy-5-(o-tolyl)imidazo[1,2-a]quinoxalin-4(5H)-one

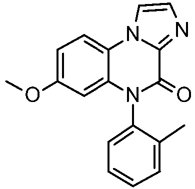

The title compound was synthesized using the same method as Example 1 by changing the starting material to 1-fluoro-4-methoxy-2-nitrobenzene.

¹H NMR (400 MHz, DMSO-d₆, ppm) δ 7.89 (s, 1H), 7.01 (s, 1H), 7.68 (s, 1H), 7.46 - 7.41 (m, 3H), 7.22 (d, 1H), 6.86 (dd, J = 2.4 Hz, 1H), 6.12 (d, J = 2.4 Hz, 1H), 3.71 (s, 3H), 2.13 (s, 3H).

### Example 18: 4-oxo-5-(o-tolyl)-4,5-dihydroimidazo[1,2-a]quinoxalin-7-carbonitrile

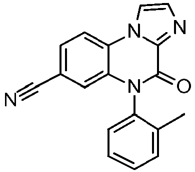

The title compound was synthesized using the same method as Example 1 by changing the starting material to 4-fluoro-3-nitrobenzonitrile.

¹H NMR (400 MHz, CDCl₃, ppm) δ 7.98 (s, 1H), 7.86 (d, J = 8.4 Hz, 1H), 7.78 (s, 1H), 7.65-7.58 (m, 1H), 7.55-7.45 (m, 3H), 7.20 (d, J = 7.6 Hz, 1H), 6.93-6.92 (m, 1H), 2.10 (s, 3H).

### Example 19: 5-(o-tolyl)-7-(trifluoromethoxy)imidazo[1,2-a]quinoxalin-4(5H)-one

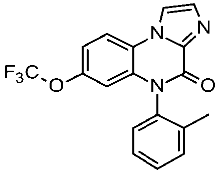

The title compound was synthesized using the same method as Example 1 by changing the starting material to 1-fluoro-2-nitro-4-(trifluoromethoxy)benzene.

¹H NMR (400 MHz, DMSO-d₆, ppm) δ 8.70 (d, J = 1.2 Hz, 1H), 8.41 (d, J = 9.2 Hz, 1H), 7.71 (d, J = 1.2 Hz, 1H), 7.59 - 7.36 (m, 5H), 6.27 (d, J = 1.6 Hz, 1H), 2.04 - 1.98 (m, 3H).

### Example 20: (Ra)-7-chloro-5-(o-tolyl)imidazo[1,2-a]quinoxalin-4(5H)-one

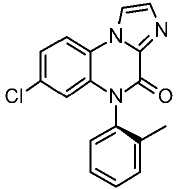

The title compound obtained in Example 1 was separated using DAICEL CHIRALPAK IC (250 mm*20 mm, 5 µm); mobile phase: [hexane-ethanol]; B%: 70%, isocratic transfer conditions (retention time: 8.55 min) to obtain the title compound as a white solid (ee%>99.9%).

¹H NMR (400 MHz, CDCl₃, ppm) 7.90 (s, 1H), 7.72 (s, 1H), 7.69 (d, J = 8.7 Hz, 1H), 7.53 - 7.40 (m, 3H), 7.31 - 7.28 (m, 1H), 7.20 (d, J = 7.6 Hz, 1H), 6.61 (d, J = 2.0 Hz, 1H), 2.10 (s, 3H).

### Example 21: (Sa)-7-chloro-5-(o-tolyl)imidazo[1,2-a]quinoxalin-4(5H)-one

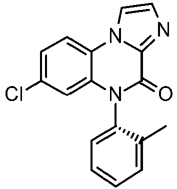

The title compound obtained in Example 1 was separated using DAICEL CHIRALPAK IC (250 mm*20 mm, 5 µm); mobile phase: [hexane-ethanol]; B%: 70%, isocratic transfer conditions (retention time: 10.55 min) (ee%>99.9%) to obtain the title compound as a white solid.

¹H NMR (400 MHz, CDCl₃, ppm) δ 7.90 (d, J = 1.3 Hz, 1H), 7.72 (d, J = 1.2 Hz, 1H), 7.69 (d, J = 8.7 Hz, 1H), 7.51 - 7.46 (m, 2H), 7.46 - 7.41 (m, 1H), 7.32 - 7.27 (m, 1H), 7.20 (d, J = 7.6 Hz, 1H), 6.61 (d, J = 2.1 Hz, 1H), 2.10 (s, 3H).

### Example 22: 7-(difluoromethyl)-5-(o-tolyl)imidazo[1,2-a]quinoxalin-4(5H)-one

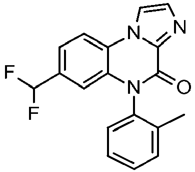

The title compound was synthesized using the same method as Example 1 by changing the starting material to 4-(difluoromethyl)-1-fluoro-2-nitrobenzene.

¹H NMR (400 MHz, CDCl₃, ppm) δ 7.97 (d, J = 1.3 Hz, 1H), 7.85 (d, J = 8.4 Hz, 1H), 7.74 (d, J = 1.3 Hz, 1H), 7.64 - 7.37 (m, 4H), 7.22 (d, J = 7.6 Hz, 1H), 6.74 (s, 1H), 6.54 (t, J = 56.1 Hz, 1H), 2.10 (s, 3H).

### Example 23: 7-chloro-5-(2-chlorophenyl)imidazo[1,2-a]quinoxalin-4(5H)-one

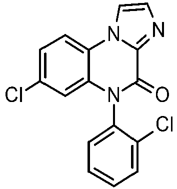

The title compound was synthesized in the same manner as Example 1-2 by changing o-tolyl boronic acid to (2-chlorophenyl) boronic acid.

¹H NMR (400 MHz, CDCl₃, ppm) δ 7.90 (d, J = 1.3 Hz, 1H), 7.72 (d, J = 1.2 Hz, 1H), 7.71 - 7.66 (m, 2H), 7.61 - 7.50 (m, 2H), 7.43 - 7.38 (m, 1H), 7.30 (dd, J = 8.7, 2.1 Hz, 1H), 6.59 (d, J = 2.1 Hz, 1H).

### Example 24: 7-acetyl-5-(o-tolyl)imidazo[1,2-a]quinoxalin-4(5H)-one

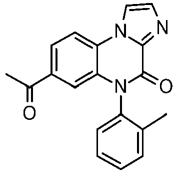

The title compound was synthesized using the same method as Example 1 by changing the starting material to 1-(4-fluoro-3-nitrophenyl)ethan-1-on.

¹H NMR (400 MHz, CDCl₃, ppm) δ 7.97 (d, J = 1.3 Hz, 1H), 7.90 (dd, J = 8.4, 1.7 Hz, 1H), 7.83 (d, J = 8.5 Hz, 1H), 7.75 (d, J = 1.3 Hz, 1H), 7.53 - 7.48 (m, 2H), 7.47 - 7.41 (m, 1H), 7.24 - 7.19 (m, 2H), 2.47 (s, 3H), 2.10 (s, 3H).

### Example 25: 7-Iodo-5-(o-tolyl)imidazo[1,2-a]quinoxalin-4(5H)-one

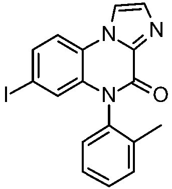

The title compound was synthesized using the same method as Example 1 by changing the starting material to 1-fluoro-4-iodo-2-nitrobenzene.

¹H NMR (400 MHz, CDCl₃, ppm) δ 7.90 (d, J = 1.3 Hz, 1H), 7.71 (d, J = 1.3 Hz, 1H), 7.62 (dd, J = 8.6, 1.7 Hz, 1H), 7.51 - 7.41 (m, 4H), 7.19 (d, J = 7.6 Hz, 1H), 6.93 (d, J = 1.7 Hz, 1H), 2.10 (s, 3H).

### Example 26: 5-(o-tolyl)-7-(trifluoromethyl)imidazo[1,2-a]quinoxalin-4(5H)-one

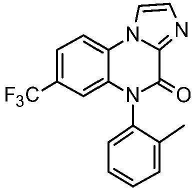

The title compound was synthesized using the same method as Example 1 by changing the starting material to 1-fluoro-2-nitro-4-(trifluoromethyl)benzene.

¹H NMR (400 MHz, CDCl₃, ppm) δ 7.97 (d, J = 1.3 Hz, 1H), 7.87 (d, J = 8.5 Hz, 1H), 7.76 (d, J = 1.3 Hz, 1H), 7.57 (d, J = 8.5 Hz, 1H), 7.53 - 7.48 (m, 2H), 7.46 (dd, J = 7.9, 3.8 Hz, 1H), 7.21 (d, J = 7.7 Hz, 1H), 6.87 (s, 1H), 2.10 (s, 3H).

### Example 27: 5-(3-fluoro-2-methylphenyl)-4-oxo-4,5-dihydroimidazo[1,2-a]quinoxalin-7-carbonitrile

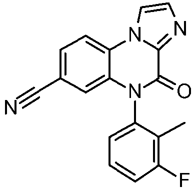

The title compound was synthesized using the same method as Example 6 by changing the starting material to 4-fluoro-3-nitrobenzonitrile.

1H NMR (400 MHz, CDCl₃, ppm) δ 7.98 (s, 1H), 7.88 (d, J = 8.4 Hz, 1H), 7.81 - 7.76 (m, 1H), 7.66 - 7.60 (m, 1H), 7.50 - 7.42 (m, 1H), 7.36 - 7.29 (m, 1H), 7.07 - 7.02 (m, 1H), 6.96 - 6.92 (m, 1H), 2.03 (s, 3H).

### Example 28: 5-(o-tolyl)-7-(2,2,2-trifluoroethoxy)imidazo[1,2-a]quinoxalin-4(5H)-one

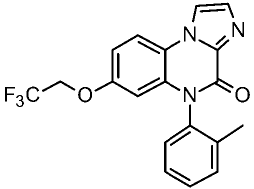

The title compound was synthesized using the same method as Example 1 by changing the starting material to 1-fluoro-2-nitro-4-(2,2,2-trifluoroethoxy)benzene.

¹H NMR (400 MHz, CDCl₃, ppm): δ 7.90 (d, J = 0.8 Hz, 1H), 7.73 (d, J = 9.2 Hz, 1H), 7.71 (s, 1H), 7.51 - 7.48 (m, 2H), 7.47 - 7.41 (m, 1H), 7.24 - 7.19 (m, 1H), 6.92 - 6.86 (m, 1H), 6.19 (d, J = 2.4 Hz, 1H), 4.28 - 4.19 (m, 2H), 2.11 (s, 3H).

### Example 29: 5-(3-fluoro-2-methylphenyl)-7-(2,2,2-trifluoroethoxy)imidazo[1,2-a]quinoxalin-4(5H)-one

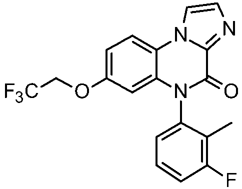

The title compound was synthesized using the same method as Example 6 by changing the starting material to 1-fluoro-2-nitro-4-(2,2,2-trifluoroethoxy)benzene.

¹H NMR (400 MHz, CDCl₃, ppm) δ 7.94 - 7.88 (m, 1H), 7.77 - 7.70 (m, 2H), 7.46 - 7.38 (m, 1H), 7.32 - 7.27 (m, 1H), 7.06 (d, J = 8.0 Hz, 1H), 6.95 - 6.88 (m, 1H), 6.21 (d, J = 2.4 Hz, 1H), 4.31 - 4.22 (m, 2H), 2.07 - 2.00 (m, 3H).

### Example 30: 7-ethoxy-5-(o-tolyl)imidazo[1,2-a]quinoxalin-4(5H)-one

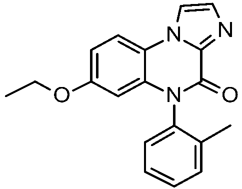

The title compound was synthesized using the same method as Example 1 by changing the starting material to 4-ethoxy-1-fluoro-2-nitrobenzene.

¹H NMR (400 MHz, DMSO-d₆, ppm) δ 8.79 (d, J = 1.6 Hz, 1H), 8.30 (d, J = 9.2 Hz, 1H), 7.91 (d, J = 1.6 Hz, 1H), 7.57 - 7.45 (m, 3H), 7.36 - 7.32 (m, 1H), 7.09 - 7.05 (m, 1H), 5.88 (d, J = 2.4 Hz, 1H), 3.95 - 3.90 (m, 2H), 2.05 - 2.01 (m, 3H), 1.24 (t, J = 6.8 Hz, 3H).

### Example 31: 7-ethoxy-5-(3-fluoro-2-methylphenyl)imidazo[1,2-a]quinoxalin-4(5H)-one

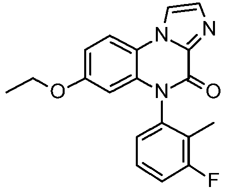

The title compound was synthesized using the same method as Example 6 by changing the starting material to 4-ethoxy-1-fluoro-2-nitrobenzene
¹H NMR (400 MHz, DMSO-d₆, ppm) δ 8.61 (d, J = 1.6 Hz, 1H), 8.30 (d, J = 9.2 Hz, 1H), 7.91 (d, J = 1.6 Hz, 1H), 7.57 - 7.45 (m, 2H), 7.36 - 7.32 (m, 1H), 7.09 - 7.05 (m, 1H), 5.88 (d, J = 2.4 Hz, 1H), 3.95 - 3.90 (m, 2H), 2.05 - 2.01 (m, 3H), 1.24 (t, J = 6.8 Hz, 3H).

### Example 32: 7-bromo-5-(o-tolyl)imidazo[1,2-a]quinoxalin-4(5H)-one

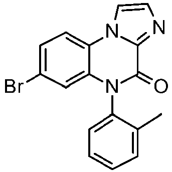

The title compound was synthesized using the same method as Example 1 by changing the starting material to 4-bromo-1-fluoro-2-nitrobenzene.

¹H NMR (400 MHz, CDCl₃, ppm) δ 7.90 (d, J = 1.4 Hz, 1H), 7.72 (d, J = 1.3 Hz, 1H), 7.62 (d, J = 8.7 Hz, 1H), 7.55 - 7.39 (m, 4H), 7.20 (d, J = 7.6 Hz, 1H), 6.76 (d, J = 2.0 Hz, 1H), 2.10 (s, 3H).

### Example 33: 7-chloro-5-(2-methylpyridin-3-yl)imidazo[1,2-a]quinoxalin-4(5H)-one

Reaction scheme for preparing compound of Example 33:

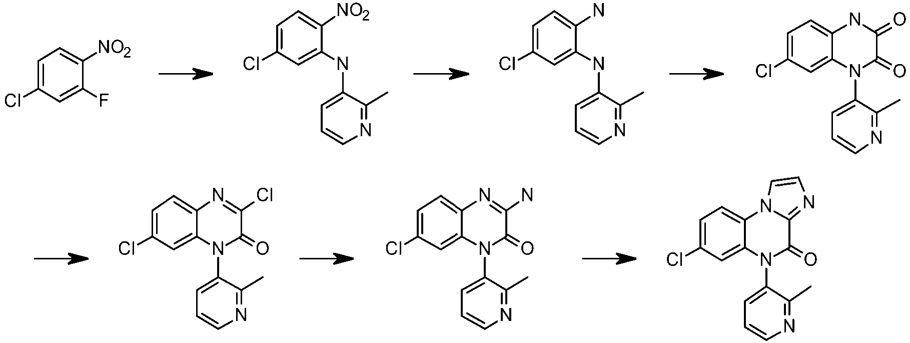

### Example 33-1: N-(5-chloro-2-nitrophenyl)-2-methylpyridin-3-amine

4-chloro-2-fluoro-1-nitrobenzene (10 g, 56.97 mmol, 1 eq.), 2-methylpyridin-3-amine (6.47 g, 59.81 mmol, 1.05 eq.), and lithium bis(trimethylsilyl)amide (1 M, 68.36 mL, 1.2 eq.) were added to tetrahydrofuran (200 mL) and purged with nitrogen three times. The mixture was stirred at -60 °C for 2 hours under nitrogen. After checking the formation of a new product by TLC, the reaction was terminated by adding a saturated aqueous ammonium chloride solution and then extracted with ethyl acetate (200 mL X 3). The organic layer was washed with a saturated aqueous chloride chloride solution (100 mL), dried over ammonium sulfate, filtered, and concentrated. The resulting concentrate was purified by column chromatography (stationary phase: silica, petroleum ether/ethyl acetate=1/0 to 3/1) to obtain the title compound (15 g, 56.89 mmol, 99.8% yield) as a yellow solid.

¹H NMR (400 MHz, CDCl₃, ppm) δ 9.38 (br s, 1H), 8.52 (d, J = 4.0 Hz, 1H), 8.21 (d, J = 9.2 Hz, 1H), 7.62 (d, J = 8.0 Hz, 1H), 7.31 - 7.28 (m, 1H), 6.81 - 6.76 (m, 1H), 6.72 (d, J = 2.0 Hz, 1H), 2.54 (s, 3H).

### Example 33-2: 5-chloro-N¹-(2-methylpyridin-3-yl)benzen-1,2-diamine

The compound obtained in Example 33-1 (15 g, 56.89 mmol, 1 eq.) was dissolved in methanol (300 mL), and Raney-Nickel (4.87 g, 56.89 mmol, 1 eq.) was added. The reaction mixture was stirred at 25 °C for 2 hours under hydrogen (15 Psi). The starting material disappeared and the m/z([M + H]+= 234.1) of the title compound was observed on LCMS, and thus, the reaction mixture was filtered and concentrated to obtain the title compound (13 g, crude mixture) as a yellow solid.

¹H NMR (400 MHz, DMSO-*d*₆, ppm): δ 7.95 - 7.86 (m, 1H), 7.08 - 7.01 (m, 1H), 6.91 - 6.87 (m, 1H), 6.85 (s, 1H), 6.80 (d, J = 2.4 Hz, 1H), 6.74 (d, J = 8.4 Hz, 2H), 5.39 - 4.56 (m, 2H), 2.44 (s, 3H).

### Example 33-3: 7-chloro-1-(2-methylpyridin-3-yl)-1,4-dihydroquinoxalin-2,3-dione

The compound obtained in Example 33-2 (12 g, 51.35 mmol, 1 eq.) was dissolved in diethyl oxalate (128.40 g, 878.60 mmol, 120.00 mL, 17.11 eq.) and stirred at 140 °C for 6 hours. The m/z([M + H]+= 288.5) of the title compound was observed on LCMS. The reaction mixture was concentrated and purified by chromatography (stationary phase: silica, mobile phase: dichloromethane:methanol = 1/0 to 10/1), and then solidified with ethyl acetate (100 mL). The title compound (11.5 g, 39.57 mmol, 77.0% yield, 99% purity) was obtained in the form of a yellow solid.

¹H NMR (400 MHz, DMSO-*d*₆, ppm): δ 12.30 (s, 1H), 8.79 - 8.50 (m, 1H), 7.87 - 7.74 (m, 1H), 7.61 - 7.47 (m, 1H), 7.33 - 7.16 (m, 2H), 6.18 (d, J = 1.2 Hz, 1H), 2.25 (s, 3H).

### Example 33-4: 3,7-dichloro-1-(2-methylpyridin-3-yl)quinoxalin-2(1H)-one

The compound obtained in Example 33-3 (1 g, 3.48 mmol, 1 eq.), phosphoryl chloride (1.60 g, 10.43 mmol, 969.01 uL, 3 eq.), and N,N-dimethylaniline (1.26 g, 10.43 mmol, 1.32 mL, 3 eq) were dissolved in toluene (10 mL) and stirred at 110 °C for 12 hours under nitrogen. The reaction mixture was concentrated, and the title compound (1 g, crude mixture) in the form of a yellow oil was used in the next reaction without further purification.

### Example 33-5: 3-amino-7-chloro-1-(2-methylpyridin-3-yl)quinoxalin-2(1H)-one

The compound obtained in Example 33-4 (1 g, 3.27 mmol, 1 eq.) was dissolved in tetrahydrofuran (1 mL), and an aqueous ammonia solution (3.82 g, 32.66 mmol, 4.19 mL, 30% purity, 10 eq.) was added. The reactant was stirred at 25 °C for 12 hours. On LCMS, the m/z([M + H]+ = 287.3) of the title compound was observed, so the reaction mixture was concentrated under reduced pressure and then filtered. The filter cake was concentrated to obtain the title compound (936 mg, crude mixture) in the form of a yellow solid.

### Example 33-6: 7-chloro-5-(2-methylpyridin-3-yl)imidazo[1,2-a]quinoxalin-4(5H)-one

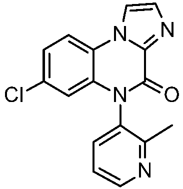

The compound obtained in Example 34-5 (300 mg, 1.05 mmol, 1 eq.) and 2-chloroacetaldehyde (164.27 mg, 2.09 mmol, 134.65 uL, 2 eq.) were added to ethanol (3 mL). The reaction mixture was stirred at 80 °C for 12 hours. The desired m/z([M + H]+ = 311.3) was detected on LCMS. The reaction mixture was concentrated under reduced pressure and purified by prep-HPLC (column: 3_Phenomenex Luna C18 75*30mm*3µm; mobile phase: [water(0.1% ammonium hydrocarbon)-acetonitrile], B%: 41%-71%, 10 minutes). The purified product was neutralized with an aqueous sodium bicarbonate solution and then extracted to obtain the title compound (140 mg, 450.09 µmol, 43.0% yield, 99.9% purity) in the form of a yellow solid.

¹H NMR (400 MHz, DMSO-*d*₆, ppm): δ 8.72 - 8.67 (m, 2H), 8.33 (d, J = 8.8 Hz, 1H), 7.92 - 7.85 (m, 1H), 7.71 (d, J = 1.2 Hz, 1H), 7.57 - 7.46 (m, 2H), 6.43 (d, J = 2.0 Hz, 1H), 2.25 (s, 3H).

### Example 34: 7-chloro-1-methyl-5-(2-methylpyridin-3-yl)imidazo[1,2-a]quinoxalin-4(5H)-one

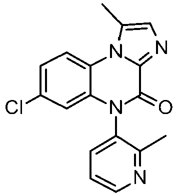

The title compound was synthesized using the same method as Example 33-6 by changing 2-chloroacetaldehyde to 2-chloropropanal.

¹H NMR (400 MHz, DMSO-*d*₆, ppm): δ 8.69 (br d, J = 4.0 Hz, 1H), 8.29 (d, J = 8.8 Hz, 1H), 7.87 (d, J = 7.6 Hz, 1H), 7.57 - 7.50 (m, 1H), 7.48 - 7.36 (m, 2H), 6.44 (d, J = 2.0 Hz, 1H), 2.86 (s, 3H), 2.23 (s, 3H).

### Example 35: 7-chloro-5-(o-tolyl)imidazo[1,2-a]pyrido[2,3-e]pyrazin-4(5H)-one

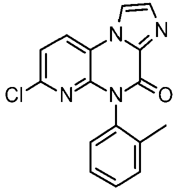

The title compound was synthesized using the same method as Example 33 by changing the starting material to 2,6-dichloro-3-nitropyridin.

¹H NMR (400 MHz, DMSO-*d*₆, ppm): δ 8.72 (d, J = 8.4 Hz, 1H), 8.69 (d, J = 1.2 Hz, 1H), 7.73 - 7.70 (m, 1H), 7.55 (d, J = 8.4 Hz, 1H), 7.46 - 7.33 (m, 3H), 7.32 - 7.27 (m, 1H), 2.00 (s, 3H).

### Example 36: 7-methyl-5-(o-tolyl)imidazo[1,2-a]pyrido[2,3-e]pyrazin-4(5H)-one

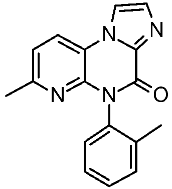

The title compound of Example 35 (400 mg, 1.29 mmol, 1 eq.), methylboronic acid (154.11 mg, 2.57 mmol, 2 eq.), Pd(dppf)Cl₂ (94.19 mg, 128.73 µmol, 0.1 eq.), and cesium carbonate (1.05 g, 3.22 mmol, 2.5 eq.) were dissolved in dioxane (3.2 mL) and water (0.8 mL), and the mixture was stirred at 100°C for 12 hours under nitrogen. After confirming the reaction, the mixture was cooled to 15°C, quenched with 10 mL of water, and extracted with ethyl acetate. The extracted organic layer was washed with a saturated sodium chloride solution, dried over anhydrous sodium sulfate, filtered, and concentrated. Purification was performed by Prep-HPLC (column: Waters Xbridge 150*25mm* 5µm; mobile phase: [water (10 mM ammonium carbonate)-ACN]; B%: 26%-56%, 8 minutes) to obtain 110 mg of a white solid.

¹H NMR (400 MHz, DMSO-*d*₆, ppm): δ 8.64 (d, J = 1.2 Hz, 1H), 8.55 (d, J = 8.0 Hz, 1H), 7.68 (d, J = 1.2 Hz, 1H), 7.44 - 7.31 (m, 3H), 7.31 - 7.23 (m, 2H), 2.31 (s, 3H), 1.97 (s, 3H).

### Example 37: 7-chloro-5-(2-methoxyphenyl)imidazo[1,2-a]quinoxalin-4(5H)-one

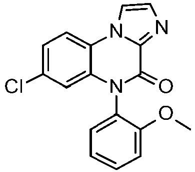

The title compound was synthesized using the same method as Example 33-1 by using 2-methoxyaniline instead of 2-methylpyridin-3-amine.

¹H NMR (400 MHz, DMSO-*d*₆, ppm): δ 8.68 (d, J = 1.2 Hz, 1H), 8.32 - 8.26 (m, 1H), 7.70 (d, J = 1.2 Hz, 1H), 7.65 - 7.59 (m, 1H), 7.49 - 7.41 (m, 2H), 7.37 - 7.32 (m, 1H), 7.24 - 7.19 (m, 1H), 6.46 - 6.45 (m, 1H), 3.72 (s, 3H).

### Example 38: 5-(2-bromophenyl)-7-chloroimidazo[1,2-a]quinoxalin-4(5H)-one

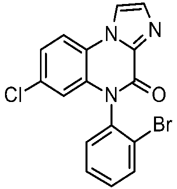

The title compound was synthesized using the same method as Example 1-2 by changing o-tolyl boronic acid to (2-bromophenyl) boronic acid.

¹H NMR (400 MHz, CDCl₃, ppm): δ 7.90 (d, J = 1.3 Hz, 1H), 7.87 (d, J = 8.0 Hz, 1H), 7.72 (s, 1H), 7.69 (d, J = 8.7 Hz, 1H), 7.59 (t, J = 7.7 Hz, 1H), 7.48 (t, J = 7.8 Hz, 1H), 7.43 - 7.37 (m, 1H), 7.30 (dd, J = 8.7, 2.2 Hz, 1H), 6.57 (d, J = 2.1 Hz, 1H).

### Example 39: 7-chloro-5-(m-tolyl)imidazo[1,2-a]quinoxalin-4(5H)-one

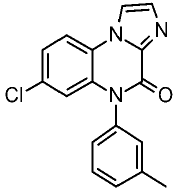

The title compound was synthesized using the same method as Example 1-2 by changing o-tolyl boronic acid to m-tolyl boronic acid.

¹H NMR (400 MHz, CDCl₃, ppm): δ 7.88 (d, J = 1.3 Hz, 1H), 7.71 (d, J = 1.3 Hz, 1H), 7.67 (d, J = 8.7 Hz, 1H), 7.52 (t, J = 7.7 Hz, 1H), 7.38 (d, J = 7.7 Hz, 1H), 7.30 - 7.26 (m, 1H), 7.11 (d, J = 8.2 Hz, 2H), 6.74 (d, J = 2.1 Hz, 1H), 2.46 (s, 3H).

### Example 40: 7-chloro-5-(3-fluorophenyl)imidazo[1,2-a]quinoxalin-4(5H)-one

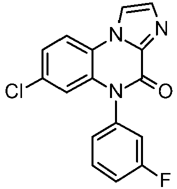

The title compound was synthesized using the same method as Example 1-2 by changing o-tolyl boronic acid to (3-fluorophenyl)boronic acid.

¹H NMR (400 MHz, CDCl₃, ppm): δ 7.90 (d, J = 1.3 Hz, 1H), 7.72 (d, J = 1.3 Hz, 1H), 7.69 (d, J = 8.7 Hz, 1H), 7.63 (td, J = 8.2, 6.1 Hz, 1H), 7.37 - 7.28 (m, 2H), 7.19 - 7.12 (m, 1H), 7.09 (dt, J = 8.7, 2.2 Hz, 1H), 6.73 (d, J = 2.1 Hz, 1H).

### Example 41: 7-fluoro-5-(o-tolyl)imidazo[1,2-a]quinoxalin-4(5H)-one

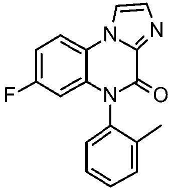

The title compound was synthesized using the same method as Example 1 by changing the starting material to 1,4-difluoro-2-nitrobenzene.

¹H NMR (400 MHz, CDCl₃, ppm): δ 7.89 (d, J = 1.3 Hz, 1H), 7.77 - 7.62 (m, 2H), 7.55 - 7.39 (m, 3H), 7.21 (d, J = 7.5 Hz, 1H), 7.03 (t, J = 6.9 Hz, 1H), 6.35 (dd, J = 10.1, 2.7 Hz, 1H), 2.10 (s, 3H).

### Example 42: 3-chloro-5-(o-tolyl)imidazo[1,2-a]pyrido[4,3-e]pyrazin-6(5H)-one

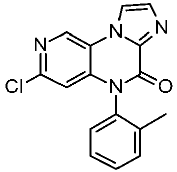

The title compound was synthesized using the same method as Example 33 by changing the starting material to 2,4-dichloro-5-nitropyridin.

¹H NMR (400 MHz, DMSO-d₆, ppm): δ 9.38 - 9.26 (m, 1H), 8.82 - 8.66 (m, 1H), 7.83 - 7.70 (m, 1H), 7.60 - 7.45 (m, 3H), 7.43 - 7.34 (m, 1H), 6.33 - 6.24 (m, 1H), 2.05 (s, 3H).

### Example 43: 2-(7-chloro-4-oxoimidazo[1,2-a]quinoxalin-5(4H)-yl)benzonitrile

Reaction scheme for preparing the compound of Example 43:

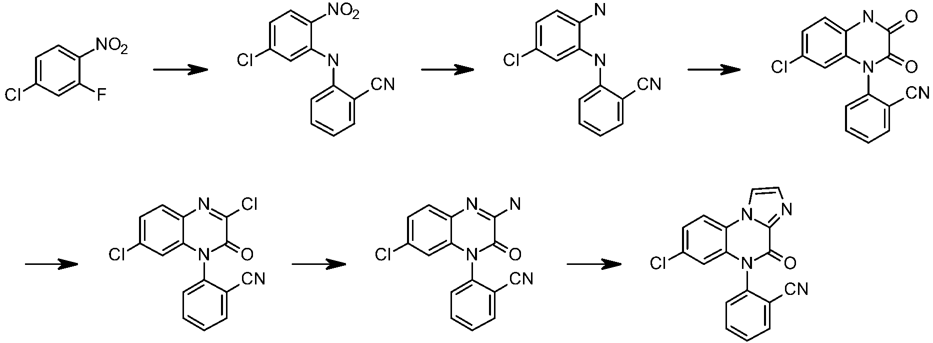

### Example 43-1: 2-((5-chloro-2-nitrophenyl)amino)benzonitrile

4-chloro-2-fluoro-1-nitrobenzene (500 mg, 2.85 mmol, 1 eq.), 2-aminobenzonitrile (606.04 mg, 5.13 mmol, 1.8 eq., and lithium hydroxide monohydrate (215.27 mg, 5.13 mmol, 1.8 eq.) were dissolved in dimethyl sulfoxide (5 mL), and the mixture was stirred at 55 °C under nitrogen for 12 hours. When the formation of a new substance was confirmed through TLC, water (50 mL) was added to the reactant, and extraction was performed with ethyl acetate (50 mL X 3). The organic layer was washed with a saturated aqueous sodium chloride solution (50 mL), dried over anhydrous sodium sulfate, filtered, and concentrated under reduced pressure. The concentrate was purified by column chromatography (stationary phase: silica, mobile phase: petroleum ether/ethyl acetate = 1/0 to 3/1) to obtain the title compound (500 mg, 1.83 mmol, 64.1% yield) in the form of a yellow solid.

¹H NMR (400 MHz, DMSO-*d*₆, ppm): δ 9.69 (br s, 1H), 8.22 (d, J = 9.2 Hz, 1H), 7.79 - 7.74 (m, 1H), 7.71 - 7.65 (m, 1H), 7.52 (d, J = 8.4 Hz, 1H), 7.38 - 7.32 (m, 1H), 7.15 (d, J = 2.0 Hz, 1H), 6.93 - 6.89 (m, 1H).

### Example 43-2: 2-((2-amino-5-chlorophenyl)amino)benzonitrile

The compound obtained in Example 43-1 (400 mg, 1.46 mmol, 1 eq.) was dissolved in ethanol (10 mL) and water (2 mL), and iron (652.98 mg, 11.69 mmol, 8 eq.) and ammonium chloride (312.73 mg, 5.85 mmol, 4 eq.) were added thereto. The reactant was stirred at 80 °C for 12 hours. The desired m/z ([M + H]+ = 244.4) was detected on LCMS. The reaction mixture was filtered, and the filtrate was concentrated under reduced pressure. The title compound (350 mg, crude mixture) in the form of a yellow solid was used in the next reaction without further purification.

### Example 43-3: 2-(7-chloro-2,3-dioxo-3,4-dihydroquinoxalin-1(2H)-yl)benzonitrile

The title compound was obtained in the same manner as in Example 33-3 using the compound obtained in Example 43-2 as a starting material.

¹H NMR (400 MHz, DMSO-*d*₆, ppm): δ 12.44 (br s, 1H), 8.22 - 8.17 (m, 1H), 8.08 - 7.99 (m, 1H), 7.86 - 7.80 (m, 1H), 7.73 (d, J = 8.0 Hz, 1H), 7.38 - 7.20 (m, 2H), 6.27 (s, 1H).

### Example 43-4: 2-(3,7-dichloro-2-oxoquinoxalin-1(2H)-yl)benzonitrile

The title compound was obtained in the same manner as in Example 33-4 using the compound obtained in Example 43-3 as a starting material.

### Example 43-5: 2-(3-amino-7-chloro-2-oxoquinoxalin-1(2H)-yl)benzonitrile

The title compound was obtained in the same manner as in Example 33-5 using the compound obtained in Example 43-4 as a starting material.

### Example 43-6: 2-(7-chloro-4-oxoimidazo[1,2-a]quinoxalin-5(4H)-yl)benzonitrile

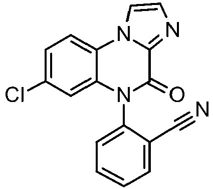

The title compound was obtained in the same manner as in Example 33-6 using the compound obtained in Example 43-5 as a starting material.

¹H NMR (400 MHz, DMSO-d₆, ppm): δ 8.74 (s, 1H), 8.37 (d, J = 8.8 Hz, 1H), 8.25 - 8.19 (m, 1H), 8.09 - 8.02 (m, 1H), 7.86 (t, J = 7.6 Hz, 1H), 7.82 (d, J = 8.0 Hz, 1H), 7.76 (s, 1H), 7.58 - 7.51 (m, 1H), 6.52 (d, J = 2.0 Hz, 1H).

### Example 44: N-(3-(7-chloro-4-oxoimidazo[1,2-a]quinoxalin-5(4H)-yl)phenyl)methanesulfonamide

Reaction scheme for preparing the compound of Example 44:

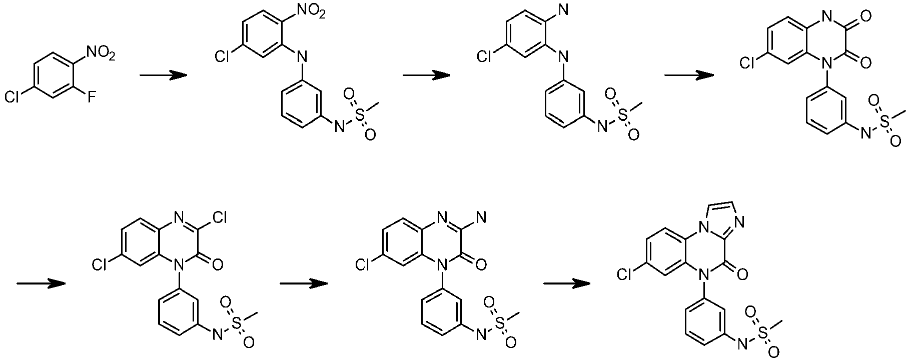

### Example 44-1: N-(3-((5-chloro-2-nitrophenyl)amino)phenyl)methanesulfonamide

4-chloro-2-fluoro-1-nitrobenzene (3 g, 17.09 mmol, 1 eq.) and N-(3-aminophenyl)methanesulfonamide (3.50 g, 18.80 mmol, 3.54 mL, 1.1 eq.) were dissolved in dimethylsulfonamide (35 mL), and N,N-diisopropylethylamine (4.42 g, 34.18 mmol, 5.95 mL, 2 eq.) was added. The reaction mixture was stirred at 110 °C for 15 hours. Water (150 mL) was added to the reaction mixture, and extraction was performed with ethyl acetate (100 mL X 3). The organic layer was washed with a saturated aqueous sodium chloride solution (50 mL), dried over anhydrous sodium sulfate, filtered, and concentrated under reduced pressure. The concentrate was purified by column chromatography (stationary phase: silica, mobile phase: petroleum ether/ethyl acetate=1/0 to 10/1) to obtain the title compound (2 g, 5.85 mmol, 34.2% yield) in the form of a red solid.

¹H NMR (400 MHz, CDCl₃, ppm): δ 9.51 (br s, 1H), 8.18 (d, J = 9.2 Hz, 1H), 7.43 (t, J = 8.0 Hz, 1H), 7.22 (d, J = 2.0Hz, 1H), 7.19 (s, 1H), 7.14 - 7.05 (m, 2H), 6.79 (dd, J = 2.0, 9.2 Hz, 1H), 6.57 (s, 1H), 3.10 (s, 3H).

### Example 44-2: N-(3-((2-amino-5-chlorophenyl)amino)phenyl)methanesulfonamide

The title compound was obtained in the same manner as in Example 33-2 using the compound obtained in Example 44-1 as a starting material.

¹H NMR (400 MHz, DMSO-*d₆*, ppm): δ 9.57 (s, 1H), 7.35 (s, 1H), 7.09 (t, J = 8.0 Hz, 1H), 6.97 (d, J = 2.4 Hz, 1H), 6.85 - 6.80 (m, 1H), 6.74 - 6.69 (m, 2H), 6.58 (br d, J = 7.6 Hz, 1H), 6.50 (br d, J = 8.0 Hz, 1H), 5.15 - 4.75 (m, 2H), 2.96 (s, 3H).

### Example 44-3: N-(3-(7-chloro-2,3-dioxo-3,4-dihydroquinoxalin-1(2H)-yl)phenyl)methanesulfonamide

The title compound was obtained in the same manner as in Example 33-3 using the compound obtained in Example 44-2 as a starting material.

¹H NMR (400 MHz, DMSO-*d*₆, ppm): δ 12.18 (br s, 1H), 10.13 (br s, 1H), 7.60 (br t, J = 7.6 Hz, 1H), 7.37 (br d, J = 7.8 Hz, 1H), 7.21 (br s, 3H), 7.12 (br d, J = 7.6 Hz, 1H), 6.28 (s, 1H), 3.08 (s, 3H).

### Example 44-4: N-(3-(3,7-dichloro-2-oxoquinoxalin-1(2H)-yl)phenyl)methanesulfonamide

The title compound was obtained in the same manner as in Example 33-4 using the compound obtained in Example 44-3 as a starting material.

### Example 44-5: N-(3-(3-amino-7-chloro-2-oxoquinoxalin-1(2H)-yl)phenyl)methanesulfonamide

The title compound was obtained in the same manner as in Example 32-5 using the compound obtained in Example 44-4 as a starting material.

### Example 44-6: N-(3-(7-chloro-4-oxoimidazo[1,2-a]quinoxalin-5(4H)-yl)phenyl)methanesulfonamide

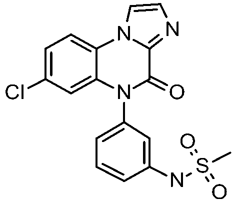

The title compound was obtained in the same manner as in Example 33-6 using the compound obtained in Example 44-5 as a starting material.

¹H NMR (400 MHz, DMSO-*d*₆, ppm): δ 10.18 - 10.08 (m, 1H), 8.64 (s, 1H), 8.28 (d, J = 8.8 Hz, 1H), 7.67 (s, 1H), 7.62 (t, J = 8.0 Hz, 1H), 7.47 - 7.38 (m, 2H), 7.26 (s, 1H), 7.19 (br d, J = 7.6 Hz, 1H), 6.52 (d, J = 2.0 Hz, 1H), 3.10 (s, 3H).

### Example 45: 7-chloro-2-methyl-5-(2-methylpyridin-3-yl)imidazo[1,2-a]quinoxalin-4(5H)-one

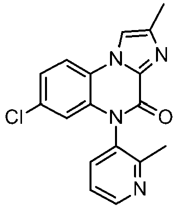

The compound obtained in Example 33-5 (210 mg, 732.43 µmol, 1 eq.) and 1-bromo-2,2-dimethoxypropane (670.33 mg, 3.66 mmol, 492.89 µL, 5 eq.) were dissolved in isopropyl alcohol (5 mL), and pyridinium p-toluenesulfonate (18.41 mg, 73.24 µmol, 0.1 eq.) was added thereto. The reaction mixture was stirred at 95 °C for 16 hours. The reaction mixture was concentrated under reduced pressure to remove isopropyl alcohol, and then water (10 mL) was added, followed by extraction with dichloromethane (10 mL X 3). The organic layer was dried over anhydrous sodium sulfate, filtered, and concentrated, and then the resulting concentrate was purified by prep-HPLC (column: Waters Xbridge C18 150*50mm* 10µm; mobile phase: [water(10mM ammonium carbonate)-acetonitrile]; B%: 22%-52%, 10 min) to obtain the title compound (164.77 mg, 492.13 µmol, 67.2% yield, 97% purity) as a white solid.

¹H NMR (400 MHz, DMSO-*d*₆, ppm): δ 8.69 (m, 1H), 8.42 (s, 1H), 8.22 (d, J = 8.8 Hz, 1H), 7.92 - 7.78 (m, 1H), 7.57 (s, 1H), 7.49 - 7.45 (m, 1H), 6.42 (d, J = 2.0 Hz, 1H), 2.37 (s, 3H), 2.23 (s, 3H).

### Example 46: 7-(difluoromethyl)-5-(o-tolyl)imidazo[1,2-a]pyrido[2,3-e]pyrazin-4(5H)-one

Reaction scheme for preparing the compound of Example 46:

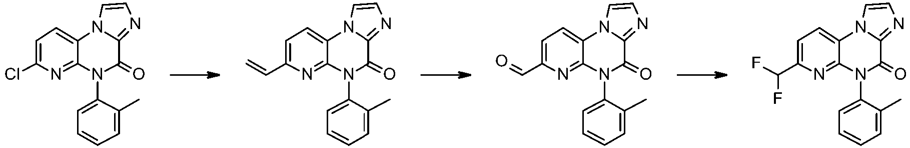

### Example 46-1: 5-(o-tolyl)-7-vinylimidazol[1,2-a]pyrido[2,3-e]pyrazin-4(5H)-one

The compound of Example 35 (300 mg, 965.45 µmol, 1 eq.), vinyltrifluoroborate calcium salt (258.64 mg, 1.93 mmol, 2 eq.), Pd(dppf)Cl₂ (70.64 mg, 96.54 µmol, 0.1 eq.), and Cs₂CO₃ (786.40 mg, 2.41 mmol, 2.5 eq.) were dissolved in dioxane (6 mL) and water (1.5 mL), and stirred for 12 hours at 100°C under nitrogen. After checking the reaction, it was quenched with water (20 mL) and extracted with ethyl acetate. The extracted organic layer was washed with saturated sodium chloride solution, dried over anhydrous sodium sulfate, filtered, and concentrated. The title compound (260 mg, 842.79 µmol, 87.30% yield, 98% purity) was obtained in the form of a yellow solid by purification with column chromatography (petroleum ether/ethyl acetate=100/0 to 0/100).

¹H NMR (400 MHz, CDCl₃, ppm): δ 9.51 (br s, 1H), 8.18 (d, J = 9.2 Hz, 1H), 7.43 (t, J = 8.0 Hz, 1H), 7.22 (d, J = 2.0Hz, 1H), 7.19 (s, 1H), 7.14 - 7.05 (m, 2H), 6.79 (dd, J = 2.0, 9.2 Hz, 1H), 6.57 (s, 1H), 3.10 (s, 3H).

### Example 46-2: 4-oxo-5-(o-tolyl)-4,5-dihydroimidazo[1,2-a]pyrido[2,3-e]pyrazin-7-carbaraldehyde

The compound of Example 46-1 (170 mg, 562.30 µmol, 1 eq.) was dissolved in dioxane (2 mL) and water (2 mL), and sodium periodate (601.36 mg, 2.81 mmol, 155.79 µL, 5 eq.) and potassium osmium(VI) oxide dihydrate (41.44 mg, 112.46 µmol, 0.2 eq.) were added dropwise and stirred at room temperature for 2 hours. After checking the reaction, it was quenched with 10 mL of water and the organic layer was extracted with ethyl acetate. The extracted organic layer was washed with saturated sodium chloride solution, dried over anhydrous sodium sulfate, filtered, and concentrated. The title compound (185 mg, crude mixture), which is a yellow solid, was obtained without further purification.

¹H NMR (400 MHz, DMSO-d₆, ppm): δ 9.62 - 9.51 (m, 1H), 8.91 - 8.82 (m, 1H), 8.80 - 8.72 (m, 1H), 8.02 - 7.93 (m, 1H), 7.78 - 7.73 (m, 1H), 7.47 - 7.30 (m, 4H), 2.06 - 2.01 (m, 3H).

### Example 46-3: 7-(difluoromethyl)-5-(o-tolyl)imidazo[1,2-a]pyrido[2,3-e]pyrazin-4(5H)-one

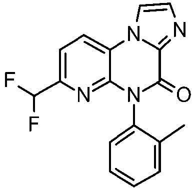

The compound of Example 46-2 (155 mg, 509.36 µmol, 1 eq.) was dissolved in dichloromethane (2.6 mL), and diethylaminosulfur trifluoride (246.31 mg, 1.53 mmol, 201.89 µL, 3 eq.) was added dropwise at 0°C. The mixture was stirred at room temperature for 12 hours, and after checking the reaction, it was quenched with 10 mL of aqueous sodium bicarbonate solution and the organic layer was extracted with dichloromethane. The extracted organic layer was washed with saturated sodium chloride solution, dried over anhydrous sodium sulfate, filtered, and concentrated. The title compound (27 mg, 81.09 µmol, 15.9% yield, 98% purity), which is a yellow solid, was obtained through purification via prep-HPLC (column: Waters Xbridge 150*25mm* 5um; mobile phase: [water(10mM ammonium carbonate)-acetonitrile]; B%: 30%-60%, 8 min).

¹H NMR (400 MHz, DMSO-*d*₆, ppm): 8.82 (d, J=8.4 Hz, 1 H), 8.67 - 8.78 (m, 1 H), 7.69 - 7.77 (m, 2 H), 7.24 - 7.46 (m, 4 H), 6.53 - 6.87 (m, 1 H), 1.99 (s, 3 H).

### Example 47: 7-isopropyl-5-(o-tolyl)imidazo[1,2-a]pyrido[2,3-e]pyrazin-4(5H)-one

Reaction scheme for preparing the compound of Example 47:

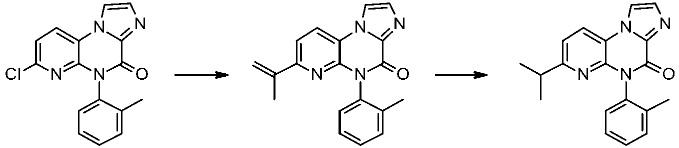

### Example 47-1: 7-(propenyl)-5-(o-tolyl)imidazo[1,2-a]pyrido[2,3-a]pyrazin-4(5H)-one

The compound of Example 35 (200 mg, 643.63 µmol, 1 eq.), isophenyltrifluoroborane calcium salt (190.48 mg, 1.29 mmol, 2 eq.), Pd(dppf)Cl₂ (47.10 mg, 64.36 µmol, 0.1 eq.), and cesium carbonate (524.27 mg, 1.61 mmol, 2.5 eq.) were dissolved in dioxane (4 mL)/ water (1 mL) and stirred at 100 °C for 12 hours. After confirming the reaction, the reaction was quenched with water and extracted with ethyl acetate. The extracted organic layer was washed with a saturated sodium chloride solution, dried over anhydrous sodium sulfate, filtered, and concentrated. Purification was performed via column chromatography (petroleum ether/ethyl acetate = 100/0 to 0/100) (ethyl acetate: methanol = 10:1) to obtain the title compound (190 mg, 510.50 µmol, 79.32% yield, 85% purity) as a yellow solid.

¹H NMR (400 MHz, DMSO-*d*₆, ppm): δ 8.69 (d, J = 1.2 Hz, 1H), 8.65 - 8.58 (m, 1H), 7.70 (d, J = 1.2 Hz, 1H), 7.68 - 7.62 (m, 1H), 7.43 - 7.39 (m, 2H), 7.39 - 7.36 (m, 1H), 7.35 - 7.32 (m, 1H), 7.30 - 7.27 (m, 1H), 5.66 (s, 1H), 5.22 - 5.14 (m, 1H), 1.98 (s, 3H), 1.86 - 1.82 (m, 3H).

### Example 47-2: 7-isopropyl-5-(o-tolyl)imidazo[1,2-a]pyrido[2,3-e]pyrazin-4(5H)-one

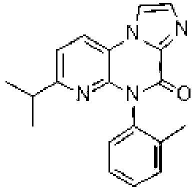

The compound of Example 47-1 (165 mg, 521.56 µmol, 1 eq.) was dissolved in methanol (5 mL), palladium/carbon (20 mg, 10%wt) was added, and the mixture was stirred at room temperature for 2 hours while injecting hydrogen. Purification was performed via prep-HPLC (column: Waters Xbridge 150*25mm* 5µm; mobile phase: [water (10 mM ammonium carbonate)-acetonitrile]; B%: 41%-71%, 8 min) to obtain the title compound (93.96 mg, 292.18 µmol, 56.02% yield, 99% purity) as a white solid.

¹H NMR (400 MHz, DMSO-*d*₆, ppm) δ 8.67 - 8.61 (m, 1H), 8.59 - 8.52 (m, 1H), 7.70 - 7.65 (m, 1H), 7.45 - 7.22 (m, 5H), 1.96 (s, 3H), 1.05 - 0.93 (m, 6H).

### Example 48: 7-chloro-5-(3-methylpyrazin-2-yl)imidazo[1,2-a]quinoxalin-4(5H)-one

Reaction scheme for preparing the compound of Example 48:

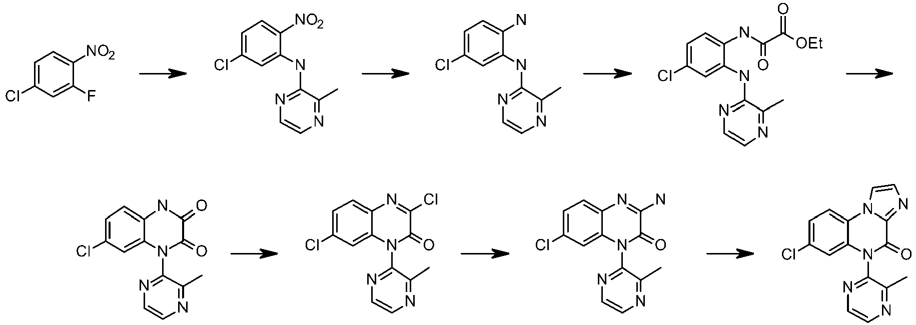

### Example 48-1: N-(5-chloro-2-nitrophenyl)-3-methylpyrazin-2-amine

4-chloro-2-fluoro-1-nitrobenzene (4 g, 22.79 mmol, 1 eq.) and 3-methylpyrazin-2-amine (2.98 g, 27.34 mmol, 1.2 eq.) were dissolved in dimethylformamide (40 mL), and potassium tert-butoxide (3.07 g, 27.34 mmol, 1.2 eq.) was added dropwise thereto. The reaction mixture was stirred at 55 °C for 15 hours. Upon completion of the reaction as confirmed by TLC, the mixture was diluted with water (200 mL) and then extracted with ethyl acetate (100 mL X 3). The organic layer was dried over anhydrous sodium sulfate, filtered, and concentrated under reduced pressure. The resulting concentrate was purified by column chromatography (stationary phase: silica, petroleum ether/ethyl acetate = 1/0 to 10/1) to obtain the title compound (1.6 g, 6.05 mmol, 26.53% yield) as a yellow solid.

### Example 48-2: 5-chloro-N¹-(3-methylpyrazin-2-yl)benzen-1,2-diamine

The title compound was obtained in the same manner as in Example 33-2 using the compound obtained in Example 48-1 as a starting material.

### Example 48-3: ethyl 2-((4-chloro-2-((3-methylpyrazin-2-yl)amino)phenyl)amino)-2-oxoacetate

The compound of Example 48-2 (2 g, 8.52 mmol, 1 eq.) and ethyl 2-chloro-2-oxo-acetate (1.40 g, 10.23 mmol, 1.14 mL, 1.2 eq.) were added to water (8 mL) and dichloromethane (32 mL), and sodium carbonate (993.58 mg, 9.37 mmol, 1.1 eq.) was added dropwise. The reaction mixture was stirred at room temperature for 12 hours. When the reaction was completed through TLC, it was diluted with water (50 mL) and then extracted with dichloromethane (50 mL X 3). The organic layer was washed with a saturated aqueous sodium chloride solution, dried over anhydrous sodium sulfate, filtered, and concentrated under reduced pressure. The resulting title compound in the form of a yellow oil (2 g, crude mixture) was used in the next reaction without further purification.

### Example 48-4: 7-chloro-1-(3-methylpyrazin-2-yl)-1,4-dihydroquinoxalin-2,3-dione

The compound of Example 48-3 (1.5 g, 4.48 mmol, 1 eq.) was dissolved in tetrahydrofuran (20 mL), and sodium hydride (896.09 mg, 22.40 mmol, 60% purity, 5 eq.) was added dropwise. The reaction mixture was stirred at room temperature for 12 hours. After quenching the reaction by adding water (50 mL) to the reaction mixture, it was extracted with ethyl acetate (50 mL X 3). The organic layer was washed with a saturated aqueous sodium chloride solution (50 mL), dried over anhydrous sodium sulfate, filtered, and concentrated under reduced pressure. The resulting concentrate was purified by column chromatography (stationary phase: silica, petroleum ether/ethyl acetate = 3/1 to 1/1) to obtain the title compound in the form of a yellow solid (0.4 g, 1.39 mmol, 30.92% yield).

¹H NMR (400 MHz, DMSO-*d*₆, ppm): δ 12.38 (br s, 1H), 8.82 (d, J = 2.4 Hz, 1H), 8.67 (d, J = 2.0 Hz, 1H), 7.28 (s, 2H), 6.45 (s, 1H), 2.39 (s, 3H).

### Example 48-5: 3,7-dichloro-1-(3-methylpyrazin-2-yl)quinoxalin-2(1H)-one

The title compound was obtained in the same manner as in Example 33-4 using the compound obtained in Example 48-4 as a starting material.

### Example 48-6: 3-amino-7-chloro-1-(3-methylpyrazin-2-yl)quinoxalin-2(1H)-one

The compound of Example 48-5 (400 mg, 1.30 mmol, 1 eq.) was dissolved in isopropyl alcohol (30 mL), and ammonia (2 M, 6.51 mL, 10 eq.) was added dropwise. The reaction mixture was stirred at room temperature for 12 hours. The desired m/z ([M + H]+ = 288.4) was detected by LCMS. The reaction mixture was concentrated. The title compound in the form of a black oil (350 mg, crude mixture) was used in the next reaction without further purification.

### Example 48-7: 7-chloro-5-(3-methylpyrazin-2-yl)imidazo[1,2-a]quinoxalin-4(5H)-one

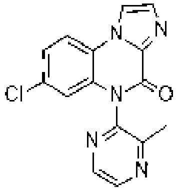

The title compound was obtained in the same manner as in Example 33-6 using the compound obtained in Example 48-6 as a starting material.

¹H NMR (400 MHz, DMSO-*d*₆, ppm) δ 8.83 (d, J = 2.4 Hz, 1H), 8.73 - 8.68 (m, 2H), 8.34 (d, J = 8.8 Hz, 1H), 7.73 (d, J = 1.2 Hz, 1H), 7.54 - 7.50 (m, 1H), 6.68 (d, J = 2.0 Hz, 1H), 2.38 (s, 3H).

### Example 49: 5-(o-tolyl)-7-(trifluoromethyl)imidazo[1,2-a]pyrido[2,3-e]pyrazin-4(5H)-one

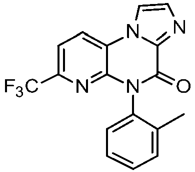

The title compound was obtained in the same manner as in Example 48 using 2-chloro-3-nitro-6-(trifluoromethyl)pyridine as a starting material.

¹H NMR (400 MHz, DMSO-*d*₆, ppm) δ 8.84 - 8.92 (m, 1 H), 8.77 (d, J = 1.2 Hz, 1 H), 7.93 (d, J = 8.0 Hz, 1 H), 7.75 (d, J =1.2 Hz, 1 H), 7.27 - 7.45 (m, 4 H), 1.96 - 2.02 (m, 3 H).

### Example 50: 7-chloro-5-(2-(trifluoromethoxy)phenyl)imidazo[1,2-a]quinoxalin-4(5H)-one

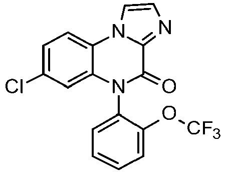

The title compound was obtained in the same manner as in Example 33-1 by changing 2-methylpyridin-3-amine to 2-(trifluoromethoxy)aniline.

¹H NMR (400 MHz, DMSO-*d*₆, ppm) δ 8.71 (d, J = 0.8 Hz, 1H), 8.34 (d, J = 8.8 Hz, 1H), 7.80 - 7.69 (m, 5H), 7.53 - 7.47 (m, 1H), 6.50 (d, J = 2.0 Hz, 1H).

### Example 51: 7-chloro-5-(2,6-dimethylphenyl)imidazo[1,2-a]quinoxalin-4(5H)-one

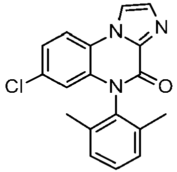

The title compound was obtained in the same manner as in Example 33-1 by changing 2-methylpyridin-3-amine to 2,6-dimethylaniline.

¹H NMR (400 MHz, DMSO-*d*₆, ppm) δ 8.71 (d, J = 1.0 Hz, 1H), 8.36 (d, J = 8.8 Hz, 1H), 7.72 (d, J = 1.2 Hz, 1H), 7.53 - 7.48 (m, 1H), 7.46 - 7.40 (m, 1H), 7.39 - 7.34 (m, 2H), 6.34 (d, J = 2.0 Hz, 1H), 1.95 (s, 6H).

### Example 52: 7-chloro-5-(2-fluoro-6-methylphenyl)imidazo[1,2-a]quinoxalin-4(5H)-one

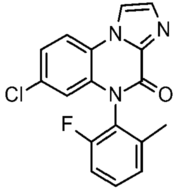

The title compound was obtained in the same manner as in Example 33-1 by changing 2-methylpyridin-3-amine to 2-fluoro-6-methylaniline.

¹H NMR (400 MHz, DMSO-*d*₆, ppm) δ 8.75 - 8.69 (m, 1H), 8.35 (d, J = 8.8 Hz, 1H), 7.72 (d, J = 1.2 Hz, 1H), 7.63 - 7.56 (m, 1H), 7.55 - 7.50 (m, 1H), 7.43 - 7.35 (m, 2H), 6.49 (d, J = 2.0 Hz, 1H), 2.08 - 2.05 (m, 3H).

### Example 53: 7-chloro-5-(3-methylpyridin-2-yl)imidazo[1,2-a]quinoxalin-4(5H)-one

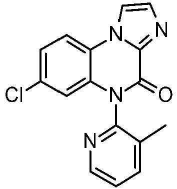

The title compound was obtained in the same manner as in Example 33-1 by changing 2-methylpyridin-3-amine to 3-methylpyridin-2-amine.

¹H NMR (400 MHz, DMSO-*d*₆, ppm): δ 8.75 - 8.67 (m, 1H), 8.59 (br d, J = 3.5 Hz, 1H), 8.40 - 8.29 (m, 1H), 8.05 (br d, J = 7.3 Hz, 1H), 7.76 - 7.71 (m, 1H), 7.66 - 7.58 (m, 1H), 7.54 - 7.46 (m, 1H), 6.38 - 6.29 (m, 1H), 2.17 - 2.06 (m, 3H).

### Example 54: 7-chloro-5-(2-chloropyridin-3-yl)imidazo[1,2-a]quinoxalin-4(5H)-one

Reaction scheme for preparing the compound of Example 54:

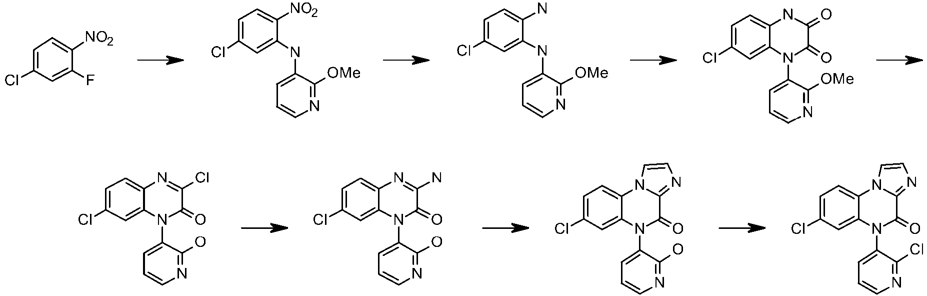

### Example 54-1: N-(5-chloro-2-nitrophenyl)-2-methoxypyridin-3-amine

2-methoxypyridin-3-amine (1.86 g, 14.95 mmol, 1.05 eq.) was dissolved in tetrahydrofuran (25 mL), and lithium bis(trimethylsilyl)amide (1 M, 17.09 mL, 1.2 eq.) was added dropwise, followed by stirring under nitrogen at 0 °C for 1 hour. To the resulting reaction mixture, a solution of 4-chloro-2-fluoro-1-nitrobenzene (2.5 g, 14.24 mmol, 1 eq.) dissolved in tetrahydrofuran (5 mL) was added dropwise at 0 °C. The reaction mixture was stirred at room temperature for 1 hour. Desired m/z ([M + H]+ = 280.2) was observed by LC-MS. Saturated aqueous ammonium chloride solution (60 mL) was added, and extraction was performed with ethyl acetate (3 X 40 mL). The organic layer was washed with saturated aqueous sodium chloride solution (3 X 40 mL), dried over anhydrous sodium sulfate, filtered, and concentrated under reduced pressure. The resulting concentrate was purified by column chromatography (stationary phase: silica, mobile phase: 0-12% ethyl acetate/petroleum ether) to obtain the title compound (2.81 g, 8.29 mmol, 58.2 % yield, 82.5 % purity) as a red solid.

¹H NMR (400 MHz, DMSO-*d*₆, ppm): δ 9.41 (s, 1H), 8.18 (d, J = 9.2 Hz, 1H), 8.12 - 8.06 (m, 1H), 7.86 - 7.78 (m, 1H), 7.15 - 7.08 (m, 1H), 6.98 - 6.90 (m, 2H), 3.96 - 3.90 (m, 3H).

### Example 54-2: 5-chloro-N¹-(2-methoxypyridin-3-yl)benzen-1,2-diamine

The title compound was synthesized in the same manner as in Example 33-2 by using the compound of Example 54-1 as a starting material.

¹H NMR (400 MHz, DMSO-*d*₆, ppm): δ 7.55 (dd, J = 1.6, 4.8 Hz, 1H), 6.94 - 6.92 (m, 1H), 6.91 - 6.86 (m, 2H), 6.85 - 6.81 (m, 1H), 6.79 - 6.77 (m, 1H), 6.73 - 6.71 (m, 1H), 5.41 - 4.99 (m, 2H), 3.96 - 3.92 (m, 3H).

### Example 54-3: 7-chloro-1-(2-methoxypyridin-3-yl)-1,4-dihydroquinoxalin-2,3-dione

The compound of Example 54-2 (1.9 g, 7.61 mmol, 1 eq.), diethyl oxalate (20.33 g, 139.11 mmol, 19.00 mL, 18.28 eq.), and N,N-diisopropylethylamine (1.97 g, 15.22 mmol, 2.65 mL, 2 eq.) were stirred under nitrogen at 160 °C for 4 hours. After the reaction mixture was cooled to room temperature, it was concentrated under reduced pressure. Petroleum ether (30 mL) was added to the crude compound and left at room temperature for 30 minutes to proceed with solidification, followed by filtration and concentration of the filter cake under reduced pressure to obtain the title compound (860 mg, 2.83 mmol, 37.1% yield, 99.8 % purity) as a green solid.

¹H NMR (400 MHz, DMSO-*d*₆, ppm): δ 12.35 (br s, 1H), 8.41 (br d, J = 3.6 Hz, 1H), 7.87 (s, 1H), 7.39 - 7.09 (m, 3H), 6.33 (s, 1H), 3.84 (s, 3H).

### Example 54-4: 3,7-dichloro-1-(2-hydroxypyridin-3-yl)quinoxalin-2(1H)-one

The title compound was synthesized in the same manner as in Example 33-4 by using the compound of Example 54-3 as a starting material.

### Example 54-5: 3-amino-7-chloro-1-(2-hydroxypyridin-3-yl)quinoxalin-2(1H)-one

The title compound was synthesized in the same manner as in Example 33-5 by using the compound of Example 54-4 as a starting material.

¹H NMR (400 MHz, DMSO-*d*₆, ppm): δ 10.03 - 8.73 (m, 1H), 7.79 (dd, J = 1.9, 7.2 Hz, 1H), 7.66 (dd, J = 2.0, 6.3 Hz, 1H), 7.53 - 7.38 (m, 1H), 7.36 (d, J = 8.8 Hz, 1H), 7.31 - 7.23 (m, 1H), 7.20 (dd, J = 2.4, 8.5 Hz, 1H), 6.63 - 6.57 (m, 1H), 6.45 - 6.39 (m, 1H)

### Example 54-6: 7-chloro-5-(2-hydroxypyridin-3-yl)imidazo[1,2-a]quinoxalin-4(5H)-one

The title compound was synthesized in the same manner as in Example 33-6 by using the compound of Example 54-5 as a starting material.

¹H NMR (400 MHz, DMSO-*d*₆, ppm): δ 12.34 (br s, 1H), 8.66 (d, J = 1.2 Hz, 1H), 8.29 (d, J = 8.8 Hz, 1H), 7.81 (dd, J = 2.0, 7.1 Hz, 1H), 7.73 - 7.66 (m, 2H), 7.47 (dd, J = 2.2, 8.8 Hz, 1H), 6.85 (d, J = 2.0 Hz, 1H), 6.46 (t, J = 6.8 Hz, 1H)

### Example 54-7: 7-chloro-5-(2-chloropyridin-3-yl)imidazo[1,2-a]quinoxalin-4(5H)-one

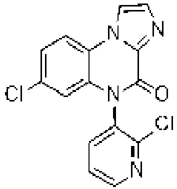

The compound of Example 54-6 (255 mg, 815.45 µmol, 1 eq.) and phosphoryl chloride (4.13 g, 26.90 mmol, 2.5 mL, 32.99 eq.), and N,N-diisopropylethylamine (158.09 mg, 1.22 mmol, 213.06 µL, 1.5 eq.) were stirred at 110 °C for 16 hours. A saturated aqueous sodium bicarbonate solution (150 mL) was slowly added to the reaction mixture, and then the mixture was extracted with ethyl acetate (40 mL X 3). The organic layer was washed with a saturated aqueous sodium chloride solution (50 mL X 2), dried over anhydrous sodium sulfate, filtered, and concentrated under reduced pressure. The resulting concentrate was purified by prep-HPLC (column: Waters Xbridge C18 150*50mm* 10µm; mobile phase: [water (10 Mm ammonium bicarbonate)-acetonitrile]; B%: 25%-55%, 10 minutes) to obtain the title compound (55 mg, 164.45 µmol, 20.2 % yield, 99.0% purity) as a white solid.

¹H NMR (400 MHz, DMSO-*d*₆, ppm) δ 8.73 - 8.71 (m, 1H), 8.70 - 8.67 (m, 1H), 8.40 - 8.30 (m, 1H), 8.24 - 8.15 (m, 1H), 7.81 - 7.75 (m, 1H), 7.75 - 7.72 (m, 1H), 7.58 - 7.47 (m, 1H), 6.65 (d, J = 2.0 Hz, 1H).

### Example 55: 2-methyl-5-(2-methylpyridin-3-yl)-7-(trifluoromethyl)imidazo[1,2-a]quinoxalin-4(5H)-one

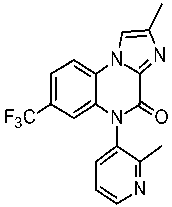

The title compound was synthesized in the same manner as in Example 45 by changing the starting material to 2-fluoro-1-nitro-4-(trifluoromethyl)benzene.

¹H NMR (400 MHz, DMSO-*d*₆, ppm): δ 8.73 - 8.69 (m, 1H), 8.53 - 8.47 (m, 1H), 8.42 - 8.37 (m, 1H), 7.92 - 7.87 (m, 1H), 7.80 - 7.74 (m, 1H), 7.58 - 7.52 (m, 1H), 6.61 (s, 1H), 2.40 (s, 3H), 2.23 (s, 3H).

### Example 56: 5-(2-methylpyridin-3-yl)-7-(trifluoromethyl)imidazo[1,2-a]quinoxalin-4(5H)-one

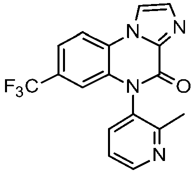

The title compound was synthesized in the same manner as in Example 33 by changing the starting material to 2-fluoro-1-nitro-4-(trifluoromethyl)benzene.

¹H NMR (400 MHz, DMSO-*d*₆, ppm): δ 8.79 (s, 1H), 8.72 - 8.71 (m, 1H), 8.53 - 8.51 (m, 1H), 7.91 - 7.89 (m, 1H), 7.81 - 7.79 (m, 1H), 7.77 - 7.73 (m, 1H), 7.57 - 7.56 (m, 1H), 6.62 (s, 1H), 2.26 (s, 3H).

### Example 57: 1-bromo-5-(2-methylpyridin-3-yl)-7-(trifluoromethyl)imidazo[1,2-a]quinoxalin-4(5H)-one

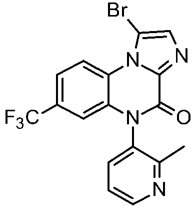

The compound of Example 56 (1 g, 2.90 mmol, 1 eq.) was dissolved in acetonitrile (15 mL), and N-bromosuccinimide (516.95 mg, 2.90 mmol, 1 eq.) was added. The reaction mixture was stirred at 25 °C for 12 hours. MS [M+H]+=424.7 of the title compound was detected through LCMS, the reaction mixture was concentrated under reduced pressure to remove the solvent, and the resulting concentrate was purified by prep-HPLC (column: Waters Xbridge 150*25mm*5µm; mobile phase: [water(10mM ammonium bicarbonate)-acetonitrile]; B%: 32%-62%, 9 min) to obtain the title compound (270 mg, 625.26 µmol, 21.5% yield, 98% purity) in the form of a yellow solid.

¹H NMR (400 MHz, DMSO-*d*₆, ppm): δ 9.25 (d, *J* = 8.8 Hz, 1H), 8.73 - 8.71 (m, 1H), 7.88 - 7.84 (m, 3H), 7.58 - 7.54 (m, 1H), 6.66 - 6.65 (m, 1H), 2.27 (s, 3H).

### Example 58: 1-methyl-5-(2-methylpyridin-3-yl)-7-(trifluoromethyl)imidazo[1,2-a]quinoxalin-4(5H)-one

Reaction scheme for preparing the compound of Example 58:

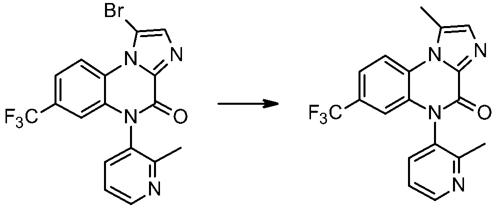

The compound of Example 57 (150 mg, 354.45 µmol, 1 eq.), 2,4,6-trimethyl-1,3,5,2,4,6-trioxaborinane (3.5 M, 405.09 µL, 4 eq.), potassium carbonate (195.96 mg, 1.42 mmol, 4 eq.), and Pd(PPh₃)₂Cl₂ (24.88 mg, 35.45 µmol, 0.1 eq. were dissolved in dimethylformamide (4 mL) and stirred at 90 °C for 12 hours. After confirming the reaction, it was quenched with saturated aqueous ammonium chloride solution (10 mL) at room temperature, and extracted with ethyl acetate. The extracted organic layer was washed with saturated sodium chloride solution, dried over anhydrous sodium sulfate, filtered, and concentrated. The title compound (75 mg, 205.13 µmol, 57.9% yield, 98% purity) was obtained as a yellow solid by purifying with prep-HPLC (column: Waters Xbridge 150*25mm* 5 µm; mobile phase: [water(10mM ammonium bicarbonate)]).

¹H NMR (400 MHz, DMSO-*d*₆, ppm): δ 8.72 - 8.71 (m, 1H), 8.50 - 8.48 (m, 1H), 7.91 - 7.89 (m, 1H), 7.74 - 7.72 (m, 1H), 7.58 - 7.56 (m, 1H), 7.50 (s, 1H), 6.64 - 6.32 (m, 1H), 2.90 (s, 3H), 2.24 (m, 3H).

### Example 59: 1-acetyl-5-(2-methylpyridin-3-yl)-7-(trifluoromethyl)imidazo[1,2-a]quinoxalin-4(5H)-one

Reaction scheme for preparing the compound of Example 59:

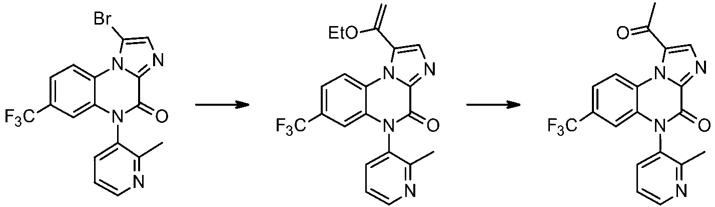

### Example 59-1: 1-(1-ethoxyvinyl)-5-(2-methylpyridin-3-yl)-7-(trifluoromethyl)imidazo[1,2-a]quinoxalin-4(5H)-one

The compound of Example 57 (160.00 mg, 378.08 µmol, 1 eq.), tributyl(1-ethoxyvinyl)stannane (218.47 mg, 604.93 µmol, 204.37 µL, 1.6 eq.), and Pd(PPh₃)₂Cl₂ (26.54 mg, 37.81 µmol, 0.1 eq.) were dissolved in dioxane (5 mL) and stirred at 90 °C for 12 hours. The next reaction was proceeded without further purification.

¹H NMR (400 MHz, DMSO-*d*₆, ppm): δ 8.72 - 8.71 (m, 1H), 8.50 - 8.48 (m, 1H), 7.91 - 7.89 (m, 1H), 7.74 - 7.72 (m, 1H), 7.58 - 7.56 (m, 1H), 7.50 (s, 1H), 6.64 - 6.32 (m, 1H), 2.90 (s, 3H), 2.24 (m, 3H).

### Example 59-2: 1-acetyl-5-(2-methylpyridin-3-yl)-7-(trifluoromethyl)imidazo[1,2-a]quinoxalin-4(5H)-one

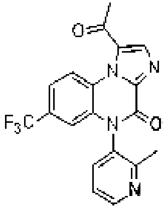

4 mL of hydrochloric acid/dioxane (1 mol/L) was added to the compound of Example 59-1, followed by stirring at 25°C for 2 hours. After terminating the reaction by adding 20 mL of an aqueous potassium fluoride solution to the reaction mixture, extraction was performed with ethyl acetate (10 mL X 3). The organic layer was washed with a saturated sodium chloride solution, dried over anhydrous sodium sulfate, filtered, and concentrated. The concentrate was purified by prep-HPLC (column: Waters Xbridge 150*25mm* 5 µm; mobile phase: [water (10 mM ammonium carbonate)-acetonitrile]; B%: 26%-56%, 9 minutes) to obtain the title compound in the form of a yellow solid (52 mg, 129.62 µmol, 34.28% yield, 96.3% purity).

¹H NMR (400 MHz, DMSO-*d*₆, ppm): δ 8.82 - 8.73 (m, 1H), 8.71 - 8.67 (m, 1H), 8.67 - 8.64 (m, 1H), 8.04 - 7.93 (m, 1H), 7.79 - 7.70 (m, 1H), 7.68 - 7.59 (m, 1H), 6.73 - 6.64 (m, 1H), 2.80 (s, 3H), 2.30 (m, 3H).

### Example 60: 2-acetyl-1-hydroxy-5-(2-methylpyridin-3-yl)-7-(trifluoromethyl)imidazo[1,2-a]quinoxalin-4(5H)-one

Reaction scheme for preparing the compound of Example 60:

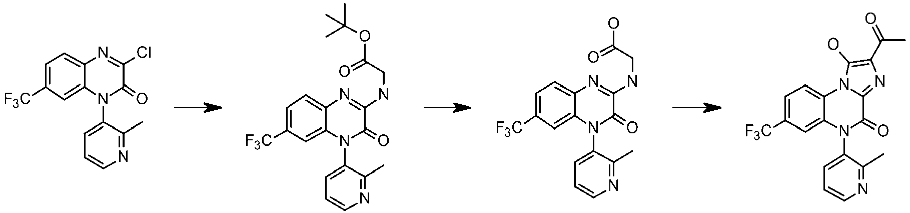

### Example 60-1: tert-butyl (4-(2-methylpyridin-3-yl)-3-oxo-6-(trifluoromethyl)-3,4-dihydroquinoxalin-2-yl)glycinate

After dissolving 3-chloro-1-(2-methylpyridin-3-yl)-7-(trifluoromethyl)quinoxalin-2(1H)-one (1 g, 2.94 mmol, 1 eq.) in 10 mL of tetrahydrofuran, triethylamine (893.64 mg, 8.83 mmol, 1.23 mL, 3 eq.) and tert-butyl 2-aminoacetate (772.29 mg, 5.89 mmol, 2 eq.) were added dropwise. The reaction solution was stirred at room temperature for 12 hours, and after terminating the reaction with water, extraction was performed with ethyl acetate. The extracted organic layer was washed with an aqueous sodium chloride solution and then dried over anhydrous sodium sulfate. Purification was performed by column chromatography (ethyl acetate/petroleum ether 0 to 50%) to obtain the title compound as a yellow solid (730 mg, 1.44 mmol, 48.8% yield, 85.4% purity).

¹H NMR (400 MHz, DMSO-*d*₆, ppm) δ 8.77 - 8.69 (m, 1H), 8.46 - 8.36 (m, 1H), 8.03 - 7.92 (m, 1H), 7.66 - 7.46 (m, 3H), 6.56 - 6.45 (m, 1H), 4.09 (dd, J = 2.4, 6.1 Hz, 2H), 2.21 - 2.16 (m, 3H), 1.47 - 1.41 (m, 10H).

### Example 60-2: (4-(2-methylpyridin-3-yl)-3-oxo-6-(trifluoro)-3,4-dihydroquinoxalin-2-yl)glycine

The compound of Example 60-1 (850 mg, 1.96 mmol, 1 eq.) was dissolved in dichloromethane, and 8.5 mL of 4M hydrogen chloride/dioxane was added dropwise, followed by stirring at room temperature for 3 hours. The reaction was terminated with water, and extraction was performed with ethyl acetate. The extracted organic layer was washed with an aqueous sodium chloride solution, dried over anhydrous sodium sulfate, filtered, and concentrated to obtain the title compound as a yellow solid (630 mg, crude mixture).

¹H NMR (400 MHz, DMSO-*d*₆, ppm): δ13.41 - 12.10 (m, 1H), 8.80 - 8.67 (m, 1H), 8.35 - 8.27 (m, 1H), 8.04 - 7.88 (m, 1H), 7.70 - 7.60 (m, 1H), 7.59 - 7.47 (m, 2H), 6.55 - 6.36 (m, 1H), 4.20 (s, 2H), 2.23 - 2.13 (m, 3H).

### Example 60-3: 2-acetyl-1-hydroxy-5-(2-methylpyridin-3-yl)-7-(trifluoromethyl)imidazo[1,2-a]quinoxalin-4(5H)-one

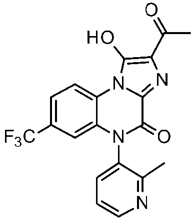

The compound of Example 60-2 (200 mg, 482.20 µmol, 1 eq.) was dissolved in 6 mL of tetrahydrofuran, and acetic anhydride (492.28 mg, 4.82 mmol, 452.88 µL, 10 eq.) and triethylamine (671.17 µL, 4.82 mmol, 10 eq.) were added dropwise at 0 °C. Thereafter, 4-dimethylaminopyridine (5.89 mg, 48.22 µmol, 0.1 eq.) was added dropwise, and the mixture was stirred at 0 °C for 1 hour. After confirming the reaction, it was terminated with water (30 mL), adjusted to pH 6-7 with an aqueous sodium bicarbonate solution, and extracted with ethyl acetate. The extracted organic layer was washed with an aqueous sodium chloride solution, dried over anhydrous sodium sulfate, filtered, and concentrated. Purification was performed by column chromatography (methanol/dichloromethane 0-15%), and the product was solidified with ethyl acetate and filtered. The title compound was obtained as a yellow solid (35 mg, 86.39 µmol, 43.4% yield, 99.3% purity).

¹H NMR (400 MHz, DMSO-*d*₆, ppm) δ 9.45 - 9.32 (m, 1H), 8.72 - 8.60 (m, 1H), 7.92 - 7.77 (m, 1H), 7.58 - 7.46 (m, 2H), 6.42 - 6.32 (m, 1H), 2.38 (s, 3H), 2.21 (s, 3H)

### Example 61: 7-chloro-2-((4-methylpiperazin-1-yl)methyl)-5-(2-methylpyridin-3-yl)imidazo[1,2-a]quinoxalin-4(5H)-one

Reaction scheme for preparing the compound of Example 61:

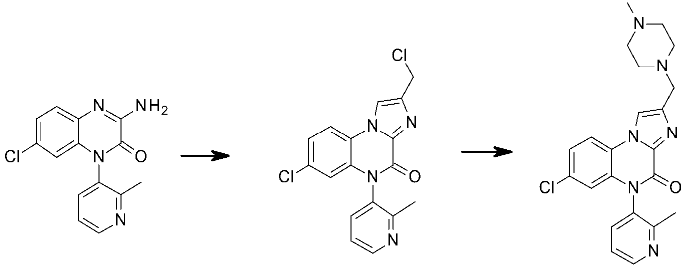

### Example 61-1: 7-chloro-2-(chloromethyl)-5-(2-methylpyridin-3-yl)imidazo[1,2-a]quinoxalin-4(5H)-one

By using the compound of Example 54-4 (200mg, 698 µmol, 1 eq.) as a starting material, 1,3-dichloro-2-propanone (443mg, 3.49 mmol, 5 eq.) was dissolved in ethyl acetate and heated to reflux. Upon completion of the reaction, after cooling to room temperature, the mixture was purified by column chromatography (ethyl acetate/hexane 0-100 %) to obtain the title compound (50mg, 139 µmol, 20% yield) as a white solid.

### Example 61-2: 7-chloro-2-((4-methylpiperazin-1-yl)methyl)-5-(2-methylpyridin-3-yl)imidazo[1,2-a]quinoxalin-4(5H)-one

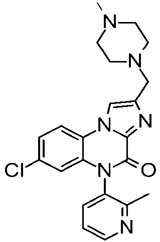

By using the compound of Example 61-1 (50 mg, 139 µmol, 1 eq.) as a starting material, 1-methylpiperazine (20.9 mg, 209 µmol, 1.5 eq.) was dissolved in ethanol and heated under reflux. Upon completion of the reaction, the mixture was cooled to room temperature and purified by column chromatography (methanol/dichloromethane 0-20%). The title compound (23 mg, 54.4 µmol, 39% yield, 99.77% purity) was obtained as a pale yellow solid.

¹H NMR (400 MHz, CDCl₃, ppm): δ 8.75 (d, 1H, J= 3.6 Hz), 7.97 (brs, 1H), 7.71 (d, 1H, J= 8.4Hz), 7.57 (d, 1H, J= 7.6Hz), 7.45 - 7.43 (m, 1H), 7.33 - 7.31 (m, 1H), 6.55(s, 1H), 3.87(s, 2H), 2.82 - 2.68 (m, 8H), 2.42 (s, 3H), 2.36 (s, 3H)

### Example 62: 7-chloro-5-(2-methylpyridin-3-yl)-2-(morpholinomethyl)imidazo[1,2-a]quinoxalin-4(5H)-one

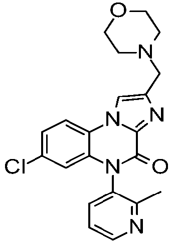

By using the compound of Example 61-1 (40mg, 111 µmol, 1 eq.) as a starting material, the title compound (37mg, 90.3 µmol, 81% yield, 99.43% purity) was obtained in the same manner as Example 61-2.

¹H NMR (400 MHz, CDCl₃, ppm): δ 8.75 (d, 1H, *J*= 3.6 Hz), 7.92 (s, 1H), 7.70 (d, 1H, *J*= 8.8Hz), 7.58 (d, 1H, *J*= 1.6Hz), 7.56 (d, 1H, *J*= 1.2Hz), 7.33 - 7.30 (m, 1H), 6.55(s, 1H), 3.82(s, 2H), 3.77 (m, 4H), 2.66 (s, 4H), 2.36 (s, 3H)

### Example 63: 2-((2-oxa-7-azaspiro[3.5]nonane-7-yl)methyl)-7-chloro-5-(2-methylpyridin-3-yl)imidazo[1,2-a]quinoxalin-4(5H)-one

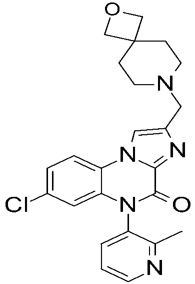

By using the compound of Example 61-1 (30 mg, 83.5 µmol, 1 eq.) as a starting material, the title compound (16 mg, 35.6 µmol, 42% yield, 97.92% purity) was obtained in the same manner as in Example 61-2.

¹H NMR (400 MHz, CDCl₃, ppm): δ 8.75 (d, 1H, J= 3.6 Hz), 7.91 (s, 1H), 7.69 (d, 1H, J= 8.8Hz), 7.57 (m, 1H), 7.43 (m, 1H), 7.33 - 7.30 (m, 1H), 6.55 (s, 1H), 4.43 (s, 4H), 3.76 (s, 2H), 2.52 (s, 4H), 2.36 (s, 3H), 1.94 (s, 4H)

### Example 64: 7-chloro-2-(((2-methoxyethyl)amino)methyl)-5-(2-methylpyridin-3-yl)imidazo[1,2-a]quinoxalin-4(5H)-one

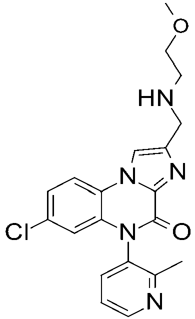

By using the compound of Example 61-1 (40 mg, 111 µmol, 1 eq.) as a starting material, the title compound (6.9 mg, 17.3 µmol, 16% yield, 99.55% purity) was obtained in the same manner as in Example 61-2.

¹H NMR (400 MHz, CDCl₃, ppm): δ 8.76 (s, 1H), 8.49 (s, 1H), 7.81 (d, 1H, *J*= 8.8Hz), 7.61 (d, 1H, *J*= 7.6Hz), 7.45 (m, 1H), 7.33 - 7.31 (m, 1H), 6.55 (s, 1H), 4.38 (s, 2H), 3.77 (m, 2H), 3.37 (s, 3H), 3.26 (m, 2H), 2.34 (s, 3H)

### Example 65: 5-(2,3-dihydrobenzofuran-4-yl)-7-(trifluoromethyl)imidazo[1,2-a]quinoxalin-4(5H)-one

Reaction scheme for preparing the compound of Example 65:

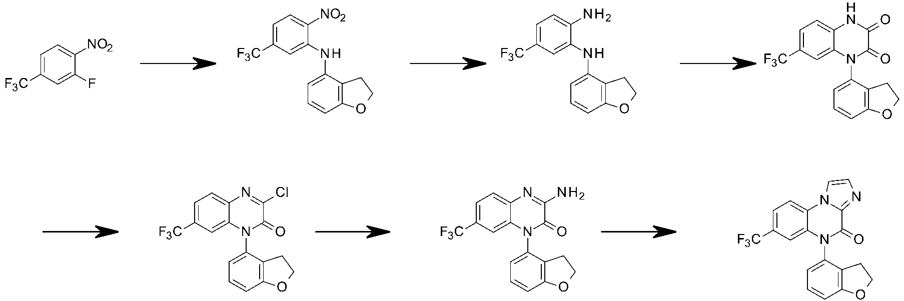

### Example 65-1: N-(2-nitro-5-(trifluoromethyl)phenyl)-2,3-dihydrobenzofuran-4-amine

The title compound (0.6 g, 1.85 mmol, 62.5% yield) as an orange solid was obtained in the same manner as in Example 33-1 using 2,3-dihydrobenzofuran-4-amine (400 mg, 2.96 mmol, 1 eq. and 2-fluoro-1-nitro-4-(trifluoromethyl)benzene (928.20 mg, 4.44 mmol, 1.5 eq. as starting materials.

¹H NMR (400 MHz, CDCl₃, ppm): δ 9.40 (brs, 1H), 8.34 (d, J = 8.8 Hz, 1H), 7.37 - 7.31 (m, 1H), 7.26 - 7.19 (m, 1H), 7.04 - 6.97 (m, 1H), 6.86 - 6.80 (m, 1H), 6.80 - 6.74 (m, 1H), 4.74 - 4.59 (m, 2H), 3.20 - 3.01 (m, 2H).

### Example 65-2: N¹-(2,3-dihydrobenzofuran-4-yl)-5-(trifluoromethyl)benzen-1,2-diamine

The title compound (515 mg, crude mixture) was obtained in the same manner as in Example 33-2 using the compound of Example 65-1 (600 mg, 1.85 mmol, 1 eq.) as a starting material.

¹H NMR (400 MHz, DMSO-*d*₆, ppm): δ 7.17 - 7.11 (m, 1H), 7.11 - 7.06 (m, 1H), 6.93 - 6.85 (m, 2H), 6.84 - 6.79 (m, 1H), 6.23 (d, J = 8.0 Hz, 1H), 6.08 (d, J = 7.6 Hz, 1H), 5.50 (brs, 2H), 4.49 (t, J = 8.8 Hz, 2H), 2.99 (t, J = 8.8 Hz, 2H).

### Example 65-3: 1-(2,3-dihydrobenzofuran-4-yl)-7-(trifluoromethyl)-1,4-dihydroquinoxalin-2,3-dione

The title compound (692 mg, crude mixture) was obtained in the same manner as in Example 33-3 using the compound of Example 65-2 (515 mg, 1.75 mmol, 1 eq.) as a starting material.

¹H NMR (400 MHz, DMSO-*d*₆, ppm): δ 12.56 - 12.36 (m, 1H), 7.62 - 7.48 (m, 1H), 7.46 - 7.27 (m, 2H), 7.04 - 6.92 (m, 1H), 6.91 - 6.79 (m, 1H), 6.63 - 6.51 (m, 1H), 4.66 - 4.47 (m, 2H), 3.11 - 2.76 (m, 2H).

### Example 65-4: 3-chloro-1-(2,3-dihydrobenzofuran-4-yl)-7-(trifluoromethyl)-1,4-dihydro quinoxalin-2(1H)-one

The title compound (500 mg, crude mixture) was obtained in the same manner as in Example 33-4 using the compound of Example 65-3 (492 mg, 1.41 mmol, 1 eq.) as a starting material.

¹H NMR (400 MHz, DMSO-*d*₆, ppm): δ 7.62 - 7.52 (m, 1H), 7.52 - 7.42 (m, 1H), 7.37 - 7.25 (m, 1H), 7.00 - 6.87 (m, 1H), 6.86 - 6.72 (m, 1H), 6.64 - 6.43 (m, 1H), 4.64 - 4.41 (m, 2H), 3.07 - 2.70 (m, 2H).

### Example 65-5: 3-amino-1-(2,3-dihydrobenzofuran-4-yl)-7-(trifluoromethyl)-1,4-dihydro quinoxalin-2(1H)-one

Using the compound of Example 65-4 as a starting material, the title compound (100 mg, 287.94 µmol, 52.8% yield) was obtained in the same manner as in Example 33-5.

¹H NMR (400 MHz, DMSO-*d*₆, ppm): δ 7.57 - 7.47 (m, 2H), 7.40 - 7.32 (m, 1H), 7.04 - 6.96 (m, 1H), 6.93 - 6.87 (m, 1H), 6.69 - 6.62 (m, 1H), 4.65 - 4.49 (m, 2H), 3.02 - 2.76 (m, 2H).

### Example 65-6: 5-(2,3-dihydrobenzofuran-4-yl)-7-(trifluoromethyl)imidazo[1,2-a]quinoxalin-4(5H)-one

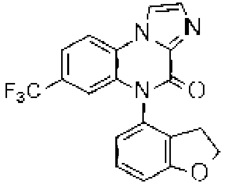

Using the compound of Example 65-5 (140 mg, 403.12 µmol, 1 eq.) as a starting material, the title compound as a yellowish white was obtained (33.66 mg, 90.20 µmol, 22.4% yield, 99.5% purity) in the same manner as in Example 33-6.

¹H NMR (400 MHz, DMSO-*d*₆, ppm): δ 8.79 - 8.71 (m, 1H), 8.53 - 8.44 (m, 1H), 7.82 - 7.70 (m, 2H), 7.42 - 7.34 (m, 1H), 7.04 - 6.98 (m, 1H), 6.94 - 6.90 (m, 1H), 6.85 - 6.82 (m, 1H), 4.67 - 4.47 (m, 2H), 3.08 - 2.77 (m, 2H).

### Example 71: 7-chloro-9-fluoro-5-(o-tolyl)imidazo[1,2-a]quinoxalin-4(5H)-one

Reaction scheme for preparing the compound of Example 71:

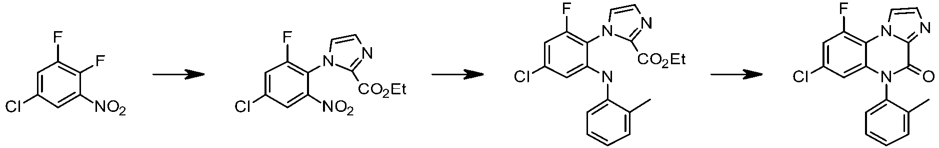

### Example 71-1: ethyl 1-(4-chloro-2-fluoro-6-nitrophenyl)-1H-imidazol-2-carboxylate

5-chloro-1,2-difluoro-3-nitrobenzene (194 g, 1 mmol, 1 eq.) and ethyl 1H-imidazole-2-carboxylate (140 mg, 1 mmol, 1 eq.) were dissolved in N,N-dimethylformamide (5 mL), and cesium carbonate (397 mg, 1.2 mmol, 1.2 eq.) was added. The reaction mixture was stirred at 55 °C for 2 hours. Whether the starting material was consumed was observed through TLC, and when the reaction was completed, after cooling to room temperature and adding water (500 mL), the precipitate was filtered, washed with water (30 mL × 3), and dried to obtain the title compound (250 mg, 767 µmol, 79.7% yield) as a light yellow solid.

¹H NMR (400 MHz, CDCl₃, ppm): δ 8.00 (t, J = 2.2 Hz, 1H), 7.58 (dd, J = 8.5, 2.3 Hz, 1H), 7.41 (d, J = 1.2 Hz, 1H), 7.13 (s, 1H), 4.31 (q, J = 7.1 Hz, 2H), 1.36 (t, J = 7.1 Hz, 3H).

### Example 71-2: ethyl 1-(4-chloro-2-fluoro-6-(o-tolylamino)phenyl)-1H-imidazol-2-carboxylate

The compound obtained in Example 71-1 (100 mg, 319 µmol, 1 eq.), o-tolylboronic acid (86.7 mg, 638 µmol, 2 eq.), and 1,2,2,3,4,4-hexamethylphosphetane 1-oxide (12 mg, 63.8 µmol, 0.2 eq.) were dissolved in toluene (1 mL), and phenylsilane (68.4 mg, 63.8 µmol, 77.9 uL, 2 eq.) was added. The reaction mixture was degassed by purging with nitrogen 3 times, and then stirred at 120 °C for 2 hours under nitrogen atmosphere. After the reaction mixture was cooled to room temperature, after adding water (20 mL), it was extracted with ethyl acetate (30 mL × 2). After the organic layer was washed with saturated aqueous sodium chloride solution (20 mL × 2), the organic layer was dried over anhydrous sodium sulfate, filtered, and concentrated. The residue was purified by column chromatography (stationary phase: silica, mobile phase: petroleum ether/ethyl acetate = 50/1 to 3/1) to obtain the title compound (74 mg, 198 µmol, 62.1% yield) in the form of a white solid.

¹H NMR (400 MHz, CDCl₃, ppm): δ 7.45 (s, 1H), 7.26 - 7.09 (m, 5H), 6.67 (dd, J = 8.9, 2.2 Hz, 1H), 6.54 (s, 1H), 4.35 (q, J = 7.1 Hz, 2H), 2.11 (s, 3H), 1.35 (t, J = 7.1 Hz, 3H).

### Example 71-3: 7-chloro-9-fluoro-5-(o-tolyl)imidazo[1,2-a]quinoxalin-4(5H)-one

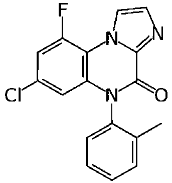

The compound obtained in Example 71-2 (74 mg, 198 µmol, 1.00 eq.) was dissolved in ethanol (2 mL), and potassium carbonate (30.1 mg, 218 µmol, 1.1 eq.) was added. The mixture was stirred at 80 °C for 2 hours. After confirming that the reaction was completed through TLC, the reaction mixture was cooled at room temperature and concentrated under reduced pressure. The residue was purified by column chromatography (stationary phase: silica, mobile phase: dichloromethane/methanol = 100/1 to 20/1) to obtain the title compound (39.5 mg, 121 µmol, 60.9% yield, 99.1% purity) as a light white solid.

¹H NMR (400 MHz, CDCl₃, ppm) δ 8.21 (s, 1H), 7.72 (d, J = 1.2 Hz, 1H), 7.54 - 7.42 (m, 3H), 7.19 (d, J = 7.6 Hz, 1H), 7.15 (dd, J = 11.5, 2.1 Hz, 1H), 6.42 (d, J = 2.0 Hz, 1H), 2.10 (s, 3H).

### Example 72: 8-bromo-7-chloro-5-(o-tolyl)imidazo[1,2-a]quinoxalin-4(5H)-one

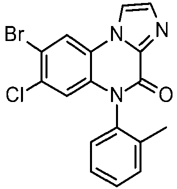

The title compound was synthesized in the same manner as Example 71, changing the starting material to 1-bromo-2-chloro-5-fluoro-4-nitrobenzene.

¹H NMR (400 MHz, CDCl₃, ppm): δ 7.99 (s, 1H), 7.87 (s, 1H), 7.73 (s, 1H), 7.56 - 7.37 (m, 3H), 7.19 (d, J = 7.6 Hz, 1H), 6.70 (s, 1H), 2.10 (s, 3H).

### Example 73: 7-chloro-8-methyl-5-(o-tolyl)imidazo[1,2-a]quinoxalin-4(5H)-one

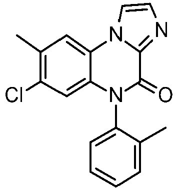

The title compound was synthesized in the same manner as Example 71, changing the starting material to 1-chloro-4-fluoro-2-methyl-5-nitrobenzene.

¹H NMR (400 MHz, CDCl₃, ppm): δ 7.89 (s, 1H), 7.70 (s, 1H), 7.60 (s, 1H), 7.52 - 7.38 (m, 3H), 7.19 (d, J = 7.6 Hz, 1H), 6.60 (s, 1H), 2.48 (s, 3H), 2.09 (s, 3H).

### Example 74: 7-bromo-8-methoxy-5-(o-tolyl)imidazo[1,2-a]quinoxalin-4(5H)-one

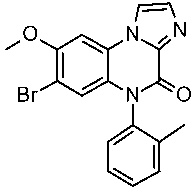

The title compound was synthesized in the same manner as Example 71, changing the starting material to 1-bromo-4-fluoro-2-methoxy-5-nitrobenzene.

¹H NMR (400 MHz, CDCl₃, ppm): δ 7.90 (s, 1H), 7.73 (s, 1H), 7.50 - 7.41 (m, 3H), 7.23 - 7.16 (m, 2H), 6.82 (s, 1H), 4.03 (s, 3H), 2.09 (s, 3H).

### Example 75: 7-bromo-8-methoxy-5-(2-methylpyridin-3-yl)imidazo[1,2-a]quinoxalin-4(5H)-one

Reaction scheme for preparing the compound of Example 75:

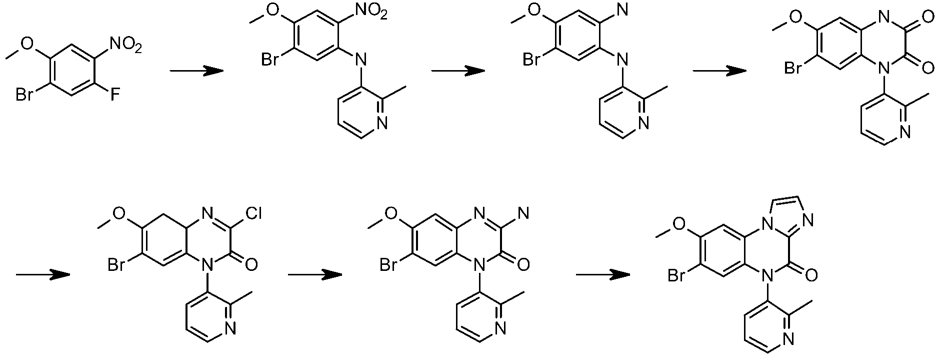

### Example 75-1: N-(5-bromo-4-methoxy-2-nitrophenyl)-2-methylpyridin-3-amine

1-bromo-5-fluoro-2-methoxy-4-nitrobenzene (2.0 g, 18.49 mmol, 1 eq.), 2-methylpyridin-3-amine (5.55 g, 22.19 mmol, 1.2 eq.), and lithium bis(trimethylsilyl)amide (1 M, 36.99 mL, 2 eq.) were added to tetrahydrofuran (30 mL), followed by purging with nitrogen three times. The mixture was stirred under nitrogen at -50 °C for 2 hours. After confirming the formation of a new product by TLC, the reaction was quenched by adding a saturated aqueous ammonium chloride solution, and then extracted with ethyl acetate (200 mL × 3). The organic layer was washed with a saturated aqueous sodium chloride solution (100 mL), dried over anhydrous sodium sulfate, filtered, and concentrated. The resulting concentrate was purified by column chromatography (stationary phase: silica, petroleum ether/ethyl acetate = 10/1 to 3/1) to obtain the title compound (4.9 g, 13.33 mmol, 72.1% yield) as a yellow liquid.

¹H NMR (400 MHz, CDCl₃, ppm) δ 9.05 (s, 1H), 8.39 (dd, J = 1.6, 4.8 Hz, 1H), 7.63 (s, 1H), 7.52 (dd, J = 1.2, 8.0 Hz, 1H), 7.19 - 7.16 (m, 1H), 7.02 (s, 1H), 3.85 (s, 3H), 2.46 (s, 3H).

### Example 75-2: 5-bromo-4-methoxy-N¹-(2-methylpyridin-3-yl)benzen-1,2-diamine

The compound (4.8 g, 14.19 mmol, 1 eq.) obtained in Example 75-1 was dissolved in methanol (40 mL), and Raney-nickel (608.06 mg, 7.10 mmol, 0.2 eq.) was added. The reaction mixture was stirred under hydrogen (15 psi) at room temperature for 12 hours. Upon the disappearance of the starting material and the observation of the m/z ([M + H]+ = 308.0) of the title compound on LCMS, the reaction mixture was filtered and concentrated to obtain the title compound (3.7 g, crude mixture) as a yellow solid. The obtained mixture was used directly in the next reaction.

### Example 75-3: 7-chloro-6-methoxy-1-(2-methylpyridin-3-yl)-1,4-dihydroquinoxalin-2,3-dione

The compound (3.40 g, 11.03 mmol, 1 eq.) obtained in Example 75-2 was dissolved in diethyl oxalate (42.80 g, 109.83 mmol, 40 mL, 26.56 eq.), and after adding N,N-diisopropylethylamine (2.85 g, 22.07 mmol, 3.84 mL, 2 eq.), the mixture was stirred at 160 °C for 3 hours. The m/z ([M + H]+ = 362.0) of the title compound was observed on LCMS. The reaction mixture was concentrated and purified by chromatography (stationary phase: silica, mobile phase: petroleum ether:ethyl acetate = 1/0 to 1/2), and then solidified with ethyl acetate (100 mL). The title compound (1.9 g, 5.25 mmol, 47.5% yield) was obtained in the form of a yellow solid.

### Example 75-4: 7-bromo-3-chloro-6-methoxy-1-(2-methylpyridin-3-yl)dihydroquinoxalin-2(1H)-one

The compound (500 mg, 1.38 mmol, 1 eq.) obtained in Example 75-3, phosphoryl chloride (656.97 mg, 5.52 mmol, 0.4 mL, 4 eq.), and N,N-dimethylformamide (0.05 mL, 3 eq.) were dissolved in toluene (10 mL) and stirred under nitrogen at 80 °C for 3 hours. The reaction mixture was concentrated, and the title compound (320 mg, crude mixture) in the form of a yellow solid was used in the next reaction without further purification.

### Example 75-5: 3-amino-7-bromo-6-methoxy-1-(2-methylpyridin-3-yl)quinoxalin-2(1H)-one

The compound (320 mg, 840.73 µmol, 1 eq.) obtained in Example 75-4 was dissolved in tetrahydrofuran (4 mL), and an aqueous ammonia solution (1.96 g, 16.81 mmol, 2.16 mL, 30% purity, 20 eq.) was added. The reactant was stirred at 25 °C for 12 hours. When the m/z ([M + H]+ = 362.9) of the title compound was observed on LCMS, the reaction mixture was concentrated under reduced pressure and then filtered. The filter cake was concentrated and purified by chromatography (stationary phase: silica, mobile phase: petroleum ether:ethyl acetate = 4/1 to 1/2) to obtain the title compound (270 mg, 747.52 µmol, 88.9% yield) in the form of a yellow solid.

¹H NMR (400 MHz, DMSO-d₆, ppm): δ 8.73 - 8.58 (m, 1H), 7.92 - 7.78 (m, 1H), 7.54 - 7.51 (m, 3H), 7.06 (s, 1H), 6.36 (s, 1H), 3.87 (s, 3H), , 2.27 - 2.16 (s, 3H).

### Example 75-6: 7-bromo-8-methoxy-5-(2-methylpyridin-3-yl)imidazo[1,2-a]quinoxalin-4(5H)-one

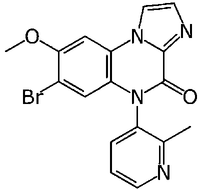

The compound (300 mg, 830.58 mmol, 1 eq.) obtained in Example 75-5 and 2-chloroacetaldehyde (814.98 mg, 4.15 mmol, 668.02 µL, 40% purity, 5 eq.) were added to ethanol (5 mL). The reaction mixture was stirred at 80 °C for 12 hours. The desired m/z ([M + H]+ = 385.2) was detected on LCMS. The reaction mixture was concentrated under reduced pressure and then recrystallized using ethanol to obtain the title compound (118 mg, 292.54 µmol, 35.2% yield, 97.5% purity) in the form of a yellow solid.

¹H NMR (400 MHz, DMSO-*d*₆, ppm): δ 8.83 (s, 1H), 8.72 - 8.71 (m, 1H), 7.96 (s, 1H),7.93-7.91 (s, 1H), 7.73 (s, 1H), 7.60 - 7.56 (m, 1H), 6.62 (s, 1H), 4.01 (s, 3H), 2.25 (s, 3H).

### Example 76: 7-bromo-5-(2-methylpyridin-3-yl)-8-(trifluoromethyl)imidazo[1,2-a]quinoxalin-4(5H)-one

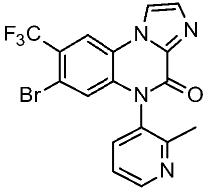

The title compound was synthesized in the same manner as Example 75, changing the starting material to 1-bromo-5-fluoro-4-nitro-2-(trifluoromethyl)benzene.

¹H NMR (400 MHz, CDCl₃, ppm): δ 8.79 (d, J = 3.4 Hz, 1H), 8.06 (s, 1H), 7.99 (d, J = 1.3 Hz, 1H), 7.79 (d, J = 1.3 Hz, 1H), 7.58 (d, J = 7.5 Hz, 1H), 7.49 - 7.43 (m, 1H), 6.90 (s, 1H), 2.38 (s, 3H).

### Example 77: 7-bromo-8-fluoro-5-(2-methylpyridin-3-yl)imidazo[1,2-a]quinoxalin-4(5H)-one

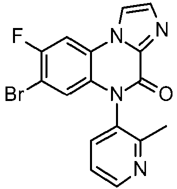

The title compound was synthesized in the same manner as Example 75, changing the starting material to 1-bromo-2,5-difluoro-4-nitrobenzene.

¹H NMR (400 MHz, CDCl₃, ppm): δ 8.84 - 8.68 (m, 1H), 7.86 (d, J = 1.3 Hz, 1H), 7.76 (d, J = 1.3 Hz, 1H), 7.67 - 7.55 (m, 2H), 7.45 (dd, J = 8.0, 4.7 Hz, 1H), 6.76 (d, J = 6.0 Hz, 1H), 2.37 (s, 3H).

### Example 78: 7-bromo-8-methyl-5-(2-methylpyridin-3-yl)imidazo[1,2-a]quinoxalin-4(5H)-one

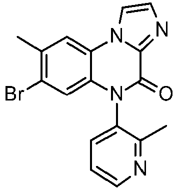

The title compound was synthesized in the same manner as Example 75, changing the starting material to 1-bromo-5-fluoro-2-methyl-4-nitrobenzene.

¹H NMR (400 MHz, CDCl₃, ppm): δ 8.75 (dd, J = 4.9, 1.6 Hz, 1H), 7.92 (d, J = 1.3 Hz, 1H), 7.73 (d, J = 1.2 Hz, 1H), 7.64 (s, 1H), 7.58 (d, J = 7.9 Hz, 1H), 7.43 (dd, J = 8.0, 4.8 Hz, 1H), 6.74 (s, 1H), 2.51 (s, 3H), 2.36 (s, 3H).

### Example 79: 7-bromo-8-chloro-5-(2-methylpyridin-3-yl)imidazo[1,2-a]quinoxalin-4(5H)-one

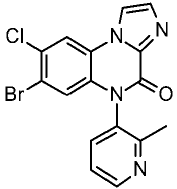

The title compound was synthesized in the same manner as Example 75, changing the starting material to 1-bromo-2-chloro-5-fluoro-4-nitrobenzene.

¹H NMR (400 MHz, CDCl₃, ppm): δ 8.83 - 8.71 (m, 1H), 7.90 (s, 1H), 7.88 (s, 1H), 7.76 (s, 1H), 7.63 - 7.54 (m, 1H), 7.45 (dd, J = 8.0, 4.9 Hz, 1H), 6.82 (s, 1H), 2.37 (s, 3H).

### Example 80: (Ra)-7-bromo-8-chloro-5-(2-methylpyridin-3-yl)imidazo[1,2-a]quinoxalin-4(5H)-one

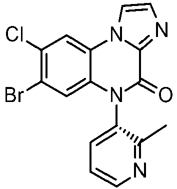

The compound of Example 79 was separated using DAICEL CHIRALCEL OD (250 mm*30 mm, 10 µm); mobile phase: [carbon dioxide-ethanol (0.1% ammonia water)]; B%: 35%, isocratic transfer conditions (retention time: 4.58 min) (ee% = 99.5%) to obtain the title compound as a white solid.

¹H NMR (400 MHz, CDCl₃, ppm): δ 8.83 - 8.71 (m, 1H), 7.90 (s, 1H), 7.88 (s, 1H), 7.76 (s, 1H), 7.63 - 7.54 (m, 1H), 7.45 (dd, J = 8.0, 4.9 Hz, 1H), 6.82 (s, 1H), 2.37 (s, 3H).

### Example 81: (Sa)-7-bromo-8-chloro-5-(2-methylpyridin-3-yl)imidazo[1,2-a]quinoxalin-4(5H)-one

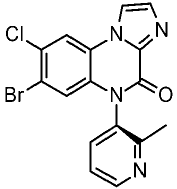

The compound of Example 79 was separated using DAICEL CHIRALCEL OD (250 mm*30 mm, 10 µm); mobile phase: [carbon dioxide-ethanol (0.1% ammonia water)]; B%: 35%, isocratic transfer conditions (retention time: 5.60 min) (ee% = 99.1%) to obtain the title compound as a white solid.

¹H NMR (400 MHz, CDCl₃, ppm): δ 8.83 - 8.71 (m, 1H), 7.90 (s, 1H), 7.88 (s, 1H), 7.76 (s, 1H), 7.63 - 7.54 (m, 1H), 7.45 (dd, J = 8.0, 4.9 Hz, 1H), 6.82 (s, 1H), 2.37 (s, 3H).

### Example 82: 7-bromo-8-(difluoromethyl)-5-(2-methylpyridin-3-yl)imidazo[1,2-a]quinoxalin-4(5H)-one

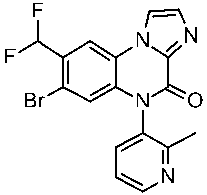

The title compound was synthesized in the same manner as Example 75, changing the starting material to 1-bromo-2-(difluoromethyl)-5-fluoro-4-nitrobenzene.

¹H NMR (400 MHz, DMSO-*d*₆, ppm): δ 8.89 (s, 1H), 8.71 - 8.70 (m, 1H), 8.59 (s, 1H), 7.88 - 7.86 (m, 1H), 7.73 (s, 1H), 7.56 - 7.53 (m, 1H), 7.21 (t, J = 54 Hz, 1H), 6.68 (s, 1H), 2.27 (s, 3H).

### Example 83: 7-bromo-8-chloro-5-(1-methyl-1H-pyrazol-5-yl)imidazo[1,2-a]quinoxalin-4(5H)-one

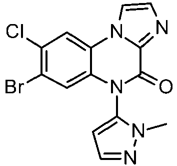

The title compound was synthesized in the same manner as Example 79 by changing 2-methylpyridin-3-amine in Example 75-1 to 1-methyl-1H-pyrazol-5-amine.

¹H NMR (400 MHz, CDCl₃, ppm): δ 7.88 (d, J = 1.4 Hz, 1H), 7.85 (s, 1H), 7.77 - 7.74 (m, 2H), 7.07 (s, 1H), 6.40 (d, J = 2.1 Hz, 1H), 3.71 (s, 3H).

### Example 84: 7-bromo-1,8-dimethyl-5-(2-methylpyridin-3-yl)imidazo[1,2-a]quinoxalin-4(5H)-one

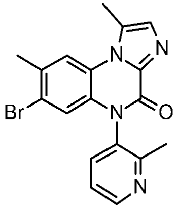

The title compound was synthesized in the same manner as in Example 78 by changing 2-chloroacetaldehyde to 2-chloropropanal in Example 75-6.

¹H NMR (400 MHz, CDCl₃, ppm): δ 8.79 - 8.68 (m, 1H), 8.00 (s, 1H), 7.57 (d, J = 8.0 Hz, 1H), 7.47 - 7.37 (m, 3H), 6.74 (s, 1H), 2.90 (s, 3H), 2.50 (s, 3H), 2.35 (s, 3H).

### Example 85: 7,9-dibromo-1,8-dimethyl-5-(2-methylpyridin-3-yl)imidazo[1,2-a]quinoxalin-4(5H)-one

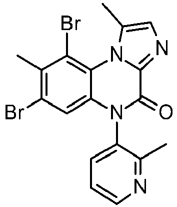

To a solution of the compound of Example 84 (60 mg, 156.58 µmol, 1 eq) dissolved in dimethylformamide (2 mL), a solution of N-bromosuccinimide (31 mg, 172.23 µmol, 1.1 eq) dissolved in dimethylformamide (2 mL) was slowly added dropwise. The reaction mixture was stirred at room temperature for 16 hours. After the reaction was terminated by adding water, the mixture was extracted with dichloromethane (50 mL × 3). The organic layer was washed with a saturated aqueous sodium chloride solution (100 mL), dried over anhydrous sodium sulfate, filtered, and concentrated. The resulting concentrate was purified by column chromatography (stationary phase: silica, mobile phase: dichloromethane:methanol = 1/0 to 10/1) to obtain the title compound (30 mg, 64.91 µmol, 41.5 % yield, 94.5% purity) as a yellow solid.

¹H NMR (400 MHz, CDCl₃, ppm): δ 8.81 - 8.69 (m, 1H), 7.99 (s, 1H), 7.56 (d, J = 7.4 Hz, 1H), 7.43 (dd, J = 7.9, 4.9 Hz, 1H), 6.75 (s, 1H), 2.91 (s, 3H), 2.51 (s, 3H), 2.34 (s, 3H).

### Example 86: 7-bromo-8-chloro-1-methyl-5-(2-methylpyridin-3-yl)imidazo[1,2-a]quinoxalin-4(5H)-one

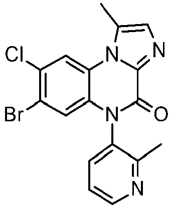

The title compound was synthesized in the same manner as in Example 79 by changing 2-chloroacetaldehyde to 2-chloropropanal in Example 75-6.

¹H NMR (400 MHz, CDCl₃, ppm): δ 8.76 (d, J = 4.6 Hz, 1H), 8.22 (s, 1H), 7.57 (d, J = 7.7 Hz, 1H), 7.49 - 7.40 (m, 2H), 6.82 (s, 1H), 2.90 (s, 3H), 2.36 (s, 3H).

### Example 87: 7-bromo-8-chloro-5-(2-cyclopropylpyridin-3-yl)imidazo[1,2-a]quinoxalin-4(5H)-one

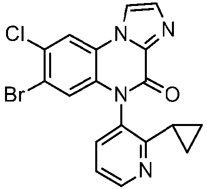

The title compound was synthesized in the same manner as in Example 79 by changing 2-methylpyridin-3-amine to 2-cyclopropylpyridin-3-amine in Example 75-1.

¹H NMR (400 MHz, CDCl₃, ppm): δ 9.25 (s, 1H), 8.77 (d, J = 4.9 Hz, 1H), 7.68 (s, 1H), 7.56 (d, J = 7.9 Hz, 1H), 7.45 (dd, J = 7.9, 4.8 Hz, 1H), 6.83 (s, 1H), 2.36 (s, 3H).

### Example 88: 1,7-dibromo-8-chloro-5-(2-methylpyridin-3-yl)imidazo[1,2-a]quinoxalin-4(5H)-one

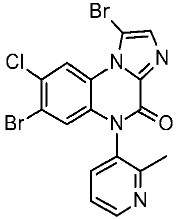

The title compound was synthesized in the same manner as Example 85 using the compound obtained in Example 79 as a starting material.

¹H NMR (400 MHz, CDCl₃, ppm): δ 8.68 (d, J = 4.8 Hz, 1H), 7.89 (s, 1H), 7.87 (s, 1H), 7.75 (s, 1H), 7.54 (d, J = 7.4 Hz, 1H), 7.32 (dd, J = 8.0, 4.7 Hz, 1H), 6.92 (s, 1H), 1.69 - 1.59 (m, 1H), 1.31 - 1.19 (m, 1H), 1.17 - 1.03 (m, 1H), 0.96 - 0.87 (m, 1H), 0.84 - 0.75 (m, 1H).

### Example 89: 7-bromo-1,8-dichloro-5-(2-methylpyridin-3-yl)imidazo[1,2-a]quinoxalin-4(5H)-one

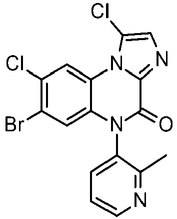

The title compound was synthesized in the same manner as Example 88, by substituting N-chlorosuccinimide for N-bromosuccinimide in Example 83.

¹H NMR (400 MHz, CDCl₃, ppm): δ 9.03 (s, 1H), 8.77 (d, J = 4.9 Hz, 1H), 7.63 (s, 1H), 7.56 (d, J = 7.9 Hz, 1H), 7.49 - 7.40 (m, 1H), 6.83 (s, 1H), 2.37 (s, 3H).

### Example 90: 7-bromo-5-(2-hydroxypyridin-3-yl)-8-methylimidazo[1,2-a]quinoxalin-4(5H)-one

Reaction scheme for preparing the compound of Example 90:

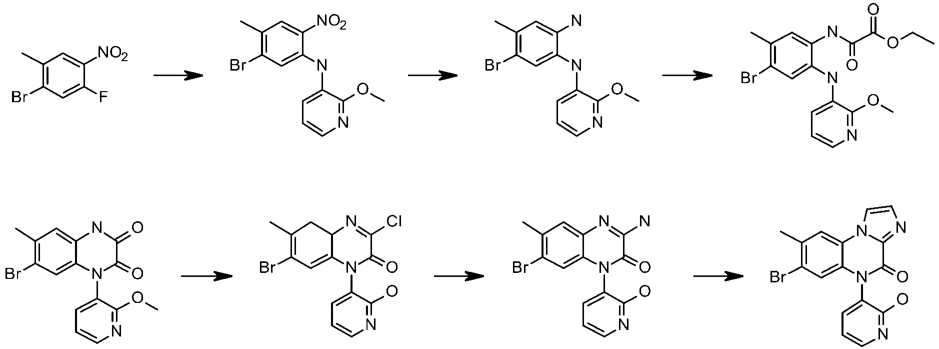

### Example 90-1: N-(5-bromo-4-methyl-2-nitrophenyl)-2-methoxypyridin-3-amine

The starting materials of Example 75-1 were changed to 1-bromo-5-fluoro-2-methyl-4-nitrobenzene and 2-methoxypyridin-3-amine, and the title compound was synthesized in the same manner as Example 75-1.

¹H NMR (400 MHz, DMSO-*d*₆, ppm): δ 9.17 (s, 1H), 8.11 (s, 1H), 8.02 (dd, *J* = 1.2, 4.8 Hz, 1H), 7.80 - 7.71 (m, 1H), 7.16 (s, 1H), 7.07 (dd, J = 4.8 Hz, 1H), 3.90 (s, 3H), 2.30 (s, 3H).

### Example 90-2: 5-bromo-N¹-(2-methoxypyridin-3-yl)-4-methylbenzen-1,2-diamine

The title compound was synthesized in the same manner as in Example 75-2 using the compound of Example 90-1 as a starting material.

¹H NMR (400 MHz, DMSO-*d*₆, ppm): δ 7.50 (dd, J = 1.6, 4.8 Hz, 1H), 7.07 (s, 1H), 6.79 - 6.70 (m, 3H), 6.58 (dd, J = 1.6, 7.6 Hz, 1H), 4.88 (s, 2H), 3.93 (s, 3H), 2.21 (s, 3H).

### Example 90-3: ethyl 2-((4-bromo-2-((2-methoxypyridin-3-yl)amino)-5-methylphenyl)amino)-2-oxoacetate

To a mixture of the compound of Example 90-2 (1.2 g, 3.89 mmol, 1 eq.) and sodium carbonate (453.98 mg, 4.28 mmol, 1.1 eq.) dissolved in dichloromethane (12 mL) and water (3 mL), ethyl 2-chloro-2-oxoacetate (637.98 mg, 4.67 mmol, 522.08 µL, 1.2 eq.) was added, and after purging with nitrogen, the mixture was stirred at room temperature for 2 hours under nitrogen. The reaction was quenched with an aqueous ammonium chloride solution (60 mL), and extracted with ethyl acetate (40 × 3 mL). The organic layer was washed with a saturated aqueous sodium chloride solution (100 mL), dried over anhydrous sodium sulfate, filtered, and concentrated. Petroleum ether (30 mL) was added to the concentrate, and then solidification was performed at room temperature for 30 minutes. The filter cake produced after filtration was dried under reduced pressure to obtain the title compound (1.5 g, 2.99 mmol, 76.9% yield) in the form of a white solid.

### Example 90-4: 7-bromo-1-(2-methoxypyridin-3-yl)-6-methyl-1,4-dihydroquinoxalin-2,3-dione

The compound of Example 90-3 (1.5 g, 3.67 mmol, 1 eq.) was dissolved in tetrahydrofuran (30 mL), sodium hydride (293.91 mg, 7.35 mmol, 60% purity, 2 eq.) was added at 0 °C, and then nitrogen purging was performed. The resulting mixture was stirred for 12 hours at room temperature under nitrogen. The reaction was quenched with aqueous ammonium chloride solution (60 mL), and extraction was performed with ethyl acetate (40 × 3 mL). The organic layer was washed with saturated aqueous sodium chloride solution (100 mL), dried over anhydrous sodium sulfate, filtered, and concentrated. Ethyl acetate (30 mL) was added to the concentrate, and then solidification was performed at room temperature for 30 minutes. The filter cake produced after filtration was dried under reduced pressure to obtain the title compound in the form of a brown solid (640 mg, 1.68 mmol, 45.8 % yield, 95.3 % purity).

¹H NMR (400 MHz, DMSO-*d*₆, ppm): δ 8.41 (d, *J* = 4.4 Hz, 1H), 7.88 (d, *J* = 7.2 Hz, 1H), 7.30-7.27 (m, 1H), 7.20 (s, 1H), 6.46 (s, 1H), 3.84 (s, 3H), 2.31 (s, 3H).

### Example 90-5: 7-bromo-3-chloro-1-(2-hydroxypyridin-3-yl)-6-methylquinoxalin-2(1H)-one

The title compound was synthesized in the same manner as in Example 75-4 using the compound of Example 90-4 as a starting material.

¹H NMR (400 MHz, DMSO-*d*₆, ppm): δ 7.86 (s,1H), 7.80 (dd, *J* = 2.0 Hz, 1H), 7.71 (dd, *J* = 2.0 Hz, 1H), 7.04 (s,1H), 7.48-7.45 (m,1H), 2.42 (s, 3H).

### Example 90-6: 3-amino-7-bromo-1-(2-hydroxypyridin-3-yl)-6-methylquinoxalin-2(1H)-one

The title compound was synthesized in the same manner as in Example 75-5 using the compound of Example 90-5 as a starting material.

### Example 90-7: 7-bromo-5-(2-hydroxypyridin-3-yl)-8-methylimidazo[1,2-a]quinoxalin-4(5H)-one

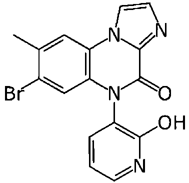

The title compound was synthesized in the same manner as in Example 75-6 using the compound of Example 90-6 as a starting material.

¹H NMR (400 MHz, DMSO-*d*₆, ppm): δ 8.60 (s,1H), 8.30 (s, 1H), 8.37 (s,1H), 7.80-7.79 (m, 1H), 7.68-7.67 (m, 2H), 6.96 (s, 1H), 6.47-6.44 (t, 1H), 2.44 (s, 3H).

### Example 91: 7-bromo-5-(2-chloropyridin-3-yl)-8-methylimidazo[1,2-a]quinoxalin-4(5H)-one

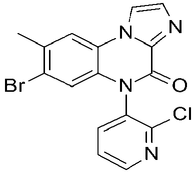

The compound of Example 90 (130 mg, 350.23 µmol, 1 eq.), phosphoryl chloride (3 mL, 32.99 eq.), and N,N-diisopropylethylamine (135.79 mg, 1.05 mmol, 183.01 µL, 3 eq.) were stirred at 110 °C for 24 hours. To the reaction mixture, saturated aqueous sodium bicarbonate solution (15 mL) was slowly added, and then the mixture was extracted with ethyl acetate (20 mL X 3). The organic layer was washed with saturated aqueous sodium chloride solution (50 mL X 2), dried over anhydrous sodium sulfate, filtered, and concentrated under reduced pressure. The resulting concentrate was purified by prep-HPLC (column: Waters Xbridge Prep OBD C18 150*40mm* 10µm; mobile phase: [water (10 mM ammonium bicarbonate)-acetonitrile]; B%: 22%-52%, 15 min) to obtain the title compound (32 mg, 80.73 µmol, 23.1 % yield, 98.3% purity) as a brown solid.

¹H NMR (400 MHz, DMSO-*d*₆, ppm): δ 8.69 - 8.67 (m, 2H), 8.37 (s,1H), 8.21 (dd, J = 2.0 Hz,1H), 7.79-7.85 (m, 1H), 7.74 (d, J = 0.8 Hz, 1H), 6.72 (s, 1H), 2.45 (s, 3H).

### Example 92: 2,7-dibromo-8-chloro-1-methyl-5-(2-methylpyridin-3-yl)imidazo[1,2-a]quinoxalin-4(5H)-one

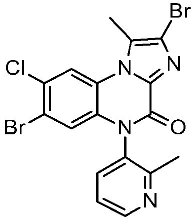

The title compound was synthesized in the same manner as Example 85 using the compound of Example 86 as the starting material.

¹H NMR (400 MHz, CDCl₃, ppm): δ 8.76 (dd, J = 4.7, 1.5 Hz, 1H), 8.22 (s, 1H), 7.62 - 7.53 (m, 1H), 7.45 (dd, J = 8.0, 4.8 Hz, 1H), 6.83 (s, 1H), 2.91 (s, 3H), 2.34 (s, 3H).

### Example 93: 8-methyl-5-(2-methylpyridin-3-yl)-7-(trifluoromethyl)imidazo[1,2-a]quinoxalin4(5H)-one

Reaction scheme for preparing the compound of Example 93

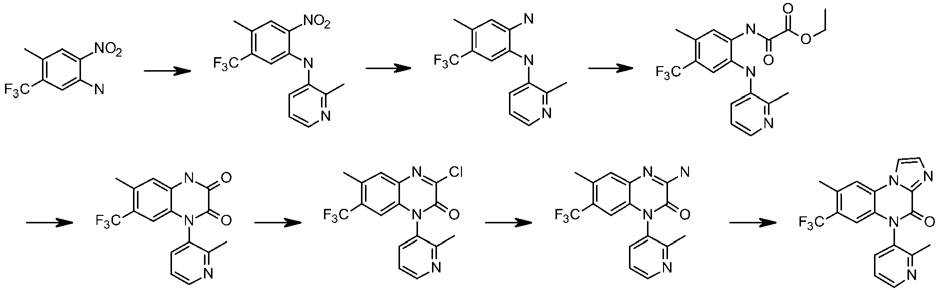

### Example 93-1: 2-methyl-N-(4-methyl-2-nitro-5-(trifluoromethyl)phenyl)pyridin-3-amine

4-methyl-2-nitro-5-(trifluoromethyl)aniline (5 g, 22.71 mmol, 1 eq.), 3-bromo-2-methylpyridine (4.30 g, 24.98 mmol, 1.1 eq.), XPhos (2.17 g, 4.54 mmol, 0.2 eq.), sodium tert-butoxide (3.06 g, 31.80 mmol, 1.4 eq.), and tris(dibenzylideneacetone)dipalladium(0) (2.08 g, 2.27 mmol, 0.1 eq.) were dissolved in toluene (45 mL), and then a nitrogen purge was performed. The resulting mixture was stirred at 90 °C under nitrogen for 12 hours. When the starting material disappeared and the desired m/z([M + H]+ = 312.1) was detected through LC-MS, 20 mL of an aqueous ammonium chloride solution was added, followed by extraction with ethyl acetate (30 mL X 2). The organic layer was dried over anhydrous sodium sulfate and concentrated under reduced pressure, and then the resulting concentrate was purified by column chromatography (stationary phase: silica, mobile phase: petroleum ether:ethyl acetate = 1/0 to 3/1) to obtain the title compound (3.9 g, 12.53 mmol, 55.2% yield, 89.2% purity) as a yellow solid.

¹H NMR (400 MHz, DMSO-*d*₆, ppm): δ 9.32 (s, 1H), 8.41 (d, J = 4.0 Hz, 1H), 8.20 (s, 1H), 7.70 (d, J = 7.6 Hz, 1H), 7.34 (d, J = 7.6, 4.0 Hz, 1H), 6.83 (s, 1H), 2.38 (s, 3H), 2.37 (s, 3H).

### Example 93-2: 4-methyl-N¹-(2-methylpyridin-3-yl)-5-(trifluoromethyl)benzen-1,2-diamine

The title compound was obtained under the same conditions as Example 75-2 using the compound obtained in Example 93-1 as a starting material. The following reaction was performed without further purification.

### Example 93-3: ethyl 2-((5-methyl-2-((2-methylpyridin-3-yl)amino)-4-(trifluoromethyl)phenyl)amino)-2-oxoacetate

The title compound was obtained under the same conditions as in Example 90-3 using the compound obtained in Example 91-2 as a starting material. The following reaction was performed without further purification.

### Example 93-4: 6-methyl-1-(2-methylpyridin-3-yl)-7-(trifluoromethyl)-1,4-dihydroquinoxalin-2,3-dione

The title compound was obtained under the same conditions as Example 90-4 using the compound obtained in Example 93-3 as a starting material.

¹H NMR (400 MHz, DMSO-*d*₆, ppm): δ 12.44 (s, 1H), 8.67 (d, J = 3.6 Hz, 1H), 7.81 (d, J = 8.0 Hz, 1H), 7.52 (dd, J = 4.8, 7.6 Hz, 1H), 7.23 (s, 1H), 6.34 (s, 1H), 2.40 (s, 3H), 2.45 (s, 3H).

### Example 93-5: 3-chloro-6-methyl-1-(2-methylpyridin-3-yl)-7-(trifluoromethyl)quinoxalin-2(1H)-one

The title compound was obtained under the same conditions as in Example 90-5 using the compound obtained in Example 93-4 as a starting material. The following reaction was performed without further purification.

### Example 93-6: 3-amino-6-methyl-1-(2-methylpyridin-3-yl)-7-(trifluoromethyl)quinoxalin-2(1H)-one

The title compound was obtained under the same conditions as Example 90-6 using the compound obtained in Example 93-5 as a starting material.

### Example 93-7: 8-methyl-5-(2-methylpyridin-3-yl)-7-(trifluoromethyl)imidazo[1,2-a]quinoxalin-4(5H)-one

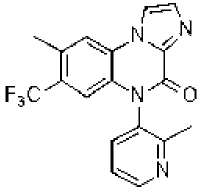

The title compound was obtained under the same conditions as Example 90-7 using the compound obtained in Example 93-6 as a starting material.

¹H NMR (400 MHz, DMSO-*d*₆, ppm): δ 8.78 - 8.61 (m, 2H), 8.43 (s, 1H), 7.95 - 7.84 (m, 1H), 7.74 (s, 1H), 7.60 - 7.47 (m, 1H), 6.58 (s, 1H), 2.54 (s, 3H), 2.24 (s, 3H).

### Example 94: (Ra)-8-methyl-5-(2-methylpyridin-3-yl)-7-(trifluoromethyl)imidazo[1,2-a]quinoxalin4(5H)-one

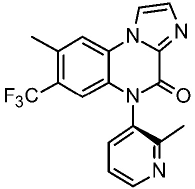

The compound of Example 93 was separated using DAICEL CHIRALCEL OD (250 mm*30 mm, 10 µm); mobile phase: [carbon dioxide-ethanol (0.1% ammonia water)]; B%: 35%, isocratic transfer conditions (retention time: 1.45 min) (ee%>99.9%) to obtain the title compound as a white solid.

¹H NMR (400 MHz, DMSO-*d*₆, ppm): δ 8.78 - 8.61 (m, 2H), 8.43 (s, 1H), 7.95 - 7.84 (m, 1H), 7.74 (s, 1H), 7.60 - 7.47 (m, 1H), 6.58 (s, 1H), 2.54 (s, 3H), 2.24 (s, 3H).

### Example 95: (Sa)-8-methyl-5-(2-methylpyridin-3-yl)-7-(trifluoromethyl)imidazo[1,2-a]quinoxalin-4(5H)-one

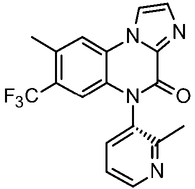

The compound of Example 93 was separated using DAICEL CHIRALCEL OD (250 mm*30 mm, 10 µm); mobile phase: [carbon dioxide-ethanol (0.1% ammonia water)]; B%: 35%, isocratic transfer conditions (retention time: 1.62 min) (ee%=99.8%) to obtain the title compound as a white solid.

¹H NMR (400 MHz, DMSO-*d*₆, ppm): δ 8.78 - 8.61 (m, 2H), 8.43 (s, 1H), 7.95 - 7.84 (m, 1H), 7.74 (s, 1H), 7.60 - 7.47 (m, 1H), 6.58 (s, 1H), 2.54 (s, 3H), 2.24 (s, 3H).

### Example 96: 8-chloro-5-(2-methylpyridin-3-yl)-7-(trifluoromethyl)imidazo[1,2-a]quinoxalin4(5H)-one

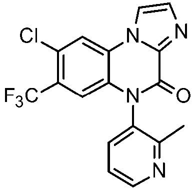

The title compound was obtained in the same manner as in Example 93-1, using 4-chloro-2-nitro-5-(trifluoromethyl)aniline instead of 4-chloro-2-nitro-5-(trifluoromethyl)aniline as the starting material.

¹H NMR (400 MHz, DMSO-*d*₆, ppm): δ 8.81-8.80 (m, 2H), 8.72 (dd, *J* = 1.6, 4.8 Hz, 1H), 7.89 (dd, *J =* 1.6, 7.8 Hz, 1H), 7.78 (d, *J* = 1.2 Hz, 1H), 7.56 (dd, *J* = 4.6, 7.8 Hz, 1H), 6.68 (s, 1H), 2.28 (s, 3H).

### Example 97: (Ra)-8-chloro-5-(2-methylpyridin-3-yl)-7-(trifluoromethyl)imidazo[1,2-a]quinoxalin-4(5H)-one

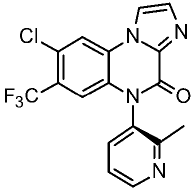

The compound of Example 96 was separated using DAICEL CHIRALPAK AD (250 mm*30 mm, 10 µm); mobile phase: [carbon dioxide-ethanol (0.1% ammonia water)]; B%: 40%, isocratic transfer conditions (retention time: 1.46 min) (ee% = 97.5%) to obtain the title compound as a white solid.

¹H NMR (400 MHz, DMSO-*d*₆, ppm): δ 8.81-8.80 (m, 2H), 8.72 (dd, *J* = 1.6, 4.8 Hz, 1H), 7.89 (dd, *J =* 1.6, 7.8 Hz, 1H), 7.78 (d, *J* = 1.2 Hz, 1H), 7.56 (dd, *J* = 4.6, 7.8 Hz, 1H), 6.68 (s, 1H), 2.28 (s, 3H).

### Example 98: (Sa)-8-chloro-5-(2-methylpyridin-3-yl)-7-(trifluoromethyl)imidazo[1,2-a]quinoxalin4(5H)-one

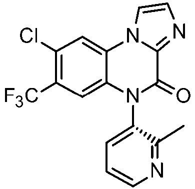

The compound of Example 96 was separated using DAICEL CHIRALPAK AD (250 mm*30 mm, 10 µm); mobile phase: [carbon dioxide-ethanol (0.1% ammonia water)]; B%: 40%, isocratic transfer conditions (retention time: 2.14 min) (ee% = 96.6%) to obtain the title compound as a white solid.

¹H NMR (400 MHz, DMSO-*d*₆, ppm): δ 8.81-8.80 (m, 2H), 8.71 (dd, *J* = 1.6, 4.8 Hz, 1H), 7.88 (dd, *J* = 1.6, 8.0 Hz, 1H), 7.77 (d, *J* = 1.2 Hz, 1H), 7.55 (dd, *J* = 4.8, 8.0 Hz, 1H), 6.67 (s, 1H), 2.27 (s, 3H).

### Example 99: 8-chloro-1-methyl-5-(2-methylpyridin-3-yl)-7-(trifluoromethyl)imidazo[1,2-a]quinoxalin-4(5H)-one

Reaction scheme for preparing the compound of Example 99:

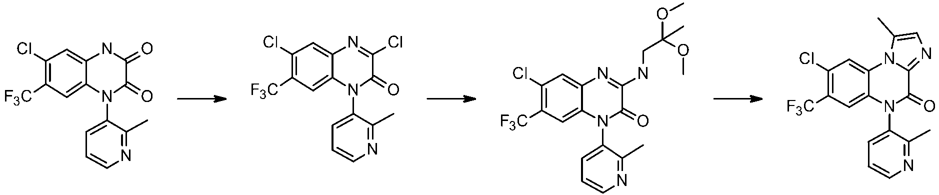

### Example 99-1: 3,6-dichloro-1-(2-methylpyridin-3-yl)-7-(trifluoromethyl)quinoxalin-2(1H)-one

To 6-chloro-1-(2-methylpyridin-3-yl)-7-(trifluoromethyl)-1,4-dihydroquinoxalin-2,3-dione (5.3 g, 14.90 mmol, 1 eq.), a synthetic intermediate of Example 96, thionyl chloride (50 mL) was added, dimethylformamide (108.91 mg, 1.49 mmol, 114.64 µL, 0.1 eq.) was added, and the mixture was stirred at 80 °C for 3 hours after a nitrogen purge. The reaction mixture was dried under reduced pressure to obtain the title compound (1.37 g, crude mixture) as a white solid.

### Example 99-2: 6-chloro-3-((2,2-dimethoxypropyl)amino)-1-(2-methylpyridin-3-yl)-7-(trifluoromethyl)quinoxalin-2(1H)-one

The compound obtained in Example 99-2 (1.0 g, 2.67 mmol, 1 eq.) was dissolved in dimethylformamide (10 mL), and 2,2-dimethoxypropan-1-amine (1.59 g, 13.36 mmol, 5 eq.) and triethylamine (811.36 mg, 8.02 mmol, 1.12 mL, 3 eq.) were added. After purging with nitrogen, the mixture was stirred at 25 °C for 2 hours. After confirming that the desired reaction had occurred through LC-MS, the reaction was terminated by adding ice water (20 mL). The pH of the reaction mixture was adjusted to 3 by adding 1 M aqueous hydrochloric acid, and then extracted with ethyl acetate (20 mL × 3). The organic layer was washed with a saturated aqueous sodium chloride solution (40 mL), dried over anhydrous sodium sulfate, filtered, and concentrated. The resulting concentrate was purified by column chromatography (stationary phase: silica, petroleum ether/ethyl acetate = 0% to 60%) to obtain the title compound (1.05 g, 2.18 mmol, 82% yield, 95% purity) as a white solid.

¹H NMR (400 MHz, DMSO-*d*₆, ppm): δ 8.71 (dd, J = 1.6, 4.8 Hz, 1H), 7.93 (dd, J = 1.6, 7.9 Hz, 1H), 7.80 (t, J = 6.4 Hz, 1H), 7.73 (s, 1H), 7.55 (dd, J = 4.8, 8.0 Hz, 1H), 6.52 (s, 1H), 3.74 (dd, J = 6.8, 13.8 Hz, 1H), 3.67 - 3.52 (m, 1H), 3.21 (d, J = 1.9 Hz, 6H), 2.21 (s, 3H), 1.29 (s, 3H).

### Example 99-3: 8-chloro-1-methyl-5-(2-methylpyridin-3-yl)-7-(trifluoromethyl)imidazo[1,2-a]quinoxalin-4(5H)-one

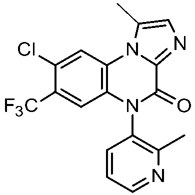

The compound (1.0 g, 2.67 mmol, 1 eq.) obtained in Example 99-2 was dissolved in dimethylformamide (10 mL), and then 2,2-dimethoxypropan-1-amine (1.59 g, 13.36 mmol, 5 eq.) and triethylamine (811.36 mg, 8.02 mmol, 1.12 mL, 3 eq.) were added, and the mixture was stirred at 25 °C for 2 hours after a nitrogen purge. After confirming that the desired reaction had progressed through LC-MS, ice water (20 mL) was added to terminate the reaction. After adjusting the pH of the reaction mixture to 3 by adding a 1 M aqueous hydrochloric acid solution, extraction was performed with ethyl acetate (20 mL × 3). The organic layer was washed with a saturated aqueous sodium chloride solution (40 mL), dried over anhydrous sodium sulfate, filtered, and concentrated. The resulting concentrate was purified by column chromatography (stationary phase: silica, petroleum ether/ethyl acetate = 0% to 60%) to obtain the title compound (1.05 g, 2.18 mmol, 82% yield, 95% purity) as a white solid.

¹H NMR (400 MHz, DMSO-*d*₆, ppm): δ 8.72 (dd, J = 1.6, 4.8 Hz, 1H), 8.40 (s, 1H), 7.90 (dd, J = 1.5, 8.0 Hz, 1H), 7.57 (dd, J = 4.8, 8.0 Hz, 1H), 7.52 (s, 1H), 6.71 (s, 1H), 2.93 (s, 3H), 2.26 (s, 3H).

### Example 100: 8-chloro-2-(dimethylamino)-1-methyl-5-(2-methylpyridin-3-yl)-7-(trifluoromethyl)imidazo[1,2-a]quinoxalin-4(5H)-one

Reaction scheme for preparing the compound of Example 100:

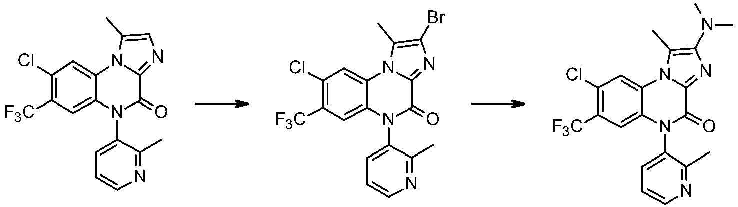

### Example 100-1: 2-bromo-8-chloro-1-methyl-5-(2-methylpyridin-3-yl)-7-(trifluoromethyl)imidazo[1,2-a]quinoxalin-4(5H)-one

Using the compound of Example 99 as a starting material, the title compound (440 mg, 913.29 µmol, 62.9% yield, 97.9% purity) as a white solid was synthesized in the same manner as in Example 85.

¹H NMR (400 MHz, DMSO-*d*₆, ppm): δ 8.73 (dd, J = 1.2, 4.8 Hz, 1H), 8.42 (s, 1H), 7.87 (dd, J = 1.2, 7.6 Hz, 1H), 7.57 (dd, J = 4.8, 8.0 Hz, 1H), 6.73 (s, 1H), 2.93 (s, 3H), 2.26 (s, 3H).

### Example 100-2: 8-chloro-2-(dimethylamino)-1-methyl-5-(2-methylpyridin-3-yl)-7-(trifluoromethyl)imidazo[1,2-a]quinoxalin-4(5H)-one

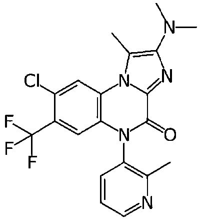

The compound of Example 100-1 (23.54 mg, 49.91 µmol, 1.0 eq.), dimethylamine (2 M, 499.08 µL, 20 eq.), copper(I) iodide (9.50 mg, 49.91 µmol, 1.0 eq.), L-proline (6.90 mg, 59.89 µmol, 1.2 eq.), and potassium tert-butoxide (31.78 mg, 149.72 µmol, 3.0 eq.) were dissolved in dimethyl sulfoxide (1 mL). The reaction mixture was purged with nitrogen and stirred at 100 °C under a nitrogen atmosphere for 12 hours. After the reaction, water (3 mL) was added, and the mixture was extracted with ethyl acetate (3 mL × 2). The organic layer was washed with saturated aqueous sodium chloride (5 mL), dried over anhydrous sodium sulfate, filtered, and concentrated. The resulting residue was purified by prep-HPLC (column: Waters Xbridge 150 × 25 mm, 5 µm; mobile phase: [water (ammonium bicarbonate)-acetonitrile]; B%: 38%-68%, 9 min) to obtain the title compound as a white solid (3 mg, 6.88 µmol, 13.8% yield).

¹H NMR (400 MHz, DMSO-*d*₆, ppm): δ 8.78 (dd, J = 1.6, 4.8 Hz, 1H), 8.29 (s, 1H), 7.56 (dd, J = 1.2, 8.0 Hz, 1H), 7.45 (dd, J = 5.2, 8.0 Hz, 1H), 6.87 (s, 1H), 2.94 (s, 6H), 2.88 (s, 3H), 2.36 (s, 3H).

### Example 101: 7-chloro-9-fluoro-5-(2-methylpyridin-3-yl)imidazo[1,2-a]quinoxalin-4(5H)-one

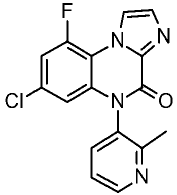

The title compound was synthesized in the same manner as Example 90, changing the starting material to 5-chloro-1,3-difluoro-2-nitrobenzene.

¹H NMR (400 MHz, DMSO-*d*₆, ppm): δ 8.81 - 8.59 (m, 1H), 8.40 (dd, J = 1.4, 2.8 Hz, 1H), 7.91 - 7.82 (m, 1H), 7.74 (d, J = 1.4 Hz, 1H), 7.68 (dd, J = 2.4, 12.0 Hz, 1H), 7.59 - 7.48 (m, 1H), 6.29 (s, 1H), 2.27 (s, 3H).

### Example 102: 7-chloro-9-methoxy-5-(2-methylpyridin-3-yl)imidazo[1,2-a]quinoxalin-4(5H)-one

Reaction scheme for preparing the compound of Example 102:

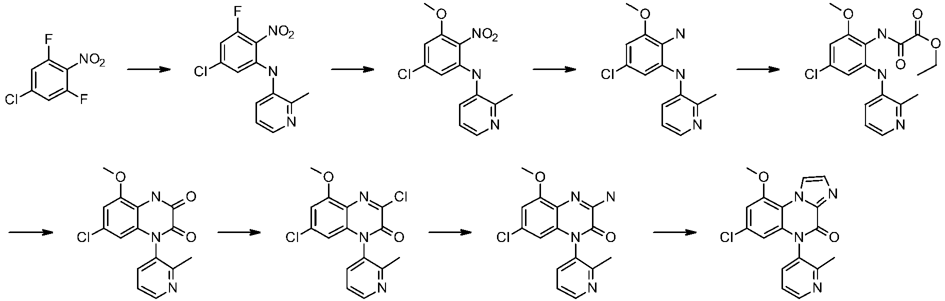

### Example 102-1: N-(5-chloro-3-fluoro-2-nitrophenyl)-2-methylpyridin-3-amine

The title compound was synthesized using 5-chloro-1,3-difluoro-2-nitrobenzene as the starting material in the same manner as Example 90-1.

¹H NMR (400 MHz, DMSO-*d*₆, ppm): δ 8.59 (br s, 1H), 8.50 (dd, J = 1.2, 4.8 Hz, 1H), 7.58 (d, J = 7.8 Hz, 1H), 7.30 - 7.26 (m, 1H), 6.64 (dd, J = 2.0, 10.8 Hz, 1H), 6.49 (t, J = 1.8 Hz, 1H), 2.53 (s, 3H).

### Example 102-2: N-(5-chloro-3-methoxy-2-nitrophenyl)-2-methylpyridin-3-amine

The compound of Example 102-1 (5 g, 17.75 mmol, 1.0 eq.) was dissolved in methanol (50 mL), and sodium methoxide (6.39 g, 35.50 mmol, 30% purity, 2.0 eq.) was added. After three nitrogen purges, the reaction mixture was stirred at 25 °C under a nitrogen atmosphere for 2 hours. Upon confirmation by LC-MS that the starting material had disappeared and the desired m/z was detected, the reaction was quenched by adding water (100 mL). The mixture was then extracted with ethyl acetate (50 mL × 3). The organic layer was washed with saturated aqueous sodium chloride (100 mL), dried over anhydrous sodium sulfate, filtered, and concentrated to afford the title compound as a white solid (5.2 g, crude mixture), which was used directly in the subsequent reaction without further purification.

¹H NMR (400 MHz, CDCl₃, ppm): δ 8.42 (dd, J = 1.4, 4.8 Hz, 1H), 7.72 (s, 1H), 7.55 (dd, J = 1.1, 7.9 Hz, 1H), 7.22 (dd, J = 4.8, 8.0 Hz, 1H), 6.45 (d, J = 2.0 Hz, 1H), 6.36 (d, J = 2.0 Hz, 1H), 3.94 (s, 3H), 2.51 (s, 3H).

### Example 102-3: 5-chloro-3-methoxy-N¹-(2-methylpyridin-3-yl)benzen-1,2-diamine

The title compound was synthesized in the same manner as in Example 90-2 using the compound of Example 102-2 as a starting material.

¹H NMR (400 MHz, CDCl₃, ppm): δ 8.07 (dd, J = 1.2, 4.6 Hz, 1H), 7.02 (dd, J = 4.8, 8.2 Hz, 1H), 6.91 (dd, J = 1.2, 8.0 Hz, 1H), 6.67 (q, J = 2.0 Hz, 2H), 5.07 (s, 1H), 3.89 (s, 3H), 3.77 (br s, 2H), 2.54 (s, 3H).

### Example 102-4: ethyl 2-((4-chloro-2-methoxy-6-((2-methylpyridin-3-yl)amino)phenyl)amino)-2-oxoacetate

The title compound was synthesized in the same manner as Example 90-3 using the compound of Example 102-3 as a starting material.

¹H NMR (400 MHz, CDCl₃, ppm): δ 9.10 (s, 1H), 8.21 (dd, J = 1.4, 4.6 Hz, 1H), 7.37 (dd, J = 1.2, 8.0 Hz, 1H), 7.08 (dd, J = 4.8, 8.0 Hz, 1H), 6.83 (s, 1H), 6.59 (d, J = 1.8 Hz, 1H), 6.54 (d, J = 2.0 Hz, 1H), 4.46 (q, J = 7.2 Hz, 2H), 3.91 (s, 3H), 2.52 (s, 3H), 1.45 (t, J = 7.2 Hz, 3H).

### Example 102-5: 7-chloro-5-methoxy-1-(2-methylpyridin-3-yl)-1,4-dihydroquinoxalin-2,3-dione

The title compound was synthesized in the same manner as in Example 90-4 using the compound of Example 102-4 as a starting material.

¹H NMR (400 MHz, DMSO-*d*₆, ppm): δ 8.60 (d, J = 3.8 Hz, 1H), 7.66 (d, J = 7.6 Hz, 1H), 7.46 (dd, J = 4.8, 7.8 Hz, 1H), 6.65 (d, J = 1.8 Hz, 1H), 5.62 (d, J = 2.0 Hz, 1H), 4.40 (br s, 1H), 3.80 (s, 3H), 3.36 (s, 3H).

### Example 102-6: 3,7-dichloro-5-methoxy-1-(2-methylpyridin-3-yl)quinoxalin-2(1H)-one

The title compound was synthesized in the same manner as in Example 90-5 using the compound of Example 102-5 as a starting material.

### Example 102-7: 3-amino-7-chloro-5-methoxy-1-(2-methylpyridin-3-yl)quinoxalin-2(1H)-one

The title compound was synthesized in the same manner as in Example 90-6 using the compound of Example 102-6 as a starting material.

¹H NMR (400 MHz, DMSO-*d*₆, ppm): δ 8.67 (dd, J = 1.6, 4.8 Hz, 1H), 7.85 (dd, J = 1.4, 7.8 Hz, 1H), 7.52 (dd, J = 4.8, 8.0 Hz, 1H), 6.91 (d, J = 2.0 Hz, 1H), 5.82 (d, J = 2.0 Hz, 1H), 3.88 (s, 3H), 2.16 (s, 3H).

### Example 102-8: 7-chloro-9-methoxy-5-(2-methylpyridin-3-yl)imidazo[1,2-a]quinoxalin-4(5H)-one

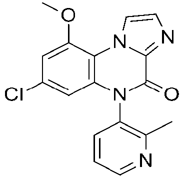

The title compound was synthesized in the same manner as in Example 90-7 using the compound of Example 102-7 as a starting material.

¹H NMR (400 MHz, DMSO-*d*₆, ppm): δ 8.76 (d, J = 1.0 Hz, 1H), 8.69 (dd, J = 1.6, 4.8 Hz, 1H), 7.86 (dd, J = 1.6, 7.8 Hz, 1H), 7.66 (d, J = 1.4 Hz, 1H), 7.54 (dd, J = 4.8, 7.8 Hz, 1H), 7.26 (d, J = 2.2 Hz, 1H), 6.02 (d, J = 2.2 Hz, 1H), 4.13 (s, 3H), 2.23 (s, 3H).

### Example 103: 7-chloro-9-(cyclopropylmethoxy)-5-(2-methylpyridin-3-yl)imidazo[1,2-a]quinoxalin-4(5H)-one

Reaction scheme for preparing the compound of Example 103:

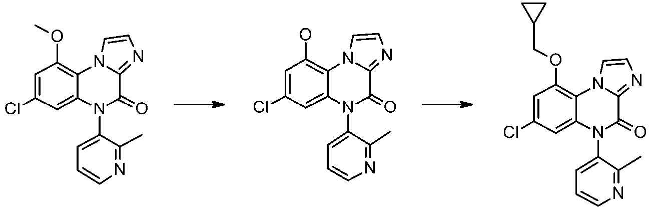

### Example 103-1: 7-chloro-9-hydroxy-5-(2-methylpyridin-3-yl)imidazo[1,2-a]quinoxalin-4(5H)-one

The compound of Example 102 (500 mg, 1.47 mmol, 1.0 eq.) was dissolved in 1,2-dichloroethane (10 mL), and boron tribromide (3.68 g, 14.67 mmol, 10 eq.) was added. The reaction mixture was stirred at 110 °C for 5 hours. Upon confirmation by LC-MS that the starting material had disappeared and the desired m/z was detected, the reaction was quenched by adding ice-cold water (20 mL) and neutralized with saturated aqueous sodium bicarbonate (30 mL). The mixture was then extracted with dichloromethane (50 mL × 3). The organic layer was washed with saturated aqueous sodium chloride (100 mL), dried over anhydrous magnesium sulfate, filtered, and concentrated to afford the title compound as a yellow solid (450 mg, crude mixture), which was used directly in the subsequent reaction without further purification.

¹H NMR (400 MHz, DMSO-*d*₆, ppm): δ 9.13 (s, 1H), 8.67 (dd, J = 1.6, 4.8 Hz, 1H), 7.85 (dd, J = 1.4, 7.8 Hz, 1H), 7.60 (d, J = 1.0 Hz, 1H), 7.53 (dd, J = 4.8, 7.8 Hz, 1H), 6.82 (d, J = 2.2 Hz, 1H), 5.59 (d, J = 1.8 Hz, 1H), 2.23 (s, 3H).

### Example 103-2: 7-chloro-9-(cyclopropylmethoxy)-5-(2-methylpyridin-3-yl)imidazo[1,2-a]quinoxalin-4(5H)-one

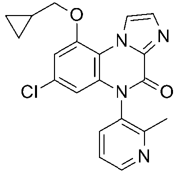

The compound of Example 103-1 (500 mg, 1.53 mmol, 1.0 eq.), bromomethylcyclopropane (413.18 mg, 3.06 mmol, 292.21 µL, 2.0 eq.), and potassium carbonate (634.48 mg, 4.59 mmol, 3.0 eq.) were added to dimethylformamide (5 mL). After three nitrogen purges, the reaction mixture was stirred at 25 °C under a nitrogen atmosphere for 5 hours. Upon confirmation by LC-MS that the starting material had disappeared and the desired m/z was detected, the reaction was quenched by adding ice-cold water (10 mL). The mixture was stirred for 10 minutes and then neutralized to pH = 8 with saturated aqueous sodium bicarbonate (30 mL). The precipitated solid in the aqueous layer was filtered, and the filtrate was extracted with ethyl acetate (20 mL × 3). The organic layer was washed with saturated aqueous sodium chloride (60 mL), dried over anhydrous sodium sulfate, filtered, and concentrated. The resulting residue was purified by prep-HPLC (column: Waters Xbridge Prep OBD C18 150 × 40 mm, 10 µm; mobile phase: [water (0.1% ammonium bicarbonate)-acetonitrile]; B%: 28%-58%, 15 min) to afford the title compound as a white solid (112 mg, 291.16 µmol, 19.0% yield, 99% purity).

¹H NMR (400 MHz, DMSO-*d*₆, ppm): δ 8.85 (d, J = 1.0 Hz, 1H), 8.70 (dd, J = 1.6, 4.8 Hz, 1H), 7.87 (dd, J = 1.4, 7.8 Hz, 1H), 7.71 (d, J = 1.0 Hz, 1H), 7.54 (dd, J = 4.8, 7.8 Hz, 1H), 7.24 (d, J = 2.0 Hz, 1H), 6.01 (d, J = 2.0 Hz, 1H), 4.41 - 3.93 (m, 2H), 2.24 (s, 3H), 1.58 - 1.34 (m, 1H), 0.79 - 0.63 (m, 2H), 0.52 - 0.32 (m, 2H).

### Example 104: 7-chloro-5-(2-methylpyridin-3-yl)-9-(pyrrolidine-1-yl)imidazo[1,2-a]quinoxalin-4(5H)-one

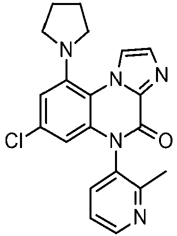

The compound of Example 101 (70 mg, 212.94 µmol, 1.0 eq.), pyrrolidine (75.72 mg, 1.06 mmol, 88.88 µL, 5.0 eq.), and diisopropylethylamine (82.56 mg, 638.83 µmol, 111.27 µL, 3.0 eq.) were dissolved in dimethylacetamide (1 mL). After three nitrogen purges, the reaction mixture was stirred at 100 °C under a nitrogen atmosphere for 2 hours. Upon confirmation by LC-MS that the starting material had disappeared and the desired m/z was detected, the reaction was quenched by adding water (5 mL). The mixture was then extracted with ethyl acetate (5 mL × 2). The organic layer was washed with saturated aqueous sodium chloride (10 mL), dried over anhydrous sodium sulfate, filtered, and concentrated. The resulting residue was purified by prep-HPLC (column: Waters Xbridge 150 × 25 mm, 5 µm; mobile phase: [water (0.1% ammonium bicarbonate)-acetonitrile]; B%: 35%-65%, 9 min) to afford the title compound as a white solid (18 mg, 47.34 µmol, 22.23% yield, 99.9% purity).

¹H NMR (400 MHz, DMSO-*d*₆, ppm): δ 8.86 (d, J = 0.8 Hz, 1H), 8.79 - 8.69 (m, 1H), 7.67 (d, J = 0.8 Hz, 1H), 7.64 (br d, J = 7.2 Hz, 1H), 7.48 (dd, J = 4.8, 8.0 Hz, 1H), 7.15 (d, J = 2.0 Hz, 1H), 6.24 (d, J = 2.0 Hz, 1H), 3.53 - 3.24 (m, 2H), 3.04 - 2.70 (m, 2H), 2.41 (s, 3H), 2.15 - 2.05 (m, 4H).

### Example 105: 7-chloro-9-(methylamino)-5-(2-methylpyridin-3-yl)imidazo[1,2-a]quinoxalin-4(5H)-one

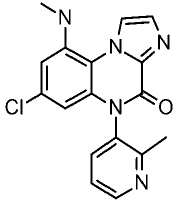

In Example 104, the reactant pyrrolidine was changed to methylamine, and the temperature was changed from 100 °C to 120 °C, and the title compound was synthesized in the same way.

¹H NMR (400 MHz, DMSO-*d*₆, ppm): δ 8.77 (d, J = 1.2 Hz, 1H), 8.69 (dd, J = 1.6, 4.8 Hz, 1H), 7.86 (dd, J = 1.6, 8.9 Hz, 1H), 7.69 (d, J = 1.2 Hz, 1H), 7.54 (dd, J = 4.8, 8.0 Hz, 1H), 6.80 (d, J = 2.0 Hz, 1H), 6.10 (br d, J = 4.4 Hz, 1H), 5.76 (d, J = 2.0 Hz, 1H), 2.87 (d, J = 4.8 Hz, 3H), 2.22 (s, 3H).

### Example 106: 7-chloro-9-(dimethylamino)-5-(2-methylpyridin-3-yl)imidazo[1,2-a]quinoxalin-4(5H)-one

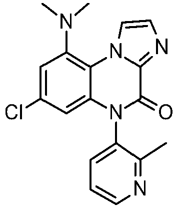

In Example 104, the reactant pyrrolidine was changed to dimethylamine, and the temperature was changed from 100 °C to 120 °C, and the title compound was synthesized in the same way.

¹H NMR (400 MHz, DMSO-*d*₆, ppm): δ 9.04 (d, J = 1.2 Hz, 1H), 8.69 (dd, J = 1.6, 4.8 Hz, 1H), 7.84 (dd, J = 1.6, 8.0 Hz, 1H), 7.66 (d, J = 1.2 Hz, 1H), 7.53 (dd, J = 4.8, 8.0 Hz, 1H), 7.30 (d, J = 2.4 Hz, 1H), 6.13 (d, J = 2.0 Hz, 1H), 2.75 (d, J = 4.8 Hz, 6H), 2.24 (s, 3H).

### Example 107: 7-bromo-8-chloro-5-(2-methoxypyridin-3-yl)imidazo[1,2-a]quinoxalin-4(5H)-one

Reaction scheme for preparing the compound of Example 107:

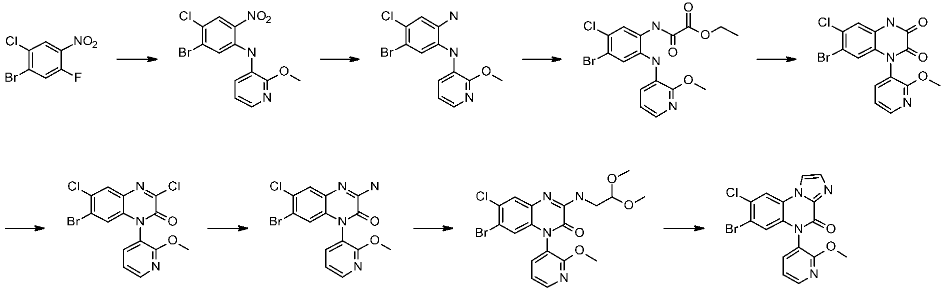

### Example 107-1: N-(5-bromo-4-chloro-2-nitrophenyl)-2-methoxypyridin-3-amine

2-Methoxypyridin-3-amine (1.76 g, 14.15 mmol, 1.2 eq.) was dissolved in tetrahydrofuran (30 mL), and sodium hydride (707.37 mg, 17.69 mmol, 60% purity, 1.5 eq.) was added under nitrogen at 0 °C. After stirring at 0 °C for 1 hour, 1-bromo-2-chloro-5-fluoro-4-nitrobenzene (3 g, 11.79 mmol, 1.0 eq.) was added. The reaction mixture was then warmed to 25 °C and stirred for 11 hours. Upon confirmation by LC-MS that the starting material had disappeared and the desired m/z was detected, the reaction was quenched by adding aqueous ammonium chloride (50 mL). The mixture was extracted with ethyl acetate (30 mL × 3). The organic layer was washed with saturated aqueous sodium chloride (30 mL × 3), dried over anhydrous sodium sulfate, filtered, and concentrated. The resulting residue was purified by silica column chromatography (mobile phase: ethyl acetate/petroleum ether; B%: 0-60%) to afford the title compound as a yellow solid (2.5 g, 5.58 mmol, 47.31% yield, 80% purity).

### Example 107-2: 5-bromo-4-chloro-N¹-(2-methoxypyridin-3-yl)benzen-1,2-diamine

The title compound was synthesized in the same manner as in Example 75-2 using the compound of Example 107-1 as a starting material.

### Example 107-3: ethyl 2-((4-bromo-5-chloro-2-((2-methoxypyridin-3-yl)amino)phenyl)amino)-2-oxoacetate

The title compound was synthesized in the same manner as in Example 90-3 using the compound of Example 107-2 as a starting material.

### Example 107-4: 7-bromo-6-chloro-1-(2-methoxypyridin-3-yl)-1,4-dihydroquinoxalin-2,3-dione

The title compound was synthesized in the same manner as in Example 90-4 using the compound of Example 107-3 as a starting material.

### Example 107-5: 7-bromo-3,6-dichloro-1-(2-methoxypyridin-3-yl)quinoxalin-2(1H)-one

The title compound was synthesized in the same manner as in Example 90-4 using the compound of Example 107-4 as a starting material.

### Example 107-6: 3-amino-7-bromo-6-chloro-1-(2-methoxypyridin-3-yl)quinoxalin-2(1H)-one

The compound of Example 107-5 (2.07 g, 5.17 mmol, 1.0 eq.) was dissolved in 2,2-dimethoxyethanamine (10 mL). The reaction mixture was stirred at 25 °C for 1 hour. Upon confirmation by LC-MS that the starting material had disappeared and the desired m/z was detected, the reaction was quenched by adding water (30 mL). The mixture was then extracted with ethyl acetate (20 mL × 2). The organic layer was washed with saturated aqueous sodium chloride (20 mL), dried over anhydrous sodium sulfate, filtered, and concentrated to afford the title compound as a yellow solid (2.2 g, crude mixture), which was used directly in the subsequent reaction without further purification.

### Example 107-7: 7-bromo-6-chloro-3-((2,2-dimethoxyethyl)amino)-1-(2-methoxypyridin-3-yl)quinoxalin-2(1H)-one

The title compound was synthesized in the same manner as in Example 99-2, using the compound of Example 107-6 as a starting material and using 2,2-dimethoxyethane-1-amine as a reactant instead of 2,2-dimethoxypropan-1-amine, the reactant of Example 99-2.

### Example 107-8: 7-bromo-8-chloro-5-(2-methoxypyridin-3-yl)imidazo[1,2-a]quinoxalin-4(5H)-one

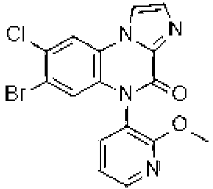

The title compound was synthesized in the same manner as in Example 99-3 using the compound of Example 107-7 as a starting material.

¹H NMR (400 MHz, DMSO-*d*₆, ppm): 8.71 (d, J=14.8 Hz, 2 H), 8.44 (br d, J = 4.0 Hz, 1 H), 7.93 (br d, J = 7.2 Hz, 1 H), 7.30 (dd, J = 7.2, 5.2 Hz, 1 H), 7.72 (s, 1 H), 6.86 (s, 1 H), δ 3.83 (s, 3 H).

### Example 108: 5-(benzo[d]thiazol-7-yl)-7-bromo-8-chloroimidazo[1,2-a]quinoxalin-4(5H)-one

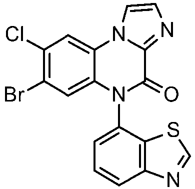

The title compound was synthesized in the same manner as Example 79, using benzo[d]thiazol-7-amine instead of 2-methylpyridin-3-amine in Example 5-1.

¹H NMR (400 MHz, DMSO-*d*₆, ppm): δ 9.48 (s, 1H), 8.81 - 8.70 (m, 2H), 8.37 (dd, J = 0.8, 8.0 Hz, 1H), 7.85 (s, 1H), 7.75 (d, J = 1.2 Hz, 1H), 7.69 (d, J = 7.6 Hz, 1H), 6.69 (s, 1H).

### Example 109: 7-bromo-8-chloro-5-(1H-indol-4-yl)imidazo[1,2-a]quinoxalin-4(5H)-one

Reaction scheme for preparing the compound of Example 109:

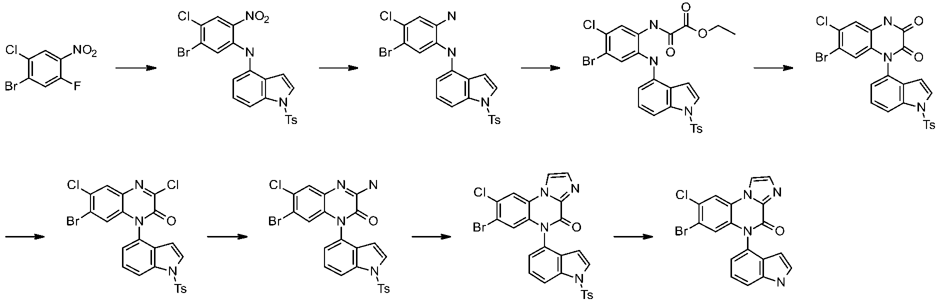

### Example 109-1: N-(5-bromo-4-chloro-2-nitrophenyl)-1-tosy-1H-indol-4-amine

The title compound was synthesized in the same manner as in Example 107-1, using 1-tosyl-1H-indol-4-amine instead of 2-methoxypyridin-3-amine, the starting material of Example 107-1.

¹H NMR (400 MHz, DMSO-*d*₆, ppm): δ 9.59 - 9.52 (m, 1H), 8.33 - 8.25 (m, 1H), 7.92 - 7.78 (m, 4H), 7.46 - 7.34 (m, 3H), 7.27 - 7.19 (m, 1H), 6.99 - 6.94 (m, 1H), 6.74 - 6.65 (m, 1H), 2.36 - 2.27 (m, 3H).

### Example 109-2: 5-bromo-4-chloro-N¹-(1-tosyl-1H-indol-4-yl)benzen-1,2-diamine

The title compound was synthesized in the same manner as in Example 107-2 using the compound of Example 109-1 as a starting material.

### Example 109-3: ethyl 2-((4-bromo-5-chloro-2-((1-tosyl-1H-indol-4-yl)amino)phenyl)amino)-2-oxoacetate

The title compound was synthesized in the same manner as in Example 107-3 using the compound of Example 109-2 as a starting material.

### Example 109-4: 7-bromo-6-chloro-1-(1-tosyl-1H-indol-4-yl)-1,4-dihydroquinoxalin-2,3-dione

The title compound was synthesized in the same manner as in Example 107-4 using the compound of Example 109-3 as a starting material.

### Example 109-5: 7-bromo-3,6-dichloro-1-(1-tosyl-1H-indol-4-yl)quinoxalin-2(1H)-one

The title compound was synthesized in the same manner as in Example 75-4 using the compound of Example 109-4 as a starting material.

### Example 109-6: 3-amino-7-bromo-6-chloro-1-(1-tosyl-1H-indol-4-yl)quinoxalin-2(1H)-one

The title compound was synthesized in the same manner as in Example 75-5 using the compound of Example 109-5 as a starting material.

¹H NMR (400 MHz, CDCl₃, ppm): δ 8.27 - 8.14 (m, 1H), 7.90 - 7.79 (m, 2H), 7.67 - 7.60 (m, 2H), 7.58 - 7.50 (m, 1H), 7.36 - 7.29 (m, 2H), 7.22 - 7.17 (m, 1H), 6.67 - 6.58 (m, 1H), 6.29 - 6.20 (m, 1H), 6.15 - 5.65 (m, 2H), 1.91 - 1.61 (m, 3H).

### Example 109-7: 7-bromo-8-chloro-5-(1-tosyl-1H-indol-4-yl)imidazo[1,2-a]quinoxalin-4(5H)-one

The title compound was synthesized in the same manner as in Example 75-6 using the compound of Example 109-6 as a starting material.

### Example 109-8: 7-bromo-8-chloro-5-(1H-indol-4-yl)imidazo[1,2-a]quinoxalin-4(5H)-one

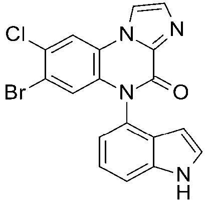

The compound of Example 109-7 (136 mg, 191.60 µmol, 1.0 eq.) was dissolved in methanol (1.5 mL), and sodium hydroxide (23.45 mg, 586.31 µmol, 3.06 eq.) was added at 25 °C. The reaction mixture was stirred at 65 °C for 4 hours. Upon confirmation by LC-MS that the starting material had disappeared and the desired m/z was detected, the reaction mixture was concentrated under reduced pressure. The resulting residue was purified by prep-HPLC (column: Waters Xbridge 150 × 25 mm, 5 µm; mobile phase: [water (0.1% ammonium bicarbonate)-acetonitrile]; B%: 35%-65%, 9 min) to afford the title compound as a white solid (42.76 mg, 103.10 µmol, 48.5% yield, 99.7% purity).

¹H NMR (400 MHz, DMSO-*d*₆, ppm): δ 11.55 - 11.47 (m, 1H), 8.75 - 8.70 (m, 1H), 8.69 - 8.65 (m, 1H), 7.72 - 7.69 (m, 1H), 7.66 - 7.60 (m, 1H), 7.43 - 7.39 (m, 1H), 7.36 - 7.29 (m, 1H), 7.11 - 7.07 (m, 1H), 6.63 - 6.59 (m, 1H), 6.27 - 6.20 (m, 1H).

### Example 110: 8-chloro-5-(2-ethylpyridin-3-yl)-7-(trifluoromethyl)imidazo[1,2-a]quinoxalin-4(5H)-one

Reaction scheme for preparing the compound of Example 110:

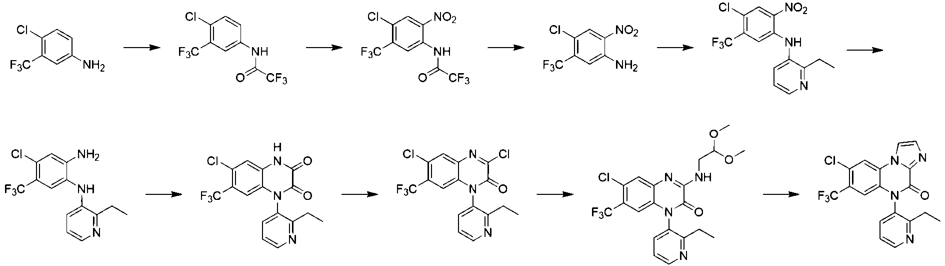

### Example 110-1: N-(4-chloro-3-(trifluoromethyl)phenyl)-2,2,2-trifluoroacetamide

4-Chloro-3-(trifluoromethyl)aniline (5 g, 25.57 mmol, 1.0 eq.) was dissolved in dichloromethane (16.5 mL), and pyridine (10.11 g, 127.83 mmol, 10.32 mL, 5.0 eq.) and (CF₃CO)₂O (5.91 g, 28.12 mmol, 3.91 mL, 1.1 eq.) were added dropwise at 0 °C. The reaction mixture was stirred at 25 °C for 2 hours. Completion of the reaction was observed by TLC. The reaction mixture was washed with saturated aqueous ammonium chloride (40 mL × 3), dried over anhydrous sodium sulfate, filtered, and concentrated to afford the title compound as an orange solid (7 g, 24.01 mmol, 93.9% yield) without further purification.

¹H NMR (400 MHz, CDCl₃, ppm): δ 8.12 - 7.95 (m, 1H), 7.94 - 7.88 (m, 1H), 7.87 - 7.76 (m, 1H), 7.62 - 7.52 (m, 1H).

### Example 110-2: N-(4-chloro-2-nitro-5-(trifluoromethyl)phenyl)-2,2,2-trifluoroacetamide

CF₃SO₃H (10.09 g, 67.22 mmol, 5.95 mL, 2.8 eq.) was dissolved in dichloromethane (37 mL), and nitric acid (2.68 g, 41.68 mmol, 1.91 mL, 98% purity, 1.74 eq.) was added at 0 °C. After the resulting reaction mixture was stirred at 0 °C for 1 hour, a solution of the mixture obtained in Example 110-1 (7 g, 24.01 mmol, 1 eq.) dissolved in dichloromethane (37 mL) was added dropwise to the reaction mixture at 0 °C, and then the mixture was stirred at 25 °C for 12 hours. After confirming that the reaction was complete by TLC, the reaction was terminated by adding a saturated aqueous sodium bicarbonate solution (50 mL), and extraction was performed with dichloromethane (20 mL X 2). The organic layer was washed with a saturated aqueous sodium chloride solution (20 mL), dried over anhydrous sodium sulfate, filtered, and concentrated. The concentrate was purified by flash chromatography (stationary phase: silica, ethyl acetate/petroleum ether 0-12%) to obtain the title compound (6.56 g, 19.50 mmol, 81.2% yield) in the form of a yellow solid.

¹H NMR (400 MHz, CDCl₃, ppm): δ 11.42 - 11.09 (m, 1H), 9.28 - 9.13 (m, 1H), 8.57 - 8.40 (m, 1H).

### Example 110-3: 4-chloro-2-nitro-5-(trifluoromethyl)aniline

The compound obtained in Example 110-2 (6.56 g, 19.50 mmol, 1 eq.) and potassium carbonate (3.23 g, 23.40 mmol, 1.2 eq.) were added to methanol (35 mL) and purified water (35 mL), and nitrogen purging was performed 3 times. The reaction mixture was stirred at 25 °C for 2 hours under nitrogen. After confirming completion of the reaction by TLC, the mixture was concentrated under reduced pressure to remove methanol and extracted with ethyl acetate (60 mL X 2). The organic layer was dried over anhydrous sodium sulfate, filtered, and concentrated. The concentrate was purified by flash chromatography (stationary phase: silica, ethyl acetate/petroleum ether 0-16%) to obtain the title compound (4.48 g, 18.63 mmol, 95.5% yield) in the form of a yellow liquid.

¹H NMR (400 MHz, CDCl₃, ppm): δ 8.19 - 8.06 (m, 1H), 7.90 - 7.71 (m, 2H), 7.65 - 7.52 (m, 1H).

### Example 110-4: N-(4-chloro-2-nitro-5-(trifluoromethyl)phenyl)-2-ethylpyridin-3-amine

The compound obtained in Example 110-3 (4 g, 16.63 mmol, 1 eq.), 3-bromo-2-ethyl-pyridine (3.40 g, 18.29 mmol, 1.1 eq.), Xantphos (1.92 g, 3.33 mmol, 0.2 eq.), cesium carbonate (16.25 g, 49.88 mmol, 3 eq.), and tris(dibenzylideneacetone)dipalladium(0) (1.52 g, 1.66 mmol, 0.1 eq.) were added to dioxane (80 mL), and a nitrogen purge was performed 3 times. The resulting reaction mixture was stirred at 100 °C for 12 hours under nitrogen. After confirming completion of the reaction by TLC, the reaction mixture was filtered, and the filtrate was concentrated. The concentrate was purified by flash chromatography (stationary phase: silica, ethyl acetate/petroleum ether 0-16%) to obtain the title compound (3.9 g, 11.28 mmol, 67.8% yield) in the form of a yellow solid.

¹H NMR (400 MHz, CDCl₃, ppm): δ 9.47 - 9.23 (m, 1H), 8.65 - 8.52 (m, 1H), 8.44 - 8.31 (m, 1H), 7.67 - 7.53 (m, 1H), 7.36 - 7.22 (m, 1H), 7.21 - 7.09 (m, 1H), 2.94 - 2.75 (m, 2H), 1.31 - 1.25 (m, 3H).

### Example 110-5: 4-chloro-N¹-(2-ethylpyridin-3-yl)-5-(trifluoromethyl)benzen-1,2-diamine

The title compound was synthesized using the compound of Example 110-4 as a starting material in the same manner as in Example 75-2.

¹H NMR (400 MHz, DMSO-*d*₆, ppm): δ 8.03 - 7.96 (m, 1H), 7.11 - 7.07 (m, 1H), 7.06 - 6.99 (m, 1H), 6.94 - 6.87 (m, 2H), 6.75 - 6.67 (m, 1H), 5.83 - 5.74 (m, 2H), 2.85 - 2.68 (m, 2H), 1.32 - 1.16 (m, 3H).

### Example 110-6: 6-chloro-1-(2-ethylpyridin-3-yl)-7-(trifluoromethyl)-1,4-dihydroquinoxalin-2,3-dione

The compound obtained in Example 110-5 (3.14 g, 9.95 mmol, 1 eq.) and triethylamine (2.22 g, 21.89 mmol, 3.05 mL, 2.2 eq.) were added to dichloromethane (95 mL), and ethyl 2-chloro-2-oxoacetate (2.72 g, 19.90 mmol, 2.22 mL, 2 eq.) was added at 0 °C. The reaction mixture was stirred at 25 °C for 2 hours. When the completion of the reaction was observed by LC-MS, the reaction mixture was filtered, and the filtrate was concentrated. Tetrahydrofuran (50 mL) was added to the concentrate, and an aqueous sodium hydroxide solution (10%) (25 mL) was added thereto, followed by stirring at 25 °C for 30 minutes. After adding purified water (75 mL) to the reaction mixture, extraction was performed with ethyl acetate (100 mL X 3). The organic layer was dried over anhydrous sodium sulfate, filtered, and concentrated. The concentrate was purified by flash chromatography (stationary phase: silica, ethyl acetate/petroleum ether 0~51%) to obtain the title compound (1.3 g, 3.52 mmol, 35.3% yield) in the form of a yellow solid.

¹H NMR (400 MHz, DMSO-*d*₆, ppm): δ 12.62 - 12.46 (m, 1H), 8.82 - 8.71 (m, 1H), 7.85 - 7.76 (m, 1H), 7.57 - 7.49 (m, 1H), 7.47 - 7.43 (m, 1H), 6.43 - 6.39 (m, 1H), 2.59 - 2.52 (m, 2H), 1.11 - 1.06 (m, 3H).

### Example 110-7: 3,6-dichloro-1-(2-ethylpyridin-3-yl)-7-(trifluoromethyl)quinoxalin-2(1H)-one

The title compound was synthesized using the compound of Example 110-6 as a starting material in the same manner as in Example 33-4.

¹H NMR (400 MHz, DMSO-*d*₆, ppm): δ 8.94 - 8.91 (m, 1H), 8.27 - 8.14 (m, 1H), 7.94 - 7.80 (m, 1H), 7.58 - 7.50 (m, 1H), 6.76 - 6.63 (m, 1H), 2.81 - 2.68 (m, 2H), 1.16 - 1.11 (m, 3H).

### Example 110-8: 6-chloro-3-((2,2-dimethoxyethyl)amino)-1-(2-ethylpyridin-3-yl)-7-(trifluoromethyl)quinoxalin-2(1H)-one

The title compound was synthesized using the compound of Example 110-7 as a starting material in the same manner as in Example 107-7.

¹H NMR (400 MHz, CDCl₃, ppm): δ 8.86 - 8.78 (m, 1H), 7.73 - 7.67 (m, 1H), 7.56 - 7.47 (m, 1H), 7.46 - 7.38 (m, 1H), 6.86 - 6.75 (m, 1H), 6.68 - 6.58 (m, 1H), 4.67 - 4.58 (m, 1H), 3.88 - 3.64 (m, 2H), 3.52 - 3.40 (m, 6H), 2.61 - 2.39 (m, 2H), 1.19 (t, *J* = 7.4 Hz, 3H).

### Example 110-9: 8-chloro-5-(2-ethylpyridin-3-yl)-7-(trifluoromethyl)imidazo[1,2-a]quinoxalin-4(5H)-one

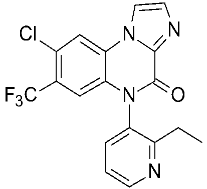

The title compound was synthesized using the compound of Example 110-8 as a starting material in the same manner as in Example 99-3.

¹H NMR (400 MHz, DMSO-*d*₆, ppm): δ 8.85 - 8.76 (m, 3H), 7.91 - 7.85 (m, 1H), 7.79 - 7.74 (m, 1H), 7.61 - 7.53 (m, 1H), 6.68 - 6.62 (m, 1H), 2.58 - 2.52 (m, 2H), 1.16 - 1.04 (m, 3H).

### Example 111: (Ra)-8-chloro-5-(2-ethylpyridin-3-yl)-7-(trifluoromethyl)imidazo[1,2-a]quinoxalin-4(5H)-one

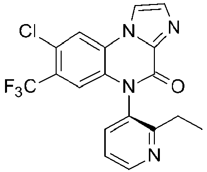

The compound of Example 110 was separated using DAICEL CHIRALPAK AD (250 mm*30 mm, 10 µm); mobile phase: [carbon dioxide-ethanol (0.1% ammonia water)]; B%: 35%, isocratic transfer conditions (retention time: 1.42 min) (ee% = 99.4%) to obtain the title compound as a white solid.

¹H NMR (400 MHz, DMSO-*d*₆, ppm): δ 8.87 - 8.73 (m, 3H), 7.90 - 7.83 (m, 1H), 7.80 - 7.74 (m, 1H), 7.59 - 7.52 (m, 1H), 6.68 - 6.62 (m, 1H), 2.58 - 2.52 (m, 2H), 1.14 - 1.04 (m, 3H).

### Example 112: (Sa)-8-chloro-5-(2-ethylpyridin-3-yl)-7-(trifluoromethyl)imidazo[1,2-a]quinoxalin4(5H)-one

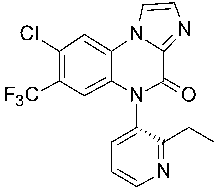

The compound of Example 110 was separated using DAICEL CHIRALPAK AD (250 mm*30 mm, 10 µm); mobile phase: [carbon dioxide-ethanol (0.1% ammonia water)]; B%: 35%, isocratic transfer conditions (retention time: 2.08 min) (ee% = 98.1%) to obtain the title compound as a white solid.

¹H NMR (400 MHz, DMSO-*d*₆, ppm): δ 8.83 - 8.76 (m, 3H), 7.91 - 7.85 (m, 1H), 7.76 (d, *J* = 1.2 Hz, 1H), 7.60 - 7.52 (m, 1H), 6.68 - 6.63 (m, 1H), 2.59 - 2.52 (m, 2H), 1.13 - 1.05 (m, 3H).

### Example 113: 9-methoxy-5-(2-methylpyridin-3-yl)-7-(trifluoromethyl)imidazo[1,2-a]quinoxalin-4(5H)-one

Reaction scheme for preparing the compound of Example 113:

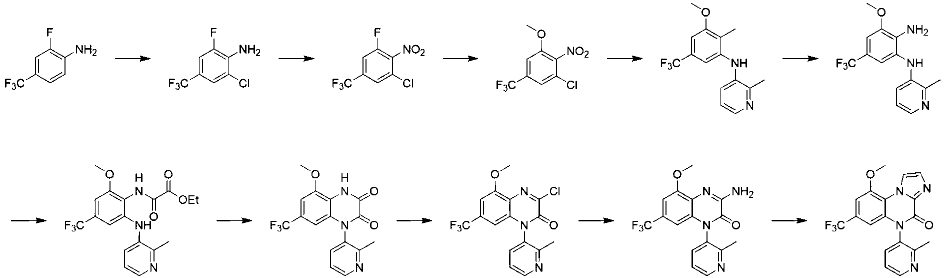

### Example 113-1: 2-chloro-6-fluoro-4-(trifluoromethyl)aniline

2-fluoro-4-(trifluoromethyl)aniline was dissolved in acetonitrile (20 mL), and N-chlorosuccinimide (1.64 g, 12.28 mmol, 1.1 eq.) was added. The reaction mixture was stirred at 85 °C for 3 hours. When all starting materials were consumed and the MS ([M+H]+= 214.2) of the desired compound was observed by LC-MS, the reaction mixture was concentrated under reduced pressure. Petroleum ether (20 mL) was added to the reaction mixture, and after trituration at 25 °C for 30 minutes, the solid was filtered out, and the filtrate was concentrated under reduced pressure to obtain the title compound (2 g, 9.37 mmol, 83.9% yield) in the form of a yellow oil. The next reaction was proceeded directly without an additional purification process.

¹H NMR (400 MHz, CDCl₃, ppm): δ 7.40 - 7.31 (m, 1H), 7.24 - 7.12 (m, 1H), 4.57 - 4.26 (m, 2H).

### Example 113-2: 1-chloro-3-fluoro-2-nitro-5-(trifluoromethyl)benzene

Sodium perborate tetrahydrate (16.21 g, 105.36 mmol, 20.26 mL, 15 eq.) was dissolved in acetic acid (67.5 mL), the temperature was raised to 50°C, and then a solution of the compound obtained in Example 113-1 (1.5 g, 7.02 mmol, 1 eq.) dissolved in acetic acid (30 mL) was slowly added. The reaction mixture was stirred at 50 °C for 24 hours. After confirming that the entire reaction proceeded by TLC, purified water (150 mL) was added, and extraction was performed with ethyl acetate (150 mL X 3). The organic layer was washed with an aqueous Na₂CO₃ solution (pH=7-8), dried over anhydrous sodium sulfate, filtered, and then concentrated under reduced pressure. The concentrate was purified by flash chromatography (stationary phase: silica, ethyl acetate/petroleum ether 0-40%) to obtain the title compound (624 mg, 2.56 mmol, 36.5% yield) in the form of a yellow oil.

¹H NMR (400 MHz, CDCl₃, ppm): δ 7.72 - 7.59 (m, 1H), 7.55 - 7.42 (m, 1H).

### Example 113-3: 1-chloro-3-methoxy-2-nitro-5-(trifluoromethyl)benzene

Lithium bis(trimethylsilyl)amide (1 M, 12.32 mL, 1.5 eq.) was dissolved in tetrahydrofuran (20 mL), and methanol (315.76 mg, 9.85 mmol, 398.78 µL, 1.2 eq.) was added. After the reaction mixture was stirred at 0 °C for 45 minutes, a solution of the compound obtained in Example 113-2 (2 g, 8.21 mmol, 1 eq.) dissolved in tetrahydrofuran (10 mL) was added at 0°C, followed by stirring at 25 °C under nitrogen for 12 hours. After confirming the progress of the reaction by TLC, a saturated aqueous ammonium chloride solution (100mL)was added at 0 °C to terminate the reaction, followed by extraction with ethyl acetate (100 mL X 3). The organic layer was dried over anhydrous sodium sulfate, filtered, and concentrated under reduced pressure to obtain the title compound (2 g, crude mixture) in the form of a yellow oil.

¹H NMR (400 MHz, CDCl₃, ppm): δ 7.39 - 7.34 (m, 1H), 7.21 - 7.16 (m, 1H), 4.02 - 3.95 (m, 3H).

### Example 113-4: N-(3-methoxy-2-methyl-5-(trifluoromethyl)phenyl)-2-methylpyridin-3-amine

The compound obtained in Example 113-3 (1.75 g, 6.85 mmol, 1 eq.), 2-methylpyridin-3-amine (888.56 mg, 8.22 mmol, 1.2 eq.), Pd₂(dba)₃ (313.51 mg, 342.36 µmol, 0.05 eq.), BINAP (285.66 mg, 458.76 µmol, 0.067 eq.), and cesium carbonate (2.23 g, 6.85 mmol, 1 eq.) were dissolved in toluene (20 mL), degassed, purged with nitrogen three times, and stirred at 110 °C for 12 hours. After confirming the progress of the reaction through LC-MS, the reaction mixture was concentrated under reduced pressure. The concentrate was purified by flash chromatography (stationary phase: silica, ethyl acetate/petroleum ether 0-25%) to obtain the title compound (1.90 g, 5.81 mmol, 84.9% yield) in the form of a yellow solid.

¹H NMR (400 MHz, DMSO-*d*₆, ppm): δ 8.36 - 8.31 (m, 1H), 8.28 - 8.23 (m, 1H), 7.55 - 7.48 (m, 1H), 7.29 - 7.21 (m, 1H), 6.98 - 6.93 (m, 1H), 6.40 (s, 1H), 3.99 - 3.89 (m, 3H), 2.32 (s, 3H).

### Example 113-5: 3-methoxy-N¹-(2-methylpyridin-3-yl)-5-(trifluoromethyl)benzen-1,2-diamine

The title compound was obtained using the compound of Example 113-4 as a starting material in the same manner as in Example 75-2.

¹H NMR (400 MHz, DMSO-*d*₆, ppm): δ 7.91 - 7.87 (m, 1H), 7.04 - 6.97 (m, 1H), 6.94 - 6.90 (m, 1H), 6.90 - 6.86 (m, 1H), 6.84 - 6.79 (m, 1H), 6.67 - 6.60 (m, 1H), 5.02 (brs, 2H), 3.87 (s, 3H), 2.44 (s, 3H).

### Example 113-6: ethyl 2-((2-methoxy-6-((2-methylpyridin-3-yl)amino)-4-(trifluoromethyl)phenyl)amino)-2-oxoacetate

The title compound was synthesized using the compound of Example 113-5 as a starting material in the same manner as in Example 48-3.

### Example 113-7: 5-methoxy-1-(2-methylpyridin-3-yl)-7-(trifluoromethyl)-1,4-dihydroquinoxalin-2,3-dione

The compound obtained in Example 113-6 (2.21 g, 5.56 mmol, 1 eq.) was dissolved in dichloromethane (25 mL), and triethylamine (2.25 g, 22.25 mmol, 3.10 mL, 4 eq.) was added. The reaction mixture was stirred at 25 °C for 12 hours. After confirming the completion of the reaction by LC-MS, the reaction mixture was concentrated under reduced pressure. Acetonitrile was added to the concentrate, and solidification was carried out at 25 °C for 30 minutes to obtain the title compound (1.95 g, crude mixture) in the form of a white solid.

¹H NMR (400 MHz, DMSO-*d*₆, ppm): δ 12.05 - 11.49 (m, 1H), 8.72 - 8.62 (m, 1H), 7.90 - 7.75 (m, 1H), 7.59 - 7.43 (m, 1H), 7.32 - 7.16 (m, 1H), 6.01 (s, 1H), 4.00 - 3.97 (m, 3H), 2.24 (s, 3H).

### Example 113-8: 3-chloro-5-methoxy-1-(2-methylpyridin-3-yl)-7-(trifluoromethyl)quinoxalin-2(1H)-one

The compound obtained in Example 113-7 (1.95 g, 5.55 mmol, 1 eq.) was dissolved in phosphoryl chloride (9.75 mL), and after purging with nitrogen three times, the reaction mixture was stirred at 110 °C for 16 hours under nitrogen. After confirming that the entire reaction proceeded by LC-MS, the reaction mixture was concentrated under reduced pressure to obtain the title compound (2.05 g, crude mixture) in the form of a black oil, which was used directly in the next reaction without further purification.

### Example 113-9: 3-amino-5-methoxy-1-(2-methylpyridin-3-yl)-7-(trifluoromethyl)quinoxalin-2(1H)-one

The title compound in the form of a yellow solid was synthesized in the same manner as in Example 33-5 using the compound obtained in Example 113-8 as a starting material.

¹H NMR (400 MHz, CDCl₃, ppm): δ 8.83 - 8.71 (m, 1H), 7.63 - 7.51 (m, 1H), 7.51 - 7.39 (m, 1H), 7.08 - 6.98 (m, 1H), 6.42 - 5.90 (m, 3H), 4.10 - 4.06 (m, 3H), 2.35 - 2.27 (m, 3H).

### Example 113-10: 9-methoxy-5-(2-methylpyridin-3-yl)-7-(trifluoromethyl)imidazo[1,2-a]quinoxalin-4(5H)-one

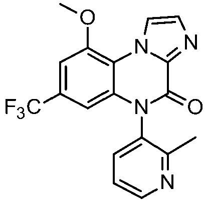

The title compound in the form of a yellow solid was obtained in the same manner as in Example 33-6, using the compound obtained in Example 113-9 as a starting material.

¹H NMR (400 MHz, DMSO-*d*₆, ppm): δ 8.88 - 8.82 (m, 1H), 8.76 - 8.67 (m, 1H), 7.94 - 7.85 (m, 1H), 7.74 - 7.68 (m, 1H), 7.61 - 7.51 (m, 1H), 7.48 - 7.42 (m, 1H), 6.29 - 6.20 (m, 1H), 4.26 - 4.17 (m, 3H), 2.27 - 2.21 (m, 3H).

### Example 115: 9-(difluoromethoxy)-5-(2-methylpyridin-3-yl)-7-(trifluoromethyl)imidazo[1,2-a]quinoxalin-4(5H)-one

Reaction scheme for preparing the compound of Example 115:

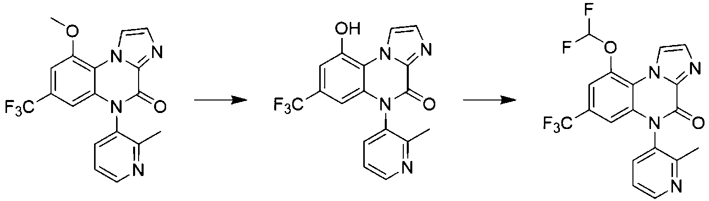

### Example 115-1: 9-hydroxy-5-(2-methylpyridin-3-yl)-7-(trifluoromethyl)imidazo[1,2-a]quinoxalin-4(5H)-one

The title compound of Example 113 (250 mg, 667.88 µmol, 1 eq.) was added to pyridine hydrochloride (3.00 g, 25.96 mmol, 38.87 eq.), and the reaction mixture was stirred at 210 °C for 1 hour. After observing that the reaction had fully proceeded by LC-MS, the reaction mixture was cooled to 25 °C, the pH was adjusted to 5-6 with an aqueous sodium bicarbonate solution, and then extracted with dichloromethane/isopropyl alcohol (100 mL X 6). The organic layer was dried over anhydrous sodium sulfate, filtered, and concentrated under reduced pressure. The concentrate was purified by flash chromatography (stationary phase: silica, petroleum ether/ethyl acetate 0-100%) to obtain the title compound in the form of a yellow solid (480 mg, 1.33 mmol, 97.78% yield).

¹H NMR (400 MHz, DMSO-*d*₆, ppm): δ 12.26 (br s, 1H), 8.95 - 8.86 (m, 1H), 8.78 - 8.62 (m, 1H), 7.91 - 7.85 (m, 1H), 7.70 - 7.66 (m, 1H), 7.58 - 7.51 (m, 1H), 7.22 - 7.17 (m, 1H), 6.09 - 6.03 (m, 1H), 2.34 - 2.17 (m, 3H).

### Example 115-2: 9-(difluoromethoxy)-5-(2-methylpyridin-3-yl)-7-(trifluoromethyl)imidazo[1,2-a]quinoxalin-4(5H)-one

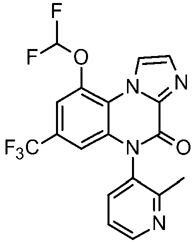

After dissolving the compound obtained in Example 115-1 (120 mg, 333.07 µmol, 1 eq.) in dimethylformamide (2.4 mL), cesium carbonate (217.04 mg, 666.13 µmol, 2 eq.) and sodium chlorodifluoroacetate (126.95 mg, 832.66 µmol, 2.5 eq.) were added. The reaction mixture was stirred at 100 °C for 2 hours. After confirming the completion of the reaction by LC-MS, the reaction mixture was filtered and the filtrate was concentrated under reduced pressure. The concentrate was purified by prep-HPLC (column: Waters Xbridge 150*25mm* 5um; mobile phase: [water (10 mM ammonium bicarbonate)-acetonitrile, B%: 30%-60%, 9 minutes) to obtain the title compound in the form of a white solid (77.05 mg, 185.24 µmol, 51.36% yield, 98.6% purity).

¹H NMR (400 MHz, DMSO-*d*₆, ppm): δ 8.75 - 8.69 (m, 1H), 8.64 - 8.59 (m, 1H), 7.91 - 7.90 (m, 1H), 7.90 - 7.87 (m, 1H), 7.79 - 7.75 (m, 1H), 7.72 - 7.69 (m, 1H), 7.67 - 7.64 (m, 1H), 7.58 - 7.54 (m, 1H), 7.53 - 7.51 (m, 1H), 6.53 - 6.49 (m, 1H), 2.36 - 2.16 (m, 3H).

### Example 117: 5-(2-methylpyridin-3-yl)-9-(2,2,2-trifluoroethoxy)-7-(trifluoromethyl)imidazo[1,2-a]quinoxalin-4(5H)-one

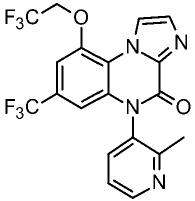

After dissolving the compound obtained in Example 115-1 (120 mg, 333.07 µmol, 1 eq.) in dimethylformamide (2.4 mL), cesium carbonate (217.04 mg, 666.13 µmol, 2 eq.) and TfOCH₂CF₃ (85.04 mg, 366.37 µmol, 50.65 µL, 1.1 eq.) were added. The reaction mixture was stirred at 25 °C for 2 hours. After confirming the completion of the reaction by LC-MS, the reaction mixture was filtered and the filtrate was concentrated under reduced pressure. The concentrate was purified by prep-HPLC (column: Waters Xbridge 150*25mm* 5um; mobile phase: [water (10 mM ammonium bicarbonate)-acetonitrile, B%: 34%-64%, 15 minutes) to obtain the title compound in the form of a white solid (97.6 mg, 219.56 µmol, 60.87% yield, 99.5% purity).

¹H NMR (400 MHz, DMSO-*d*₆, ppm): δ 8.79 - 8.67 (m, 1H), 8.62 - 8.50 (m, 1H), 7.95 - 7.86 (m, 1H), 7.82 - 7.75 (m, 1H), 7.67 - 7.62 (m, 1H), 7.61 - 7.53 (m, 1H), 6.38 - 6.30 (m, 1H), 5.46 - 5.26 (m, 2H), 2.33 - 2.18 (m, 3H).

### Example 118: 9-ethoxy-5-(2-methylpyridin-3-yl)-7-(trifluoromethyl)imidazo[1,2-a]quinoxalin-4(5H)-one

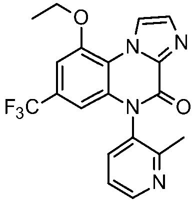

After dissolving the compound obtained in Example 115-1 (120 mg, 333.07 µmol, 1 eq.) in dimethylformamide (2.4 mL), cesium carbonate (217.04 mg, 666.13 µmol, 2 eq.) and bromoethane (97.99 mg, 899.28 µmol, 67.12 µL, 2.7 eq.) were added. The reaction mixture was stirred at 25 °C for 2 hours. After confirming the completion of the reaction by LC-MS, the reaction mixture was filtered and the filtrate was concentrated under reduced pressure. The concentrate was purified by prep-HPLC (column: Waters Xbridge 150*25mm* 5um; mobile phase: [water (10 mM ammonium bicarbonate)-acetonitrile, B%: 30%-60%, 9 minutes) to obtain the title compound in the form of a white solid (79.79 mg, 202.89 µmol, 56.25% yield, 98.7% purity).

¹H NMR (400 MHz, DMSO-d6, ppm): δ 8.85 - 8.78 (m, 1H), 8.75 - 8.67 (m, 1H), 7.93 - 7.84 (m, 1H), 7.74 - 7.69 (m, 1H), 7.59 - 7.50 (m, 1H), 7.47 - 7.39 (m, 1H), 6.26 - 6.19 (m, 1H), 4.56 - 4.41 (m, 2H), 2.31 - 2.19 (m, 3H), 1.62 - 1.52 (m, 3H).

### Experimental Example 1: Evaluation of MAT2A Inhibitory Activity

In vitro assay: Methionine and adenosine triphosphate (ATP) are converted into S-adenosylmethionine (SAM) and inorganic phosphate (pi) via the catalytic action of methionine adenosyltransferase 2A (MAT2A). The produced pi is detected by the PiColorLock^{™} Phosphate Detection Reagent, which is a colorimetric assay, and the activity of MAT2A can be evaluated according to the amount of pi generated during the enzyme reaction. Each compound was evaluated under Test Condition 1 or 2 as described below.

### [Test Condition 1]

Concentration of MAT2A inhibitor: maximum concentration of 50 µM, 5-fold dilution (7 groups in total)
MAT2A concentration: 12 ng/µL
ATP and Met concentration: 100 µM, 80 µM

### [Test Condition 2]

Concentration of MAT2A inhibitor: maximum concentration of 1 µM, 3-fold dilution (7 groups in total)
MAT2A concentration: 4 ng/µL
ATP and Met concentration: 300 µM, 150 µM
Reagents: Methionine (catalog number M5308) and ATP (catalog number A2383) were purchased from Sigma-Aldrich (USA). MAT2A (catalog number 71401) was purchased from BPS Bioscience (USA), and PiColorLock^{™} Phosphate Detection Reagent (catalog number 303-0030) was purchased from Novus (USA).

Assay buffer composition: 50 mM Tris-HCl pH 8.0, 50 mM KCI, 15 mM MgCl₂, 0.3 mM EDTA, 0.005 % (w/v) BSA

Experimental procedure: Each compound was diluted 5-fold or 3-fold based on the maximum concentration (5 mM or 1 mM or 0.1 mM) using DMSO. The diluted compounds were diluted 20-fold using assay buffer, and then mixed at a volume ratio of 1:2 with MAT2A (30 ng/µL or 10 ng/µL) diluted with assay buffer and treated for 30 minutes at room temperature. The negative control group is a group untreated with both the MAT2A enzyme and the compound according to an embodiment of the present application, and the positive control group is a group treated only with the MAT2A enzyme and untreated with the compound according to an embodiment of the present application.

In each well of a 384-well plate, 30 µL of the mixed solution and 20 µL of a 1:1 mixed solution of ATP (0.5 mM or 1.5 mM) and Met (0.4 mM or 0.75 mM) were mixed and then treated for 1 hour at room temperature. After the enzyme reaction, 13 µL of a 100:1 mixed solution of PiColorLockTM and Accelerator was treated in each well and reacted for 5 minutes at room temperature. After treating 5 µL of Stabilizer in each well and reacting for 25 minutes at room temperature, the absorbance at 630 nm was measured using SpectraMax M4 (Molecular Devices, USA) to calculate the IC₅₀ value of the compound.

Analysis method: The enzyme activity (%) of each experimental group was calculated as {(average O.D. of compound-treated group - average O.D. of negative control group)/(average O.D. of positive control group - average O.D. of negative control group)} * 100. After preparing a dose-response curve (4-parameter) using the enzyme activity according to the treatment concentration of each compound in GraphPad Prism 9, the 50 % of enzyme inhibitory activity value (IC₅₀) was calculated through non-linear regression analysis. The IC₅₀ results measured for the MAT2A activity inhibitory ability of the compounds according to an embodiment of the present application are shown in Table 2 (+++: more than 1 to 100 nM, ++: more than 100 to 1,000 nM, +: more than 1,000 to 10,000 nM, -: more than 10,000 nM).

**[Table 2]**

| Example | MAT2A enzyme IC₅₀ |
|---|---|
| 1 | ++ |
| 2 | ++ |
| 3 | ++ |
| 4 | ++ |
| 5 | ++ |
| 6 | ++ |
| 7 | ++ |
| 8 | + |
| 9 | - |
| 10 | +++ |
| 11 | ++ |
| 12 | - |
| 13 | ++ |
| 14 | ++ |
| 15 | + |
| 16 | ++ |
| 17 | ++ |
| 18 | + |
| 19 | + |
| 20 | - |
| 21 | +++ |
| 22 | +++ |
| 23 | +++ |
| 24 | ++ |
| 25 | +++ |
| 26 | +++ |
| 27 | ++ |
| 28 | - |
| 29 | - |
| 30 | - |
| 31 | - |
| 32 | +++ |
| 33 | +++ |
| 34 | +++ |
| 35 | ++ |
| 36 | ++ |
| 37 | ++ |
| 38 | ++ |
| 39 | ++ |
| 40 | ++ |
| 41 | + |
| 42 | - |
| 43 | - |
| 44 | +++ |
| 45 | +++ |
| 46 | ++ |
| 47 | - |
| 48 | ++ |
| 49 | ++ |
| 50 | + |
| 51 | - |
| 52 | ++ |
| 53 | - |
| 54 | +++ |
| 55 | +++ |
| 56 | ++ |
| 57 | ++ |
| 58 | ++ |
| 59 | + |
| 60 | ++ |
| 61 | - |
| 62 | ++ |
| 63 | - |
| 64 | ++ |
| 65 | +++ |
| 71 | + |
| 72 | + |
| 73 | ++ |
| 74 | ++ |
| 75 | ++ |
| 76 | - |
| 77 | +++ |
| 78 | +++ |
| 79 | +++ |
| 80 | - |
| 81 | +++ |
| 82 | - |
| 83 | + |
| 84 | ++ |
| 85 | ++ |
| 86 | ++ |
| 87 | +++ |
| 88 | ++ |
| 89 | ++ |
| 90 | - |
| 91 | +++ |
| 92 | ++ |
| 93 | +++ |
| 94 | - |
| 95 | +++ |
| 96 | +++ |
| 97 | - |
| 98 | +++ |
| 99 | +++ |
| 100 | ++ |
| 101 | +++ |
| 102 | +++ |
| 103 | +++ |
| 104 | - |
| 105 | +++ |
| 106 | - |
| 107 | +++ |
| 108 | +++ |
| 109 | +++ |
| 110 | +++ |
| 111 | - |
| 112 | +++ |
| 113 | +++ |
| 115 | ++ |
| 117 | +++ |
| 118 | +++ |

As shown in Table 2, the compounds according to an embodiment of the present application had high inhibitory ability against MAT2A activity.

### Experimental Example 2: Evaluation of cell proliferation inhibitory ability in MTAP-deficient cancer cells induced by MAT2A inhibition

The HCT116 (MTAP del) cell line, which is a colorectal cancer cell line with deletion of MTAP gene, was treated with a compound according to an embodiment of the present application as a MAT2A inhibitor, and after culturing for 120 hours, the change in the number of cells was measured with a WST-1 assay reagent. The WST-1 assay is an assay that measures the number of cells by measuring the generation of a chromogenic substance called formazan from tetrazolium salts by intracellular mitochondrial dehydrogenase. In addition, as a control group, a wild-type colorectal cancer HCT116 (MTAP WT) cell line containing the MTAP gene was used to perform the experiment in the same manner as the experimental group.

Cell lines and reagents: HCT116 (MTAP WT (catalog number HD PAR-034), MTAP del (catalog number HD R02-033)) cell lines were purchased from Horizon Discovery (USA), and RPMI1640 medium (catalog number 22400-093), FBS (Fetal Bovine serum, catalog number 16000044), and Penicillin/Streptomycin (catalog number 15140-122) were purchased from GIBCO (USA). WST-1 (catalog number EZ-BULK150) was purchased from Dogen (Korea).

Experimental procedure: HCT116 (MTAP WT, MTAP del) cell lines were dispensed into a 96-well plate at a concentration of 1,000 cells/well in RPMI1640 culture medium containing 10% FBS and 1% Penicillin/Streptomycin, and allowed to attach for 24 hours. When the cells were attached to the plate, the drug was diluted 4-fold from a maximum concentration of 10 µM and treated on the cells. After 120 hours of drug treatment, 20 µL of WST-1 solution was treated for 2 hours, and absorbance at 450 nm was measured using SpectraMax M4 (Molecular Devices). Data analysis was calculated by taking the DMSO control group value as 100%. For the IC50 value, the concentration at which cell proliferation is inhibited by 50% was calculated through interpolation using a 4-parameter fit of the dose-response curve using the GraphPad Prism 8.4.3 program. The cancer cell proliferation inhibitory ability and selectivity in MTAP-deficient cancer cells of the compounds according to an embodiment of the present application are described in Table 3 (+++: 1 to 100 nM, ++: more than 100 to 1,000 nM, +: more than 1 to 10 µM, -: more than 10 µM).

**[Table 3]**

| Example | HCT116 MTAP^{-/-} | HCT116 WT |
|---|---|---|
| 1 | ++ | - |
| 2 | + | - |
| 3 | ++ | - |
| 4 | ++ | - |
| 5 | + | - |
| 6 | ++ | + |
| 7 | + | - |
| 8 | - | - |
| 9 | - | - |
| 10 | +++ | ++ |
| 11 | +++ | ++ |
| 13 | ++ | - |
| 14 | ++ | - |
| 15 | ++ | - |
| 16 | ++ | - |
| 17 | ++ | - |
| 18 | + | - |
| 19 | + | - |
| 20 | - | - |
| 21 | +++ | - |
| 22 | +++ | - |
| 23 | ++ | - |
| 24 | - | - |
| 25 | +++ | ++ |
| 26 | +++ | - |
| 27 | ++ | - |
| 28 | - | - |
| 30 | - | - |
| 32 | +++ | + |
| 33 | +++ | ++ |
| 34 | +++ | ++ |
| 35 | ++ | - |
| 36 | ++ | - |
| 37 | ++ | - |
| 38 | +++ | - |
| 39 | ++ | - |
| 40 | ++ | - |
| 45 | +++ | ++ |
| 46 | ++ | - |
| 49 | ++ | - |
| 52 | + | - |
| 54 | +++ | ++ |
| 55 | +++ | + |
| 56 | +++ | + |
| 57 | +++ | ++ |
| 58 | +++ | ++ |
| 60 | - | - |
| 65 | +++ | + |
| 71 | - | - |
| 73 | ++ | - |
| 74 | ++ | - |
| 75 | ++ | - |
| 77 | +++ | + |
| 78 | +++ | - |
| 79 | +++ | + |
| 81 | +++ | + |
| 84 | +++ | - |
| 85 | +++ | - |
| 86 | ++ | - |
| 87 | +++ | - |
| 88 | ++ | - |
| 89 | ++ | - |
| 91 | +++ | - |
| 92 | - | - |
| 93 | +++ | - |
| 95 | +++ | - |
| 96 | +++ | - |
| 98 | +++ | - |
| 99 | +++ | - |
| 100 | ++ | - |
| 101 | +++ | - |
| 102 | +++ | - |
| 103 | +++ | - |
| 105 | +++ | - |
| 107 | - | - |
| 108 | ++ | + |
| 109 | ++ | - |
| 110 | - | - |
| 112 | - | - |
| 113 | +++ | - |

As shown in Table 3, it was confirmed that the compounds according to an embodiment of the present application exhibited high cell proliferation inhibitory ability and selectivity against MTAP-deficient cancer cells associated with MAT2A. That is, the compounds according to an embodiment of the present application have superior cell proliferation inhibitory ability against the HCT116 (MTAP del) cell line, which is a colorectal cancer cell line with the MTAP gene deletion, compared to the wild-type colorectal cancer HCT116 (MTAP WT) cell line containing the MTAP gene, and thus exhibited excellent selectivity for effectively inhibiting cancer with the MTAP gene deletion. Therefore, the compound according to an embodiment of the present application can act more effectively in patient populations with MTAP-deficient cancers.

Through the above results, it was shown that the compound according to an embodiment of the present application can effectively inhibit the growth of MTAP-deficient cancer cells by suppressing MAT2A activity, and can be usefully used as a pharmaceutical composition for the prevention or treatment of cancer.

## Claims

1. A compound of chemical formula 1, an optical isomer, a stereoisomer, a racemate, an isotopologue, a solvate, a hydrate, or a pharmaceutically acceptable salt thereof: 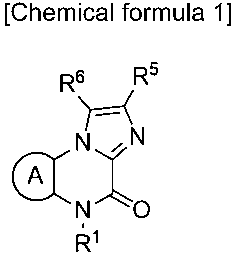 in chemical formula 1,
A is 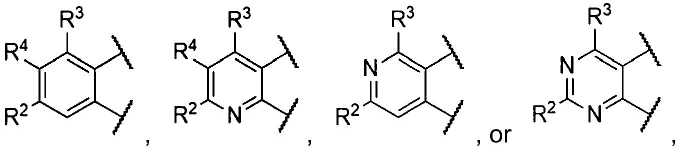
R² is (C₁₋₆)alkyl, (C₂₋₆)alkenyl, (C₂₋₆)alkynyl, (C₁₋₆)alkoxy, (C₁₋₆)alkylsulfonyl, halo, (C₁₋₆)haloalkyl, (C₁₋₆)haloalkoxy, (C₃₋₁₀)cycloalkyl, cyano, nitro, amino, acetyl, (C₁₋₆)alkylamino, (C₁₋₆)alkylcarbonyl, (C₁₋₆)alkoxycarbonyl, aminocarbonyl, (C₁₋₆)alkylaminocarbonyl, (C₁₋₆)hydroxyalkyl, (C₁₋₆)hydroxyalkoxy, (C₁₋₆)hydroxyalkylamino, (C₁₋₆)alkoxy-(C₁₋₆)alkyl, (C₁₋₆)alkoxy-(C₁₋₆)alkoxy, (C₁₋₆)alkoxy-(C₁₋₆)alkylamino, (C₁₋₆)aminoalkyl, (C₁₋₆)aminoalkoxy, (C₁₋₆)aminoalkylamino, 3 to 10-membered heteroaryl containing one or more heteroatoms selected from N, O, and S, 3 to 10-membered heteroaryloxy containing one or more heteroatoms selected from N, O, and S, 3 to 10-membered heteroarylamino containing one or more heteroatoms selected from N, O, and S, 3 to 10-membered heterocyclyl containing one or more heteroatoms selected from N, O, and S, 3 to 10-membered heterocyclyloxy containing one or more heteroatoms selected from N, O, and S, 3 to 10-membered heterocyclylamino containing one or more heteroatoms selected from N, O, and S, 3 to 10-membered heterocyclyloxyalkoxy containing one or more heteroatoms selected from N, O, and S, or 3 to 10-membered heterocyclyloxyalkylamino containing one or more heteroatoms selected from N, O, and S, wherein the heterocyclyl or the heteroaryl is unsubstituted or substituted with one or more from R^{a}, R^{b}, and R^{c}, whrein R^{a}, R^{b}, and R^{c} are independently selected from (C₁₋₆)alkyl, (C₃₋₁₀)cycloalkyl, (C₁₋₆)haloalkyl, (C₁₋₆)haloalkoxy, (C₁₋₆)alkoxy, hydroxy, halo, cyano, (C₁₋₆)hydroxyalkyl, (C₁₋₆)alkoxy-(C₁₋₆)alkyl, and (C₁₋₆)aminoalkyl;
R³ is hydrogen, (C₁₋₆)alkyl, (C₂₋₆)alkenyl, (C₂₋₆)alkynyl, (C₁₋₆)alkoxy, halo, halo(C₁₋₆)alkyl-(C₁₋₆)alkoxy, (C₁₋₆)alkylsulfonyl, (C₁₋₆)haloalkyl, (C₁₋₆)haloalkoxy, (C₃₋₁₀)cycloalkyl, (C₁₋₆)alkyloxy, cyano, amino, (C₁₋₆)alkylamino, aminocarbonyl-(C₁₋₆)alkylaminocarbonyl, (C₁₋₆)hydroxyalkyl, (C₁₋₆)hydroxyalkoxy, (C₁₋₆)hydroxyalkylamino, (C₂₋₆)alkoxyalkyl, (C₁₋₆)alkoxy-(C₁₋₆)alkoxy, (C₁₋₆)alkoxy-(C₁₋₆)alkylamino, (C₁₋₆)aminoalkyl, (C₁₋₆)aminoalkoxy, (C₁₋₆)aminoalkylamino, 3 to 10-membered heteroaryl containing one or more heteroatoms selected from N, O, and S, 3 to 10-membered heteroaryloxy containing one or more heteroatoms selected from N, O, and S, 3 to 10-membered heteroarylamino containing one or more heteroatoms selected from N, O, and S, 3 to 10-membered heterocyclyl containing one or more heteroatoms selected from N, O, and S, 3 to 10-membered heterocyclyloxy containing one or more heteroatoms selected from N, O, and S, 3 to 10-membered heterocyclylamino containing one or more heteroatoms selected from N, O, and S, 3 to 10-membered heterocyclyl-(C₁₋₆)alkyloxy containing one or more heteroatoms selected from N, O, and S, 3 to 10-membered heterocyclyloxy-(C₁₋₆)alkoxy containing one or more heteroatoms selected from N, O, and S, or 3 to 10-membered heterocyclyloxy-(C₁₋₆)alkylamino containing one or more heteroatoms selected from N, O, and S, wherein the alkoxy, the heterocyclyl or the heteroaryl is unsubstituted or substituted with one or more from R^{a}, R^{b}, and R^{c}, wherein R^{a}, R^{b}, and R^{c} are independently selected from (C₁₋₆)alkyl, (C₃₋₁₀)cycloalkyl, (C₁₋₆)haloalkyl, (C₁₋₆)haloalkoxy, (C₁₋₆)alkoxy, hydroxy, halo, cyano, (C₁₋₆)alkoxycarbonyl, (C₁₋₆)hydroxyalkyl, (C₁₋₆)alkoxy-(C₁₋₆)alkyl, and (C₁₋₆)aminoalkyl;
R⁴ is hydrogen, (C₁₋₆)alkyl, (C₁₋₆)alkenyl, (C₁₋₆)alkynyl, (C₁₋₆)alkoxy, (C₁₋₆)alkyl-(C₁₋₆)alkoxy, (C₁₋₆)alkylthiol, (C₁₋₆)alkylsulfonyl, halo, (C₁₋₆)haloalkyl, (C₁₋₆)haloalkoxy, (C₃₋₁₀)cycloalkyl, cyano, amino, (C₁₋₆)alkylamino, aminocarbonyl, or (C₁₋₆)alkylaminocarbonyl;
R¹ is 5 to 20-membered mono or bicyclic aryl or 3 to 10-membered mono or bicyclic heteroaryl containing one or more heteroatoms selected from N, O, and S, wherein the aryl or the heteroaryl is unsubstituted or substituted with one or more substituent of R^{d};
R^{d} is halo, oxo, cyano, hydroxy, (C₁₋₆)alkyl, (C₁₋₆)alkoxy, (C₃₋₁₀)cycloalkyl, (C₃₋₁₀)cycloalkyl-(C₁₋₆)alkyl, (C₁₋₆)haloalkyl, (C₁₋₆)haloalkoxy, (C₁-C₆)hydroxyalkyl, (C₁-C₆)alkoxy-(C₁-C₆)alkyl, (C₁-C₆)alkylamino, di(C₁-C₆)alkylamino, (C₁-C₆)alkylsulfonamide, 3 to 10-membered heteroaryl containing one or more heteroatoms selected from N, O, and S, 3- to 10-membered heterocycloalkyl containing one or more heteroatoms selected from N, O, and S, or phenyl;
R⁵ and R⁶ are independently hydrogen, halo, hydroxy, acetyl, (C₁₋₆)alkyl, (C₁₋₆)haloalkyl, (C₁₋₆)hydroxyalkyl, (C₁₋₆)alkoxy(C₁₋₆)alkyl, -(C₁₋₆)alkyl-amino, aminocarbonyl, (C₁₋₆)alkylaminocarbonyl, di(C₁₋₆)alkylaminocarbonyl, aminocarbonyl(C₁₋₆)alkyl, aminosulfonyl(C₁₋₆)alkyl, (C₁₋₆)alkoxy, -amino(C₁₋₆)-alkyl, -amino-di(C₁₋₆)alkyl, (C₁₋₆)alkylcarbonyl, or -(C₁₋₆)alkyl-R^{e}, and
the R^{e} is halo, (C₁₋₆)alkoxy, -amino-(C₁₋₆)alkyl-(C₁₋₆)alkoxy, -amino-(C₁₋₆)alkyl-hydroxy, (C₁₋₆)haloalkoxy, (C₁₋₆)alkoxy-(C₁₋₆)alkoxy, (C₁₋₆)hydroxyalkoxy, (C₁₋₆)alkylamino, or 3- to 10-membered mono or bicyclic heterocycle containing one or more heteroatoms selected from N, O, and S, wherein the R^{e} is unsubstituted or substituted with (C₁₋₆)alkyl or hydroxy.

2. The compound, an optical isomer, a stereoisomer, a racemate, an isotopologue, a solvate, a hydrate, or a pharmaceutically acceptable salt thereof according to claim 1,
wherein the R¹ is 5- to 20-membered mono- or bicyclic aryl, or 3- to 10-membered mono- or bicyclic heteroaryl containing one or more heteroatoms selected from N, O, and S, and the R¹ is substituted with one or more substituent of R^{d}.

3. The compound, an optical isomer, a stereoisomer, a racemate, an isotopologue, a solvate, a hydrate, or a pharmaceutically acceptable salt thereof according to claim 1,
wherein the R² is (C₁₋₆)alkyl, (C₁₋₆)alkoxy, halo, (C₁₋₆)haloalkyl, (C₁₋₆)haloalkoxy, (C₃₋₁₀)cycloalkyl,cyano, acetyl, or (C₁₋₆)alkylcarbonyl;
wherein the R³ is hydrogen, (C₁₋₆)alkyl, (C₁₋₆)alkoxy, halo, (C₁₋₆)haloalkoxy, halo(C₁₋₆)alkyl(C₁₋₆)alkoxy, halo(C₁₋₆)alkyl, (C₁₋₆)alkylamino, or pyrrolidine;
wherein the R⁴ is hydrogen, (C₁₋₆)alkyl, (C₁₋₆)alkoxy, halo, or (C₁₋₆)haloalkyl;
wherein the R¹ is unsubstituted or substituted with 1 to 3 substituents of R^{d};
wherein the R^{d} is halo, oxo, cyano, hydroxy, (C₁₋₆)alkyl, (C₁₋₆)alkoxy, (C₃₋₁₀)cycloalkyl, (C₁₋₆)haloalkyl, (C₁₋₆)haloalkoxy, or (C₁-C₆)alkylsulfonamide, and
wherein the R⁵ and R⁶ are independently hydrogen, halo, hydroxy, acetyl, (C₁₋₆)alkyl, (C₁₋₆)haloalkyl, (C₁₋₆)hydroxyalkyl, (C₁₋₆)alkoxy(C₁₋₆)alkyl, amino(C₁₋₆)alkyl, di(C₁₋₆)alkylaminocarbonyl, (C₁₋₆)alkoxy, (C₁₋₆)alkylamino, di(C₁₋₆)alkylamino, (C₁₋₆)alkylamino(C₁₋₆)alkyl, (C₁₋₆)alkylamino(C₁₋₆)alkylhydroxy, (C₁₋₆)alkylamino(C₁₋₆)alkyl(C₁₋₆)alkoxy, or (C₁₋₆)alkylcarbonyl.

4. The compound, an optical isomer, a stereoisomer, a racemate, an isotopologue, a solvate, a hydrate, or a pharmaceutically acceptable salt thereof according to claim 1,
wherein the R² is (C₁₋₆)alkyl, (C₁₋₆)alkoxy, halo, (C₁₋₆)haloalkyl, (C₁₋₆)haloalkoxy, (C₃₋₁₀)cycloalkyl, cyano, acetyl, or (C₁₋₆)alkylcarbonyl;
wherein the R³ is hydrogen, (C₁₋₆)alkyl, (C₁₋₆)alkoxy, halo, (C₁₋₆)haloalkoxy, halo(C₁₋₆)alkyl(C₁₋₆)alkoxy, halo(C₁₋₆)alkyl, (C₁₋₆)alkylamino, or pyrrolidine;
wherein the R⁴ is hydrogen, (C₁₋₆)alkyl, (C₁₋₆)alkoxy, halo, or (C₁₋₆)haloalkyl;
wherein the R¹ is substituted with 1 to 3 substituents of R^{d};
wherein the R^{d} is halo, oxo, cyano, hydroxy, (C₁₋₆)alkyl, (C₁₋₆)alkoxy, (C₃₋₁₀)cycloalkyl, (C₁₋₆)haloalkyl, (C₁₋₆)haloalkoxy, or (C₁-C₆)alkylsulfonamide; and
wherein the R⁵ and R⁶ are independently hydrogen, halo, hydroxy, acetyl, (C₁₋₆)alkyl, (C₁₋₆)haloalkyl, (C₁₋₆)hydroxyalkyl, (C₁₋₆)alkoxy(C₁₋₆)alkyl, amino(C₁₋₆)alkyl, di(C₁₋₆)alkylaminocarbonyl, (C₁₋₆)alkoxy, (C₁₋₆)alkylamino, di(C₁₋₆)alkylamino, (C₁₋₆)alkylamino(C₁₋₆)alkyl, (C₁₋₆)alkylamino(C₁₋₆)alkylhydroxy, (C₁₋₆)alkylamino(C₁₋₆)alkyl(C₁₋₆)alkoxy, or (C₁₋₆)alkylcarbonyl.

5. The compound, an optical isomer, a stereoisomer, a racemate, an isotopologue, a solvate, a hydrate, or a pharmaceutically acceptable salt thereof according to claim 1,
wherein the R¹ is phenyl, pyrazine, pyrazole, pyridine, indole, dihydrobenzofuran, or benzothiazole, and the R¹ is unsubstituted or substituted with halo, cyano, hydroxy, (C₁₋₆)alkyl, (C₁₋₆)alkoxy, (C₃₋₁₀)cycloalkyl, (C₁₋₆)haloalkyl, (C₁₋₆)haloalkoxy, or (C₁-C₆)alkylsulfonamide.

6. The compound, an optical isomer, a stereoisomer, a racemate, an isotopologue, a solvate, a hydrate, or a pharmaceutically acceptable salt thereof according to claim 1,
wherein the R¹ is phenyl, pyrazine, pyrazole, pyridine, indole, dihydrobenzofuran, or benzothiazole, and the R¹ is substituted with halo, cyano, hydroxy, (C₁₋₆)alkyl, (C₁₋₆)alkoxy, (C₃₋₁₀)cycloalkyl, (C₁₋₆)haloalkyl, (C₁₋₆)haloalkoxy, or (C₁-C₆)alkylsulfonamide.

7. The compound, an optical isomer, a stereoisomer, a racemate, an isotopologue, a solvate, a hydrate, or a pharmaceutically acceptable salt thereof according to claim 1,
wherein the R² is (C₁₋₃)alkyl, (C₁₋₃)alkoxy, halo, (C₃₋₆)cycloalkyl, cyano, acetyl, (C₁₋₃)dihaloalkyl, (C₁₋₃)trihaloalkyl, or (C₁₋₃)trihaloalkoxy.

8. The compound, an optical isomer, a stereoisomer, a racemate, an isotopologue, a solvate, a hydrate, or a pharmaceutically acceptable salt thereof according to claim 1,
wherein the A is 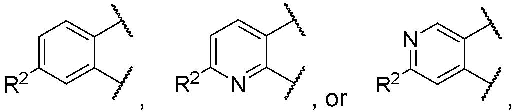 and
wherein the R² is (C₁₋₆)alkyl, (C₁₋₆)alkoxy, halo, (C₁₋₆)haloalkyl, (C₁₋₆)haloalkoxy, (C₃₋₁₀)cycloalkyl,cyano, acetyl, or (C₁₋₆)alkylcarbonyl.

9. The compound, an optical isomer, a stereoisomer, a racemate, an isotopologue, a solvate, a hydrate, or a pharmaceutically acceptable salt thereof according to claim 8,
wherein the R² is fluoro, chloro, bromo, iodo, methyl, isopropyl, acetyl, cyano, nitro, cyclopropyl, difluoromethyl, trifluoromethyl, methoxy, ethoxy, trifluoromethoxy, or trifluoroethoxy.

10. The compound, an optical isomer, a stereoisomer, a racemate, an isotopologue, a solvate, a hydrate, or a pharmaceutically acceptable salt thereof according to claim 1,
wherein the A is 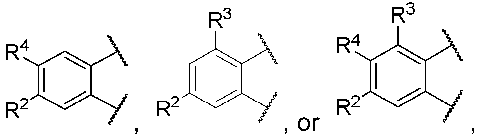
wherein the R² is (C₁₋₆)alkyl, (C₁₋₆)alkoxy, halo, (C₁₋₆)haloalkyl, (C₁₋₆)haloalkoxy, (C₃₋₁₀)cycloalkyl,cyano, acetyl, or (C₁₋₆)alkylcarbonyl,
wherein the R³ is (C₁₋₆)alkyl, (C₁₋₆)alkoxy, halo, (C₁₋₆)haloalkoxy, halo(C₁₋₆)alkyl(C₁₋₆)alkoxy, halo(C₁₋₆)alkyl, (C₁₋₆)alkylamino, or pyrrolidine, and
wherein the R⁴ is (C₁₋₆)alkyl, (C₁₋₆)alkoxy, halo, or (C₁₋₆)haloalkyl.

11. The compound, an optical isomer, a stereoisomer, a racemate, an isotopologue, a solvate, a hydrate, or a pharmaceutically acceptable salt thereof according to claim 10,
wherein the A is one selected from the group consisting of: 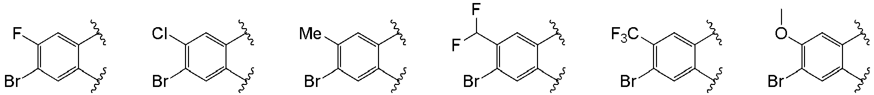 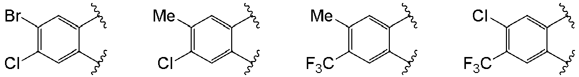 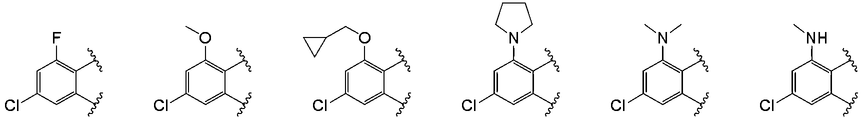 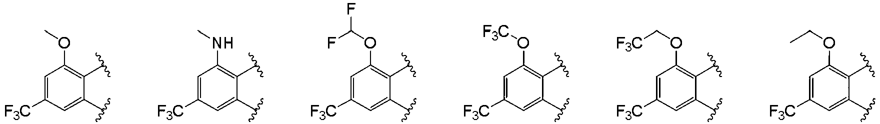 and

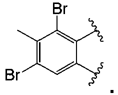

12. The compound, an optical isomer, a stereoisomer, a racemate, an isotopologue, a solvate, a hydrate, or a pharmaceutically acceptable salt thereof according to claim 11,
wherein the A is one selected from the group consisting of: 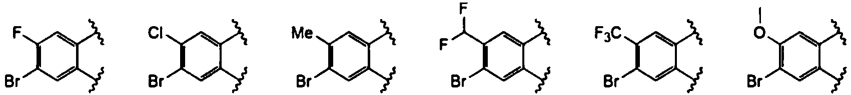 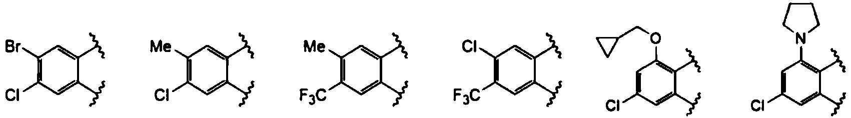 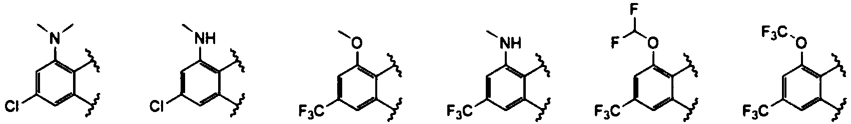 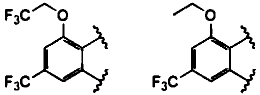 and

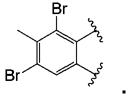

13. The compound, an optical isomer, a stereoisomer, a racemate, an isotopologue, a solvate, a hydrate, or a pharmaceutically acceptable salt thereof according to claim 1,
wherein the R⁵ is hydrogen, halo, (C₁₋₆)alkyl, (C₁₋₆)alkoxy, (C₁-₆)alkyl-amino-, di(C₁₋₆)alkyl-amino-, (C₁₋₆)haloalkyl, acetyl, or -CH₂R^{e}, and
wherein the R^{e} is halo, (C₁₋₆)alkoxy, -amino-(C₁₋₆)alkyl-(C₁₋₆)alkoxy, -amino-(C₁₋₆)alkyl-hydroxy, (C₁₋₆)haloalkoxy, (C₁₋₆)alkoxy-(C₁₋₆)alkoxy, (C₁₋₆)hydroxyalkoxy, (C₁₋₆)alkyl-amino-, or 3- to 10-membered mono- or bicyclic heterocycle containing one or more heteroatoms selected from N, O, and S, and the R^{e} is unsubstituted or substituted with (C₁₋₆)alkyl or hydroxy.

14. The compound, an optical isomer, a stereoisomer, a racemate, an isotopologue, a solvate, a hydrate, or a pharmaceutically acceptable salt thereof according to claim 13,
wherein the R^{e} is halo, (C₁₋₆)alkoxy, -amino-(C₁₋₆)alkyl-(C₁₋₆)alkoxy, -amino-(C₁₋₆)alkyl-hydroxy, (C₁₋₆)haloalkoxy, (C₁₋₆)alkoxy-(C₁₋₆)alkoxy, (C₁₋₆)hydroxyalkoxy, (C₁₋₆)alkyl-amino-, azetidine, morpholine, piperazine, or heterospirocarbocyclyl, and the R^{e} is unsubstituted or substituted with (C₁₋₆)alkyl or hydroxy.

15. The compound, an optical isomer, a stereoisomer, a racemate, an isotopologue, a solvate, a hydrate, or a pharmaceutically acceptable salt thereof according to claim 13,
wherein the R^{e} is methoxy, methylamine, hydroxyethylamine, methoxyethylamine, azetidine, 3-hydroxyazetidine, methylpiperazine, morpholine, or 2-oxa-7-azaspiro[3.5]nonane.

16. The compound, an optical isomer, a stereoisomer, a racemate, an isotopologue, a solvate, a hydrate, or a pharmaceutically acceptable salt thereof according to claim 1,
wherein the R⁶ is hydrogen, halo, (C₁₋₆)alkyl, (C₁₋₆)haloalkyl, acetyl, or hydroxy.

17. The compound, an optical isomer, a stereoisomer, a racemate, an isotopologue, a solvate, a hydrate, or a pharmaceutically acceptable salt thereof according to claim 1,
wherein the compound of chemical formula 1 is selected from (1) to (120):
1) 7-chloro-5-(o-tolyl)imidazo[1,2-a]quinoxalin-4(5H)-one
2) 7-chloro-5-phenylimidazo[1,2-a]quinoxalin-4(5H)-one
3) 7-chloro-5-(2-ethylphenyl)imidazo[1,2-a]quinoxalin-4(5H)-one
4) 7-chloro-5-(2,3-dimethylphenyl)imidazo[1,2-a]quinoxalin-4(5H)-one
5) 7-chloro-5-(2-isopropylphenyl)imidazo[1,2-a]quinoxalin-4(5H)-one
6) 7-chloro-5-(3-fluoro-2-methylphenyl)imidazo[1,2-a]quinoxalin-4(5H)-one
7) 7-chloro-5-(2-(trifluoromethyl)phenyl)imidazo[1,2-a]quinoxalin-4(5H)-one
8) 7-chloro-5-(4-fluoro-2-methylphenyl)imidazo[1,2-a]quinoxalin-4(5H)-one
9) 7-chloro-5-(5-fluoro-2-methylphenyl)imidazo[1,2-a]quinoxalin-4(5H)-one
10) 5-(3-fluoro-2-methylphenyl)-7-(trifluoromethyl)imidazo[1,2-a]quinoxalin-4(5H)-one
11) 7-chloro-5-(3-chloro-2-methylphenyl)imidazo[1,2-a]quinoxalin-4(5H)-one
12) 7-chloro-5-(2-fluorophenyl)imidazo[1,2-a]quinoxalin-4(5H)-one
13) 7-cyclopropyl-5-(3-fluoro-2-methylphenyl)imidazo[1,2-a]quinoxalin-4(5H)-one
14) 5-(3-fluoro-2-methylphenyl)-7-methoxyimidazo[1,2-a]quinoxalin-4(5H)-one
15) 5-(3-fluoro-2-methylphenyl)-7-(trifluoromethoxy)imidazo[1,2-a]quinoxalin-4(5H)-one
16) 7-cyclopropyl-5-(o-tolyl)imidazo[1,2-a]quinoxalin-4(5H)-one
17) 7-methoxy-5-(o-tolyl)imidazo[1,2-a]quinoxalin-4(5H)-one
18) 4-oxo-5-(o-tolyl)-4,5-dihydroimidazo[1,2-a]quinoxalin-7-carbonitrile
19) 5-(o-tolyl)-7-(trifluoromethoxy)imidazo[1,2-a]quinoxalin-4(5H)-one
20) (*R*ₐ)-7-chloro-5-(o-tolyl)imidazo[1,2-a]quinoxalin-4(5H)-one
21) (*S*ₐ)-7-chloro-5-(o-tolyl)imidazo[1,2-a]quinoxalin-4(5H)-one
22) 7-(difluoromethyl)-5-(o-tolyl)imidazo[1,2-a]quinoxalin-4(5H)-one
23) 7-chloro-5-(2-chlorophenyl)imidazo[1,2-a]quinoxalin-4(5H)-one
24) 7-acetyl-5-(o-tolyl)imidazo[1,2-a]quinoxalin-4(5H)-one
25) 7-lodo-5-(o-tolyl)imidazo[1,2-a]quinoxalin-4(5H)-one
26) 5-(o-tolyl)-7-(trifluoromethyl)imidazo[1,2-a]quinoxalin-4(5H)-one
27) 5-(3-fluoro-2-methylphenyl)-4-oxo-4,5-dihydroimidazo[1,2-a]quinoxalin-7-carbonitrile
28) 5-(o-tolyl)-7-(2,2,2-trifluoroethoxy)imidazo[1,2-a]quinoxalin-4(5H)-one
29) 5-(3-fluoro-2-methylphenyl)-7-(2,2,2-trifluoroethoxy)imidazo[1,2-a]quinoxalin-4(5H)-one
30) 7-ethoxy-5-(o-tolyl)imidazo[1,2-a]quinoxalin-4(5H)-one
31) 7-ethoxy-5-(3-fluoro-2-methylphenyl)imidazo[1,2-a]quinoxalin-4(5H)-one
32) 7-bromo-5-(o-tolyl)imidazo[1,2-a]quinoxalin-4(5H)-one
33) 7-chloro-5-(2-methylpyridin-3-yl)imidazo[1,2-a]quinoxalin-4(5H)-one
34) 7-chloro-1-methyl-5-(2-methylpyridin-3-yl)imidazo[1,2-a]quinoxalin-4(5H)-one
35) 7-chloro-5-(o-tolyl)imidazo[1,2-a]pyrido[2,3-e]pyrazin-4(5H)-one
36) 7-methyl-5-(o-tolyl)imidazo[1,2-a]pyrido[2,3-e]pyrazin-4(5H)-one
37) 7-chloro-5-(2-methoxyphenyl)imidazo[1,2-a]quinoxalin-4(5H)-one
38) 5-(2-bromophenyl)-7-chloroimidazo[1,2-a]quinoxalin-4(5H)-one
39) 7-chloro-5-(m-tolyl)imidazo[1,2-a]quinoxalin-4(5H)-one
40) 7-chloro-5-(3-fluorophenyl)imidazo[1,2-a]quinoxalin-4(5H)-one
41) 7-fluoro-5-(o-tolyl)imidazo[1,2-a]quinoxalin-4(5H)-one
42) 3-chloro-5-(o-tolyl)imidazo[1,2-a]pyrido[4,3-e]pyrazin-6(5H)-one
43) 2-(7-chloro-4-oxoimidazo[1,2-a]quinoxalin-5(4H)-yl)benzonitrile
44) N-(3-(7-chloro-4-oxoimidazo[1,2-a]quinoxalin-5(4H)-yl)phenyl)methanesulfonamide
45) 7-chloro-2-methyl-5-(2-methylpyridin-3-yl)imidazo[1,2-a]quinoxalin-4(5H)-one
46) 7-(difluoromethyl)-5-(o-tolyl)imidazo[1,2-a]pyrido[2,3-e]pyrazin-4(5H)-one
47) 7-isopropyl-5-(o-tolyl)imidazo[1,2-a]pyrido[2,3-e]pyrazin-4(5H)-one
48) 7-chloro-5-(3-methylpyrazin-2-yl)imidazo[1,2-a]quinoxalin-4(5H)-one
49) 5-(o-tolyl)-7-(trifluoromethyl)imidazo[1,2-a]pyrido[2,3-e]pyrazin-4(5H)-one
50) 7-chloro-5-(2-(trifluoromethoxy)phenyl)imidazo[1,2-a]quinoxalin-4(5H)-one
51) 7-chloro-5-(2,6-dimethylphenyl)imidazo[1,2-a]quinoxalin-4(5H)-one
52) 7-chloro-5-(2-fluoro-6-methylphenyl)imidazo[1,2-a]quinoxalin-4(5H)-one
53) 7-chloro-5-(3-methylpyridin-2-yl)imidazo[1,2-a]quinoxalin-4(5H)-one
54) 7-chloro-5-(2-chloropyridin-3-yl)imidazo[1,2-a]quinoxalin-4(5H)-one
55) 2-methyl-5-(2-methylpyridin-3-yl)-7-(trifluoromethyl)imidazo[1,2-a]quinoxalin-4(5H)-one
56) 5-(2-methylpyridin-3-yl)-7-(trifluoromethyl)imidazo[1,2-a]quinoxalin-4(5H)-one
57) 1-bromo-5-(2-methylpyridin-3-yl)-7-(trifluoromethyl)imidazo[1,2-a]quinoxalin-4(5H)-one
58) 1-methyl-5-(2-methylpyridin-3-yl)-7-(trifluoromethyl)imidazo[1,2-a]quinoxalin-4(5H)-one
59) 1-acetyl-5-(2-methylpyridin-3-yl)-7-(trifluoromethyl)imidazo[1,2-a]quinoxalin-4(5H)-one
60) 2-acetyl-1-hydroxy-5-(2-methylpyridin-3-yl)-7-(trifluoromethyl)imidazo[1,2-a]quinoxalin-4(5H)-one
61) 7-chloro-2-((4-methylpiperazin-1-yl)methyl)-5-(2-methylpyridin-3-yl)imidazo[1,2-a]quinoxalin-4(5H)-one
62) 7-chloro-5-(2-methylpyridin-3-yl)-2-(morpholinomethyl)imidazo[1,2-a]quinoxalin-4(5H)-one
63) 2-((2-oxa-7-azaspiro[3.5]nonane-7-yl)methyl)-7-chloro-5-(2-methylpyridin-3-yl)imidazo[1,2-a]quinoxalin-4(5H)-one
64) 7-chloro-2-(((2-methoxyethyl)amino)methyl)-5-(2-methylpyridin-3-yl)imidazo[1,2-a]quinoxalin-4(5H)-one
65) 5-(2,3-dihydrobenzofuran-4-yl)-7-(trifluoromethyl)imidazo[1,2-a]quinoxalin-4(5H)-one
66) 2-((methylamino)methyl)-5-(2-methylpyridin-3-yl)-7-(trifluoromethyl)-3,3a-dihydroimidazo[1,2-a]quinoxalin-4(5H)-one
67) 2-(methoxymethyl)-5-(2-methylpyridin-3-yl)-7-(trifluoromethyl)-3,3a-dihydroimidazo[1,2-a]quinoxalin-4(5H)-one
68) 2-(((2-hydroxyethyl)amino)methyl)-5-(2-methylpyridin-3-yl)-7-(trifluoromethyl)-3,3a-dihydroimidazo[1,2-a]quinoxalin-4(5H)-one
69) 2-(azetidin-1-ylmethyl)-5-(2-methylpyridin-3-yl)-7-(trifluoromethyl)-3,3a-dihydroimidazo[1,2-a]quinoxalin-4(5H)-one
70) 2-((3-hydroxyazetidin-1-yl)methyl)-5-(2-methylpyridin-3-yl)-7-(trifluoromethyl)-3,3a-dihydroimidazo[1,2-a]quinoxalin-4(5H)-one
71) 7-chloro-9-fluoro-5-(o-tolyl)imidazo[1,2-a]quinoxalin-4(5H)-one
72) 8-bromo-7-chloro-5-(o-tolyl)imidazo[1,2-a]quinoxalin-4(5H)-one
73) 7-chloro-8-methyl-5-(o-tolyl)imidazo[1,2-a]quinoxalin-4(5H)-one
74) 7-bromo-8-methoxy-5-(o-tolyl)imidazo[1,2-a]quinoxalin-4(5H)-one
75) 7-bromo-8-methoxy-5-(2-methylpyridin-3-yl)imidazo[1,2-a]quinoxalin-4(5H)-one
76) 7-bromo-5-(2-methylpyridin-3-yl)-8-(trifluoromethyl)imidazo[1,2-a]quinoxalin-4(5H)-one
77) 7-bromo-8-fluoro-5-(2-methylpyridin-3-yl)imidazo[1,2-a]quinoxalin-4(5H)-one
78) 7-bromo-8-methyl-5-(2-methylpyridin-3-yl)imidazo[1,2-a]quinoxalin-4(5H)-one
79) 7-bromo-8-chloro-5-(2-methylpyridin-3-yl)imidazo[1,2-a]quinoxalin-4(5H)-one
80) (*R*ₐ)-7-bromo-8-chloro-5-(2-methylpyridin-3-yl)imidazo[1,2-a]quinoxalin-4(5*H*)-one
81) (*S*ₐ)-7-bromo-8-chloro-5-(2-methylpyridin-3-yl)imidazo[1,2-a]quinoxalin-4(5*H*)-one
82) 7-bromo-8-(difluoromethyl)-5-(2-methylpyridin-3-yl)imidazo[1,2-a]quinoxalin-4(5H)-one
83) 7-bromo-8-chloro-5-(1-methyl-1H-pyrazol-5-yl)imidazo[1,2-a]quinoxalin-4(5H)-one
84) 7-bromo-1,8-dimethyl-5-(2-methylpyridin-3-yl)imidazo[1,2-a]quinoxalin-4(5H)-one
85) 7,9-dibromo-1,8-dimethyl-5-(2-methylpyridin-3-yl)imidazo[1,2-a]quinoxalin-4(5*H*)-one
86) 7-bromo-8-chloro-1-methyl-5-(2-methylpyridin-3-yl)imidazo[1,2-a]quinoxalin-4(5H)-one
87) 7-bromo-8-chloro-5-(2-cyclopropylpyridin-3-yl)imidazo[1,2-a]quinoxalin-4(5H)-one
88) 1,7-dibromo-8-chloro-5-(2-methylpyridin-3-yl)imidazo[1,2-a]quinoxalin-4(5H)-one
89) 7-bromo-1,8-dichloro-5-(2-methylpyridin-3-yl)imidazo[1,2-a]quinoxalin-4(5H)-one
90) 7-bromo-5-(2-hydroxypyridin-3-yl)-8-methylimidazo[1,2-a]quinoxalin-4(5H)-one
91) 7-bromo-5-(2-chloropyridin-3-yl)-8-methylimidazo[1,2-a]quinoxalin-4(5H)-one
92) 2,7-dibromo-8-chloro-1-methyl-5-(2-methylpyridin-3-yl)imidazo[1,2-a]quinoxalin-4(5H)-one
93) 8-methyl-5-(2-methylpyridin-3-yl)-7-(trifluoromethyl)imidazo[1,2-a]quinoxalin-4(5H)-one
94) (*R*ₐ)-8-methyl-5-(2-methylpyridin-3-yl)-7-(trifluoromethyl)imidazo[1,2-a]quinoxalin-4(5H)-one
95) (*S*ₐ)-8-methyl-5-(2-methylpyridin-3-yl)-7-(trifluoromethyl)imidazo[1,2-a]quinoxalin-4(5H)-one
96) 8-chloro-5-(2-methylpyridin-3-yl)-7-(trifluoromethyl)imidazo[1,2-a]quinoxalin-4(5H)-one
97) (*R*ₐ)-8-chloro-5-(2-methylpyridin-3-yl)-7-(trifluoromethyl)imidazo[1,2-a]quinoxalin-4(5H)-one
98) (*S*ₐ)-8-chloro-5-(2-methylpyridin-3-yl)-7-(trifluoromethyl)imidazo[1,2-a]quinoxalin-4(5*H*)-one
99) 8-chloro-1-methyl-5-(2-methylpyridin-3-yl)-7-(trifluoromethyl)imidazo[1,2-a]quinoxalin-4(5*H*)-one
100) 8-chloro-2-(dimethylamino)-1-methyl-5-(2-methylpyridin-3-yl)-7-(trifluoromethyl)imidazo[1,2-a]quinoxalin-4(5H)-one
101) 7-chloro-9-fluoro-5-(2-methylpyridin-3-yl)imidazo[1,2-a]quinoxalin-4(5H)-one
102) 7-chloro-9-methoxy-5-(2-methylpyridin-3-yl)imidazo[1,2-a]quinoxalin-4(5H)-one
103) 7-chloro-9-(cyclopropylmethoxy)-5-(2-methylpyridin-3-yl)imidazo[1,2-a]quinoxalin-4(5H)-one
104) 7-chloro-5-(2-methylpyridin-3-yl)-9-(pyrrolidine-1-yl)imidazo[1,2-a]quinoxalin-4(5H)-one
105) 7-chloro-9-(methylamino)-5-(2-methylpyridin-3-yl)imidazo[1,2-a]quinoxalin-4(5H)-one
106) 7-chloro-9-(dimethylamino)-5-(2-methylpyridin-3-yl)imidazo[1,2-a]quinoxalin-4(5H)-one
107) 7-bromo-8-chloro-5-(2-methoxypyridin-3-yl)imidazo[1,2-a]quinoxalin-4(5H)-one
108) 5-(benzo[d]thiazol-7-yl)-7-bromo-8-chloroimidazo[1,2-a]quinoxalin-4(5H)-one
109) 7-bromo-8-chloro-5-(1H-indol-4-yl)imidazo[1,2-a]quinoxalin-4(5H)-one
110) 8-chloro-5-(2-ethylpyridin-3-yl)-7-(trifluoromethyl)imidazo[1,2-a]quinoxalin-4(5H)-one
111) (*R*ₐ)-8-chloro-5-(2-ethylpyridin-3-yl)-7-(trifluoromethyl)imidazo[1,2-a]quinoxalin-4(5H)-one
112) (*S*ₐ)-8-chloro-5-(2-ethylpyridin-3-yl)-7-(trifluoromethyl)imidazo[1,2-a]quinoxalin-4(5H)-one
113) 9-methoxy-5-(2-methylpyridin-3-yl)-7-(trifluoromethyl)imidazo[1,2-a]quinoxalin-4(5H)-one
114) 9-methylamino-5-(2-methylpyridin-3-yl)-7-(trifluoromethyl)imidazo[1,2-a]quinoxalin-4(5H)-one
115) 9-(difluoromethoxy)-5-(2-methylpyridin-3-yl)-7-(trifluoromethyl)imidazo[1,2-a]quinoxalin-4(5H)-one
116) 5-(2-methylpyridin-3-yl)-9-(trifluoromethoxy)-7-(trifluoromethyl)imidazo[1,2-a]quinoxalin-4(5H)-one
117) 5-(2-methylpyridin-3-yl)-9-(2,2,2-trifluoroethoxy)-7-(trifluoromethyl)imidazo[1,2-a]quinoxalin-4(5H)-one
118) 9-ethoxy-5-(2-methylpyridin-3-yl)-7-(trifluoromethyl)imidazo[1,2-a]quinoxalin-4(5H)-one
119) 8-chloro-1-methyl-2-(methylamino)-5-(2-methylpyridin-3-yl)-7-(trifluoromethyl)imidazo[1,2-a]quinoxalin-4(5H)-one
120) 8-chloro-2-methoxy-1-methyl-5-(2-methylpyridin-3-yl)-7-(trifluoromethyl)imidazo[1,2-a]quinoxalin-4(5H)-one

18. A pharmaceutical composition comprising the compound, an optical isomer, a stereoisomer, a racemate, an isotopologue, a solvate, a hydrate, or a pharmaceutically acceptable salt thereof according to any one of claims 1 to 17.

19. The pharmaceutical composition according to claim 18, wherein the pharmaceutical composition is for prevention or treatment of a disease related to the methionine adenosyltransferase 2A (MAT2A) protein.

20. The pharmaceutical composition according to claim 19, wherein the disease related to the MAT2A protein is cancer.

21. The pharmaceutical composition according to claim 20, wherein the cancer is a cancer with reduced activity or expression of methylthioadenosine phosphorylase (MTAP), or a cancer in which all or part of the MTAP gene is deleted.

22. The pharmaceutical composition according to claim 20, wherein the cancer is one or more selected from the group consisting of acoustic neuroma, adenocarcinoma, adrenal cancer, anal cancer, angiosarcoma, benign monoclonal gammopathy, bile duct cancer, bladder cancer, breast cancer, brain cancer, lymphoma, multiple myeloma, lacrimal gland tumor, bronchial cancer, cervical cancer, craniopharyngioma, colorectal cancer, epithelial carcinoma, ependymoma, endothelial sarcoma, endometrial cancer, esophageal cancer, Barrett's adenocarcinoma, Ewing's sarcoma, eye cancer, gallbladder cancer, gastric cancer, colon cancer, gastrointestinal stromal tumor (GIST), head and neck cancer, oral squamous cell carcinoma (OSCC), throat cancer, hematologic malignancy, hemangioblastoma, inflammatory myofibroblastic tumor, immunoglobulin light chain (AL) amyloidosis, kidney cancer, renal cell carcinoma, liver cancer, lung cancer, melanoma, uveal melanoma, acute myeloid leukemia (AML), myelodysplastic syndrome (MDS), mesothelioma, myeloproliferative disorder (MPD), chronic idiopathic myelofibrosis, chronic myeloid leukemia (CML), chronic neutrophilic leukemia (CNL), neuroblastoma, neurofibroma, neuroendocrine cancer, osteosarcoma, ovarian cancer, papillary adenocarcinoma, pancreatic cancer, penile cancer, prostate cancer, rectal cancer, rhabdomyosarcoma, salivary gland cancer, skin cancer, small intestine cancer, soft tissue sarcoma, thyroid cancer, urethral cancer, vaginal cancer, glioblastoma (GBM), glioma, peripheral nerve sheath tumor, thymic cancer, adenoid cystic carcinoma (ACC), and vulvar cancer.

23. The pharmaceutical composition according to claim 20, wherein the cancer is one or more selected from the group consisting of lung cancer, bladder cancer, gastric cancer, esophageal cancer, head and neck cancer, pancreatic cancer, colon cancer, bile duct cancer, mesothelioma, peripheral nerve sheath tumor, thymic cancer, and adenoid cystic carcinoma (ACC).

24. The pharmaceutical composition according to claim 22, wherein the brain cancer is one or more selected from the group consisting of meningioma, glioma, medulloblastoma, glioblastoma, and brain metastases.

25. A composition for inhibiting an activity of MAT2A protein, comprising the compound, an optical isomer, a stereoisomer, a racemate, an isotopologue, a solvate, a hydrate, or a pharmaceutically acceptable salt thereof according to any one of claims 1 to 17.

26. A pharmaceutical combination for treating and/or preventing cancer in a subject, comprising a combination of the compound, an optical isomer, a stereoisomer, a racemate, an isotopologue, a solvate, a hydrate, or a pharmaceutically acceptable salt thereof according to any one of claims 1 to 17; and a cytotoxic anticancer agent.

27. A pharmaceutical combination for treating and/or preventing cancer in a subject, comprising a combination of the compound, an optical isomer, a stereoisomer, a racemate, an isotopologue, a solvate, a hydrate, or a pharmaceutically acceptable salt thereof according to any one of claims 1 to 17; and a targeted anticancer agent.

28. The pharmaceutical combination according to claim 26, wherein the anticancer agent comprises one or more selected from the group consisting of alkylating agents, antimetabolites, DNA polymerase inhibitors and microtubule inhibitors.

29. The pharmaceutical combination according to claim 27, wherein the targeted anticancer agent comprises one or more selected from the group consisting of a monoclonal antibody, a small molecule targeted therapeutic agent, an antibody-drug conjugate, and an immune checkpoint inhibitor.

30. The pharmaceutical combination according to claim 26 or 27, wherein the cancer is a cancer with reduced activity or expression of methylthioadenosine phosphorylase (MTAP), or a cancer in which all or part of the MTAP gene is deleted.

31. The pharmaceutical combination according to claim 26 or 27, wherein the cancer is one or more selected from the group consisting of acoustic neuroma, adenocarcinoma, adrenal cancer, anal cancer, angiosarcoma, benign monoclonal gammopathy, bile duct cancer, bladder cancer, breast cancer, brain cancer, lymphoma, multiple myeloma, lacrimal gland tumor, bronchial cancer, cervical cancer, craniopharyngioma, colorectal cancer, epithelial carcinoma, ependymoma, endothelial sarcoma, endometrial cancer, esophageal cancer, Barrett's adenocarcinoma, Ewing's sarcoma, eye cancer, gallbladder cancer, gastric cancer, colon cancer, gastrointestinal stromal tumor (GIST), head and neck cancer, oral squamous cell carcinoma (OSCC), throat cancer, hematologic malignancy, hemangioblastoma, inflammatory myofibroblastic tumor, immunoglobulin light chain (AL) amyloidosis, kidney cancer, renal cell carcinoma, liver cancer, lung cancer, melanoma, uveal melanoma, acute myeloid leukemia (AML), myelodysplastic syndrome (MDS), mesothelioma, myeloproliferative disorder (MPD), chronic idiopathic myelofibrosis, chronic myeloid leukemia (CML), chronic neutrophilic leukemia (CNL), neuroblastoma, neurofibroma, neuroendocrine cancer, osteosarcoma, ovarian cancer, papillary adenocarcinoma, pancreatic cancer, penile cancer, prostate cancer, rectal cancer, rhabdomyosarcoma, salivary gland cancer, skin cancer, small intestine cancer, soft tissue sarcoma, thyroid cancer, urethral cancer, vaginal cancer, glioblastoma (GBM), glioma, peripheral nerve sheath tumor, thymic cancer, adenoid cystic carcinoma (ACC), and vulvar cancer.

32. The pharmaceutical combination according to claim 26 or 27, wherein the cancer is one or more selected from the group consisting of lung cancer, bladder cancer, gastric cancer, esophageal cancer, head and neck cancer, pancreatic cancer, colon cancer, bile duct cancer, mesothelioma, peripheral nerve sheath tumor, thymic cancer, and adenoid cystic carcinoma (ACC).

33. The pharmaceutical combination according to claim 31, wherein the brain cancer is one or more selected from the group consisting of meningioma, glioma, medulloblastoma, glioblastoma, and brain metastasis.
